# EUROPEAN PATENT SPECIFICATION

(11) **EP 0 482 080 B1**
(45) Date of publication and mention of the grant of the patent: **26.03.1997**
(21) Application number: 90911371.4
(22) Date of filing: 05.07.1990
(51) Int. Cl.: C07K 14/75, C07K 14/78, A61K 38/36, A61K 38/39, C07K 7/56

(54) **SMALL CYCLIC PLATELET AGGREGATION INHIBITORS**
KLEINE ZYKLISCHE INHIBITOREN DER BLUTPLÄTTCHENAGGREGATION
INHIBITEURS D'AGGREGATION DE PLAQUETTES A BASE DE PETITS PEPTIDES CYCLIQUES

(30) Priority: 17.07.1989 US 380957
(43) Date of publication of application: 29.04.1992
(73) Proprietor: GENENTECH, INC., South San Francisco California 94080 (US)
(72) Inventor: BARKER, Peter, L., El Granada, CA 94018 (US); BURNIER, John, P., Pacifica, CA 94044 (US); GADEK, Thomas, Oakland, CA 94611 (US); THORSETT, Eugene, D., Moss Beach, CA 94038 (US)
(74) Representative: Nicholls, Kathryn Margaret
(86) International application number: PCT/US90/03788
(87) International publication number: WO 91/01331

(56) References cited:
- EP-A- 0 406 428
- EP-A- 0 410 541
- WO-A-89/07609
- WO-A-90/02751
- Peptides, Chemistry, Structure and Biology, Proceedings of the Eleventh American Peptide Symposium, 9-14 July 1989, La Jolla, California, US, F. EL-FAHAIL ALI et al., pp. 94-96
- The Journal of Biological Chemistry, vol. 262, no. 36, 25 December 1987, Baltimore, US, M.D. PIERSCHBACHER et al., pp. 17294-17298

## Description

### Field of the Invention

The present invention relates to inhibitors of platelet aggregation. Specifically, the invention is directed to antagonists of the final common pathway of platelet aggregation and that as potent antithrombotics. The invention further relates to therapeutic applications of these inhibitors in diseases for which blocking of platelet aggregation and intracellular adhesion is indicated.

### Background of the Invention

Platelets are particles found in whole blood which participate in the process of thrombus formation and blood coagulation. A membrane bound glycoprotein, commonly known as GP IIbIIIa is present on the exterior surface of platelets. Glycoprotein IIbIIIa is a non-covalent, calcium ion dependent heterodimer complex composed of alpha and beta subunits (Jennings, et al., *J. Biol. Chem*. (1982) **257**, 10458). These glycoproteins contribute to normal platelet function through interactions with proteins containing the amino acid sequence Arg-Gly-Asp, such as fibrinogen. The interaction of GP IIbIIIa with fibrinogen is stimulated by certain factors released or exposed when a blood vessel is injured. Multiple factors, including a variety of physiologic stimuli and soluble mediators, initiate platelet activation via several pathways. These pathways have a common final step which is the activation of the GP IIbIIIa receptor on the platelet surface and its subsequent binding to fibrinogen followed by aggregation and thrombus formation. By virtue of these interactions GP IIbIIIa is a component of the platelet aggregation system (Pytela et al., *Science* (1986) **231**, 1559). Thus, inhibition of the interaction of GP IIbIIIa with Arg-Gly-Asp containing ligands such as fibrinogen is a useful means of modulating thrombus formation. An inhibitor which prevents this binding interaction would antagonize platelet activation by any stimulus and therefore would have important antithrombotic properties.

Many common human disorders are characteristically associated with a hyperthrombotic state leading to intravascular thrombi and emboli. These are a major cause of medical morbidity, leading to infarction, stroke and phlebitis and of mortality from stroke and pulmonary and cardiac emboli. Patients with atherosclerosis are predisposed to arterial thromboembolic phenomena for a variety of reasons. Atherosclerotic plaques form niduses for platelet plugs and thrombii that lead to vascular narrowing and occlusion, resulting in myocardial and cerebral ischemic disease. This may happen spontaneously or following procedures such as angioplasty or endarteroectomy. Thrombii that break off and are released into the circulation cause infarction of different organs, especially the brain, extremities, heart and kidneys.

In addition to being invoked in arterial thrombosis, platelets may also play a role in venous thrombosis. A large percentage of such patients have no antecedent risk factors and develop venous thrombophlebitis and subsequent pulmonary emboli without a known cause. Other patients who form venous thrombi have underlying diseases known to predispose to these syndromes. Some of these patients may have genetic or acquired deficiencies of factors that normally prevent hypercoagulability, such as antithrombin-3. Others have mechanical obstructions to venous flow, such as tumor masses, that lead to low flow states and thrombosis. Patients with malignancy have a high incidence of thrombotic phenomena for unclear reasons. Antithrombotic therapy in this situation with currently available agents is dangerous and often ineffective.

Patients whose blood flows over artificial surfaces, such as prosthetic synthetic cardiac valves or through extracorporeal perfusion devices, are also at risk for the development of platelet plugs, thrombii and emboli. It is standard practice that patients with artificial cardiac valves be chronically anti-coagulated. However, in all instances, platelet activation and emboli formation may still occur despite adequate anticoagulation treatment.

Thus, a large category of patients, including those with atherosclerosis, coronary artery disease, artifical heart valves, cancer, and a history of stroke, phlebitis, or pulmonary emboli, are candidates for limited or chronic antithrombotic therapy. The number of available therapeutic agents is limited and these, for the most part, act by inhibiting or reducing levels of circulating clotting factors. These agents are frequently not effective against the patient's underlying hematologic problem, which often concerns an increased propensity for platelet aggregation and adhesion. They also cause the patent to be susceptible to abnormal bleeding. Available antiplatelet agents, such as aspirin, inhibit only part of the platelet activation process and are therefore often inadequate for therapy.

An agent which effectively inhibits the final common pathway of platelet activation, namely fibrinogen binding to the GP IIbIIIa receptor, should accordingly be useful in a large group of disorders characterized by a hyperthrombotic state as described above. The present invention contemplates such an agent which is a new composition, namely a cyclic polypeptide consisting in part of natural amino acids and in part of unnatural amino acids. This new composition interferes with the interaction of Arg-Gly-Asp containing peptides, particularly fibrinogen, with the GP IIbIIIa complex thereby preventing platelet aggregation. Platelet aggregation has been identified as an early step in the formation of platelet plugs, emboli and thrombii in the circulatory system which in turn have been shown to play an active role in cardiovascular complications and disease. Inhibition of fibrinogen binding to the GP IIbIIIa complex has been shown to be an effective antithrombotic treatment in animals (H. K. Gold, et al., *Circulation* (1988) **77***,* 670-677; T. Yasuda, et al., *J*. *Clin. Invest* (1988) **81**, 1284-1291; B. S. Coller, et al., *Blood* (1986) **68**, 783-786.)

Other proteins such as fibronectin contain the Arg-Gly-Asp sequence of amino acids. Large polypeptide fragments of fibronectin have been shown to have activity for cell attachment to various surfaces which has been disclosed in U.S. Patents 4,517,686; 4,589,881; and 4,661,111. These large polypeptides contain the amino acid sequence Arg-Gly-Asp-Ser in the interior portion of the polypeptide chain. Short peptides derived from the large polypeptides were also found to promote cell attachment to various substrates when bound on the substrate. Alternatively, the same short peptides were found to inhibit cell attachment to the same substrates when dissolved or suspended in the medium surrounding the substrate. This activity has been disclosed in U.S. Patents 4,578,079, 4,614,517 and 4,792,525. The short peptides were defined as Q-Arg-Gly-Asp-AA1-B where Q is hydrogen or an amino acid; AA1 is serine, threonine, or cysteine; and B is hydroxy or an amino acid. No discussion of cyclizing these short peptides is presented.

A number of synthetic peptides, including cyclic disulfides, have been disclosed as inhibitors of fibrinogen binding to platelets all of which contain the Arg-Gly-Asp sequence. See U.S. Patent 4,683,291; WO89/05150; EPO 0 319 506 A2; EPO 0 341 915 *A2;* Plow et al., *Proc. Natl. Acad. Sci. USA* (1985) **82***,* 8057-8061; Ruggeri et al., *Proc. Natl. Acad. Sci. USA* (1986) **83***,* 5708-5712*;* Haverstick et al., *Blood* (1985) **66**, 946-952; Plow et al., *Blood* (1987) **70**,110-115; F. ElF. Ali, et al., *Proc. Eleventh Amer. Peptide Symp.* (1990) 94-96; M. Pierschbacher and E. Ruoslahti, *J*. *Biol. Chem.* (1987) **262**, 17294-17298; and references cited in the above publications. F.ELF. Ali, et al., Proc. Eleventh Amer. Symp. (1990) 94-96 discloses that cyclic disulphides containing the RGD sequence show greater potency than their linear counterparts.

Several synthetic cyclic peptides containing the thioether linkage have been synthesized. Gero et al., *Biochem. Biophys. Res. Comm.* (1984) **120**, 840-845 describe a pseudohexapeptide analog of somatostatin where the group [CH₂-S] is substituted for a peptide bond. Similarly, Edwards et al., *Biochem. Biophys. Res. Comm.* (1986) **136**, 730-736 compare the biological activity of linear and cyclic enkephalin pseudopeptide analogs containing the thiomethylene ether linkage. Other enkephalin related pseudopeptides and macrocycles containing the [CH₂-S] substitution for peptides have been described, Spatola et al., *Biopolymers* (1986) **25**, 229-244 and Spatola et al., *Tetrahedron* (1988) **44***,* 821-833.

None of these references disclose a small cyclic peptide containing a stable ring structure having high platelet aggregation inhibition activity.

Accordingly, it is an object of this invention to produce a small cyclic peptide having high platelet aggregation inhibition activity. It is a further object to produce such small cyclic peptides that are stable to ring opening. It is still a further object of this invention to provide a platelet aggregation inhibitor having a long *in vivo* lifetime.

These and other objects of this invention will be apparent from consideration of the invention as a whole.

### Summary of the Invention

The objects of this invention are accomplished by providing a small cyclic peptide containing the tripeptide sequence Arg-Gly-Asp and containing a thioether, sulfoxide or sidechain amide bond within the cycle. Preferably, the cydic peptide has about 5 amino acids forming the ring of the cycle. More preferably, the ring of the cyclic peptide contains from about 17 to about 18 atoms, most preferably 18 atoms.

Also preferably, the ring will contain a D amino acid most preferably linked to the Arg of the tripeptide sequence.

The preferred and most preferred compounds of the instant invention are represented by Formulae I and Ia below.

The invention in its broad aspects relates to peptide derivatives which are useful as inhibitors platelet function mediated by the GP IIbIIIa receptor and for the prevention of thrombus formation. Preferred compounds of this invention are represented by Formula I: 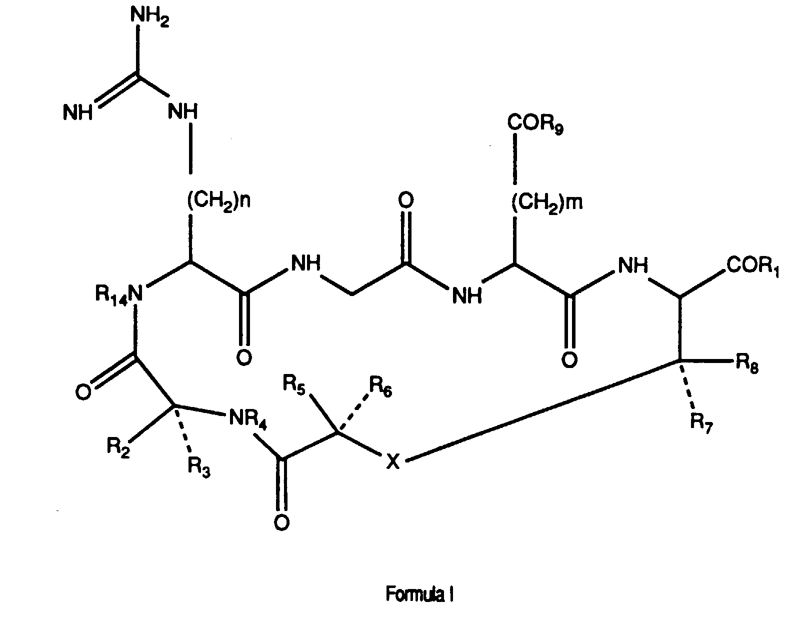 wherein
R₁ and R₉ are the same or different and are selected from
   hydroxy,
   C₁-C₈ alkoxy,
   C₃-C₁₂ alkenoxy,
   C₆-C₁₂ aryloxy,
   di-C₁-C₈ alkylamino-C₁-C₈-alkoxy,
   acylamino-C₁-C₈-alkoxy selected from the group acetylaminoethoxy, nicotinoylaminoethoxy, and succinamidoethoxy,
   pivaloyloxyethoxy,
   C₆-C₁₂ aryl-C₁-C₈-alkoxy where the aryl group is unsubstituted or substituted with one or more of the groups nitro, halo (F, Cl, Br, I), C₁-C₄-alkoxy, and amino,
   hydroxy-C₂-C₈-alkoxy,
   dihydroxy-C₃-C₈-alkoxy, and
   NR₁₀R₁₁ wherein R₁₀ and R₁₁ are the same or different and are hydrogen, C₁-C₈-alkyl, C₃-C₈-alkenyl, C₆-C₁₂-aryl where the aryl group is unsubstituted or substituted with one or more of the groups
      nitro, halo (F, Cl, Br, I), C₁-C₄-alkoxy, and amino, C₆-C₁₂-aryl-C₁-C₈-alkyl where the aryl group is unsubstituted or substituted by one or more of the groups nitro,
      halo (F, Cl, Br, I), C₁-C₄-alkoxy, and amino;
R₂, R₃, R₅, R₆, R₇, R₈ are the same or different and are selected from hydrogen,
C₆-C₁₂ aryl where the aryl group is unsubstituted or substituted by one or more of the groups nitro, hydroxy, halo (F, Cl, Br, I), C₁-C₈ alkyl, halo-C₁-C₈ alkyl, C₁-C₈-alkoxy, amino, phenyloxy, phenyl, acetamido, benzamido, di-C₁-C₈ alkylamino, C₁-C₈ alkylamino, C₆-C₁₂ aroyl, C₁-C₈ alkanoyl, and hydroxy-C₁-C₈ alkyl,
C₁-C₁₂ alkyl either substituted or unsubstituted, branched or straight chain where the substituents are selected from halo (F, G, Br, I),
   C₁-C₈ alkoxy,
   C₆-C₁₂ aryloxy where the aryl group is unsubstituted or substituted by one or more of the groups nitro, hydroxy, halo (F, Cl, Br, I), C₁-C₈ alkyl, C₁-C₈-alkoxy, amino, phenyloxy, acetamido, benzamido, di-C₁-C₈ alkylamino, C₁-C₈ alkylamino, C₆-C₁₂ aroyl, and C₁-C₈ alkanoyl,
   isothioureido,
   C₃-C₇ cycloalkyl,
   ureido,
   amino,
   C₁-C₈ alkylamino,
   di-C₁-C₈ alkylamino,
   hydroxy,
   amino-C₂-C₈ akylthio,
   amino-C₂-C₈ alkoxy,
   acetamido,
   benzamido wherein the phenyl ring is unsubstituted or substituted by one or more of the groups nitro, hydroxy, halo (F, Cl, Br, I), C₁-C₈ alkyl, C₁-C₈-alkoxy, amino, phenyloxy, acetamido, benzamido, di-C₁-C₈ alkylamino, C₁-C₈ alkylamino, C₆-C₁₂ aroyl, and C₁-C₈ alkanoyl,
   C₆-C₁₂ arylamino wherein the aryl group is unsubstituted or substituted by one or more of the groups nitro, hydroxy, halo, C₁-C₈ alkyl, C₁-C₈-akoxy, amino, phenyloxy, acetamido, benzamido, di-C₁-C₈ alkylamino, C₁-C₈ alkylamino, C₆-C₁₂ aroyl, and C₁-C₈ alkanoyl.
   guanidino,
   phthalimido,
   mercapto,
   C₁-C₈ alkylthio,
   C₆-C₁₂ arylthio,
   carboxy,
   carboxamide,
   carbo-C₁-C₈ alkoxy,
   C₆-C₁₂ aryl wherein the aryl group is unsubstituted or substituted by one or more of the groups nitro, hydroxy, halo, C₁-C₈ alkyl, C₁-C₈-alkoxy, amino, phenyloxy, acetamido, benzamido, di-C₁-C₈ alkylamino, C₁-C₈ alkylamino, hydroxy-C₁-C₈ alkyl, C₆-C₁₂ aroyl, and C₁-C₈ alkanoyl, and
   aromatic heterocycle wherein the heterocyclic groups have 5-10 ring atoms and contain up to two O, N, or S heteroatoms;
R₂ and R₃, R₅ and R₆, or R₇ and R₈ may optionally and independently be joined together to form a carboxyclic or heterocyclic ring of from four to seven atoms where the heteroatoms are selected from O, S or NR₁₂ where R₁₂ is selected from
   hydrogen, C₁-C₈-alkyl, C₃-C₈-alkenyl, C₆-C₁₂-aryl, C₆-C₁₂-aryl-C₁-C₈-alkyl, C₁-C₈ alkanoyl, and C₆-C₁₂ aroyl;
R₄ is selected from
   hydrogen,
   C₁-C₈ alkyl,
   C₃-C₁₀ cycloalkyl,
   C₆-C₁₂ aryl, and
   C₆-C₁₂ aryl-C₁-C₈-alkyl;
R₂ or R₃ may be optionally pined with R₄ to form a piperidine, pyrrolidine or thiazolidine ring;
R₁₄ is selected from
   hydrogen, C₁-C₈-alkyl, C₃-C₈-alkenyl, C₆-C₁₂-aryl, and C₆-C₁₂ aryl-C₁-C₈-alkyl;
X is selected from
   an O or S atom,
   an S atom bearing one or two O atoms,
   NR₁₃ wherein R₁₃ is hydrogen, C₁-C₈-alkyl, C₃-C₈-alkenyl, C₆-C₁₂-aryl, C₆-C₁₂-aryl-C₁-C₈-alkyl, C₁-C₈ alkanoyl, and C₆-C₁₂ aroyl, and
   C₆-C₁₂ aryl,
   C₁-C₈ alkanoyl,
   (CH₂)ₖ where k is an integer from 0 to 5;
n is an integer from 1 to 6;
m is an integer from 0 to 4; and
pharmaceutically acceptable salts thereof.

As used herein and unless specified otherwise: alkyl, alkenyl and alkynyl denote straight and branched hydrocarbon chains having single, double and triple bonds, respectively; C₆-C₁₂ aryl groups denote unsubstituted aromatic ring or fused rings such as, for example, phenyl or naphthyl; hetero denotes the heteroatoms O, N, or S; aromatic heterocyclic groups have 5-10 ring atoms and contain up to four heteroatoms; halogen or halo denote F, Cl Br, or I atoms; alkoxy denotes an alkyl group attached to O.

Examples of C₁-C₈ alkyl or C₂-C₈ alkenyl groups include methyl, ethyl, propyl, isopropyl, butyl, t-butyl, pentyl, isopentyl, hexyl, vinyl, allyl, butenyl and the like; examples of C₃-C₁₀-cycloalkyl groups include cyclopropyl, cyclopentyl, cyclohexyl, and the like; aromatic heterocyclic groups include but are not limited to pyridyl, thienyl, furyl, indolyl, benzthienyl, imidazolyl, thiazolyl, quinolinyl and isoguinolinyl.

Most preferred compounds of the instant invention are represented by Formula Ia: 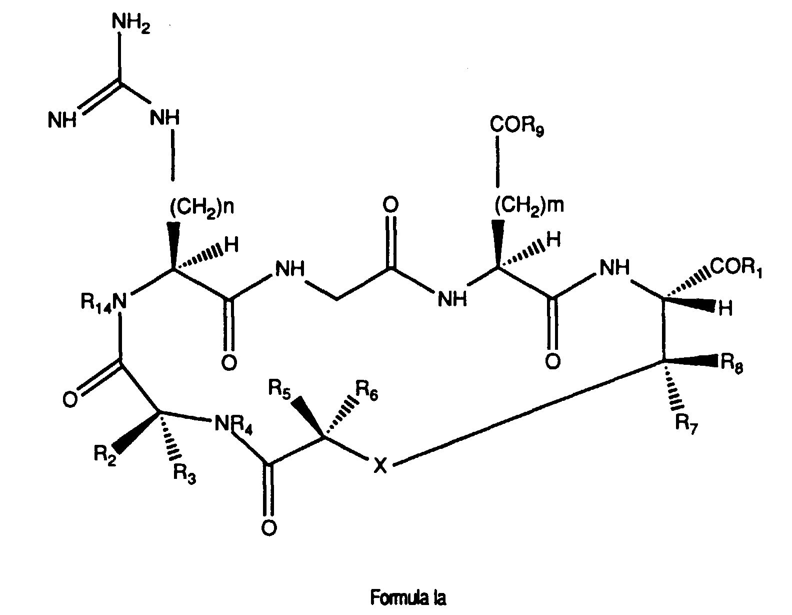 wherein
R₁ and R₉ are the same or different and are hydroxy, NH₂, C₁-C₄ alkoxy or benzyloxy;
R₂ is hydrogen
R₃ is selected from
   hydrogen,
   C₁-C₆ alkyl branched or unbranched, unsubstituted or substituted with substituents selected from amino, hydroxy, mercapto, methylthio, carboxy, carboxamide, guanidino, phenyl, 4-hydroxyphenyl, 4-methoxyphenyl, 3-indolyl, and 4-imidazole,
   phenyl either unsubstituted or substituted with one to three substituents that may be independently nitro, hydroxy, halo (F, Cl, Br, I), C₁-C₄ alkyl, C₁-C₄-alkoxy, amino, phenyloxy, phenyl, acetamido, benzamido, di-C₁-C₄ alkylamino, C₁-C₄ alkylamino, halo-C₁-C₄ alkyl, C₆-C₁₂ aroyl, and C₁-C₄ alkanoyl,
   1-naphtyl,
   2-naphthyl,
   2-thienyl,
   2-pyridyl,
   3-pyridyl, and
   4-pyridyl;
R₅ and R₆ are independently selected from
   hydrogen,
   C₁-C₆ alkyl, either branched or unbranched, unsubstituted or substituted with substituents selected from amino, hydroxy, mercapto, carboxy, carboxamide, guanidino, phenyl or 4-hydroxyphenyl, 4-methoxyphenyl, 3-indolyl, and 4-imidazolyl,
   phenyl, either unsubstituted or substituted with one to three substituents that may be independently selected from nitro, hydroxy, halo (F, Cl, Br, I), C₁-C₄ alkyl, C₁-C₄-alkoxy, amino, phenyloxy, phenyl, acetamido, benzamido, di-C₁-C₄ alkylamino, C₁-C₄ alkylamino, halo-C₁-C₄ alkyl, C₆-C₁₂ aroyl, and C₁-C₄ alkanoyl,
   1-naphthyl,
   2-naphthyl,
   2-thienyl,
   2-pyridyl,
   3-pyridyl, and
   4-pyridyl;
R₇ or R₈ are the same or different and are selected from
   hydrogen,
   C₁-C₄ alkyl,
   phenyl either unsubstituted or substituted with from one to three substituents independently selected from hydroxy, halo (F, Cl, Br, I), C₁-C₄ alkyl, and C₁-C₄-alkoxy;
R₄ is hydrogen or may be joined with R₃ to form a heterocyclic ring selected from piperidine, pyrrolidine or thiazoidine;
R₁₄ is hydrogen or methyl;
X is selected from
   an O or S atom,
   an S atom bearing one or two O atoms,
   NR₁₃ where R₁₃ is selected from hydrogen, C₁-C₄ alkyl, benzyl, phenyl, C₁-C₄ alkanoyl, benzoyl and
   (CH₂)ₖ, where k is 0 to 5;
n is 3 or 4;
m is 1; and
pharmaceutically acceptable salts thereof.

The present invention includes a method of making the compounds of Formulae I and Ia.

The present invention also includes a method for reducing platelet aggregation in a mammal. This method involves administering a therapeutically effective amount of the compounds of the present invention alone or in combination with a pharmacologically acceptable carrier. This general method may also be applied to treat a mammal having an increased propensity for thrombus formation.

Additionaly, the present invention is directed to compositions of matter for reducing platelet aggregation in a mammal; treating a mammal having an increased propensity for thrombus formation; or inhibiting binding of a ligand to GP IIbIIIa in a mammal; wherein each of these compositions contains as an active ingredient one or more of the cyclic peptides defined in Formula I.

### Detailed Description of the Invention

The products of Formula I and the preferred substituents can be made by using one of the methods depicted below or by other methods known in the art (see e.g., Spatola et al., *Tetrahedron* (1988) **44**, 821-833, and references cited therein). The definitions of the substituent groups are the same as for Formula I except where noted.

### Method A

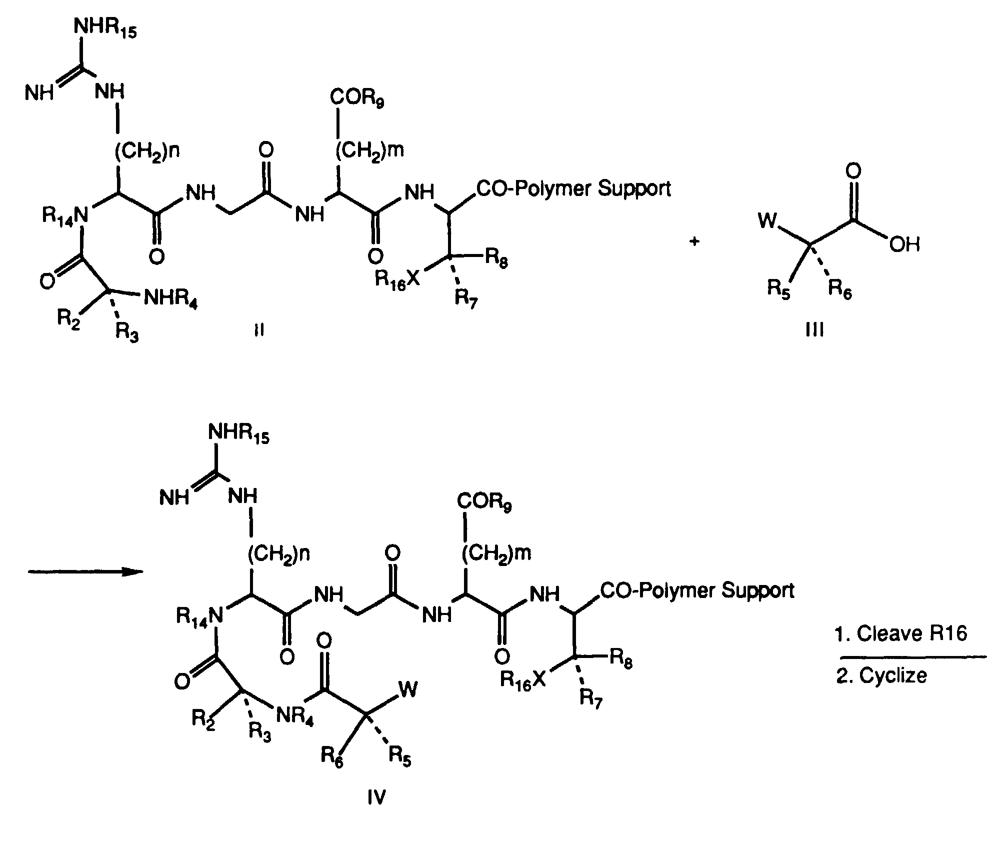

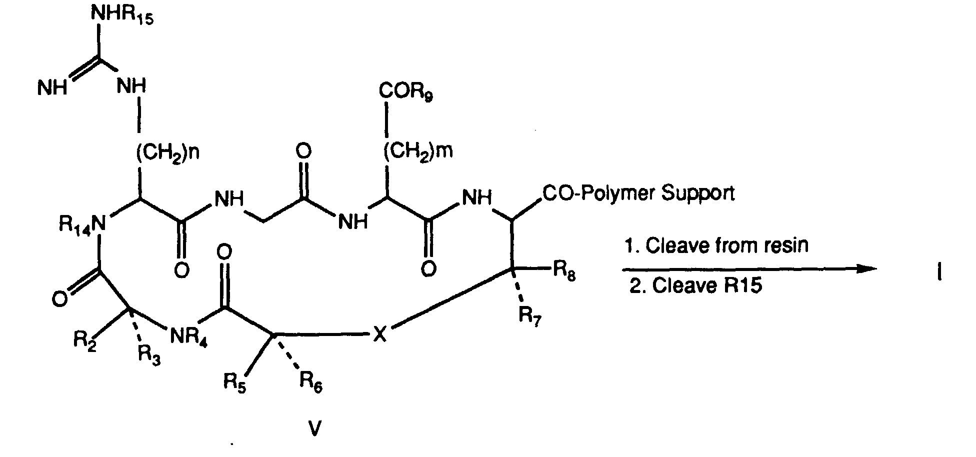

A peptide derivative bound to a polymer support, depicted by intermediate II, may be prepared by sequential coupling of individual amino acid derivatives by standard techniques. (Merrifield, R. B., J. *Am. Chem.* Soc. (1963) **85**, 2149-2154; Stewart, J. M. and Young, J. D., *Solid Phase Peptide Synthesis (1984),* Pierce Chemical Co., Rockford, IL and additional references cited in the above publications). When the tetrapeptide derivative II is obtained, the terminal amino group is acylated with a suitable carboxylic acid derivative III. The acylation to yield IV may be accomplished using a number of standard methods which require activation of the carboxylic acid group of III. For example, activation may be obtained by the addition of an equimolar amount of dicyclohexylcarbodiimide or related carbodiimide reagent. If desired an additive such as 1-hydroxybenztriazole or N-hydroxysuccinimide may be incorporated. Alternatively, the carboxyl group may be activated by conversion to a halo derivative. For example, the chloride may be obtained by treatment of the acid with thionyl chloride or oxalyl chloride in a compatible solvent such as dichloromethane, toluene, or ethylene dichloride if desired. The substituent W is chosen such that it is readily displaceable by the group X. Suitable substituents W are, for example, halo atoms such as bromine or iodine or activated oxygen functions such as methanesulfonyloxy or p-toluensulfonyloxy and related sulfonic acid esters.

Cyclization to the resin bound intermediate V may be accomplished by selectively exposing the nucleophilic group X by removal of R₁₆ and allowing X to react such that it displaces group W with formation of a new chemical bond. For example, if X is a sulfur or oxygen atom and R₁₆ is a triphenylmethyl group, then R₁₆ may be selectively deaved from X using a very dilute solution of a strong acid such as trifluoroacetic acid in a solvent compatible with the polymer resin. Examples of resin compatible solvents are dimethylacetamide, dimethylformamide or dichloromethane and the like.

The end result of the cleavage process is replacement of the R16 group with a hydrogen atom. After cleavage of R₁₆, the resin bound peptide derivative V (R₁₆ = H) is allowed to react in a suitable solvent such as dimethylacetamide until cyclization is complete. If desired, a base such as N-methylmorpholine may be incorporated into the reaction. Other protecting groups in the peptide molecule IV must be stable to the reaction conditions chosen to form V. For example, R₉ may be a group which affords an ester such as methoxy, ethoxy, benzyloxy, t-butyloxy and the like or an amide or substituted amide. R₁₅ may be an arylsulfonyl group such as 2,2,5,7,8-pentamethylchroman-6-sulfonyl (PMC) or p-toluenesulfonyl. Final cleavage of the cyclized peptide product from the polymer resin may be accomplished in a variety of ways dependent upon the type of resin used and the chemical linkage between the cydized peptide and the resin. If, for example, the resin is derived from a polymerized p-alkoxybenzyl alcohol derivative, then cleavage of the peptide-resin linkage may be carried out using a strong acid such as trifluoroacetic acid. If desired, additives such as phenol, anisole and ethanedithiol may be added to the reaction.

The groups R₉ and R₁₅ may be chosen, if desired, to also be cleavable concurrently with cleavage of the cyclized peptide from the polymer resin. Examples of such chemical groups are R₉ = t-butyloxy, cleavage of which yields R₉ = OH and R₁₅ = 2,2,5,7,8-pentamethylchroman-6-sulfonyl, cleavage of which affords R₁₅ = H. The crude product thus obtained may be further purified using chromatographic or other methods of chemical purification to obtain I.

Further derivatization of I may be carried out if desired. For example, if X is S, treatment of I with a stoichiometric amount of an oxidizing agent such as 3-chloroperoxybenzoic acid or similar agent will produce the sulfoxide derivative where X is SO. Use of an excess amount of oxidant will afford the sulfone derivative where X is SO₂.

### Method B

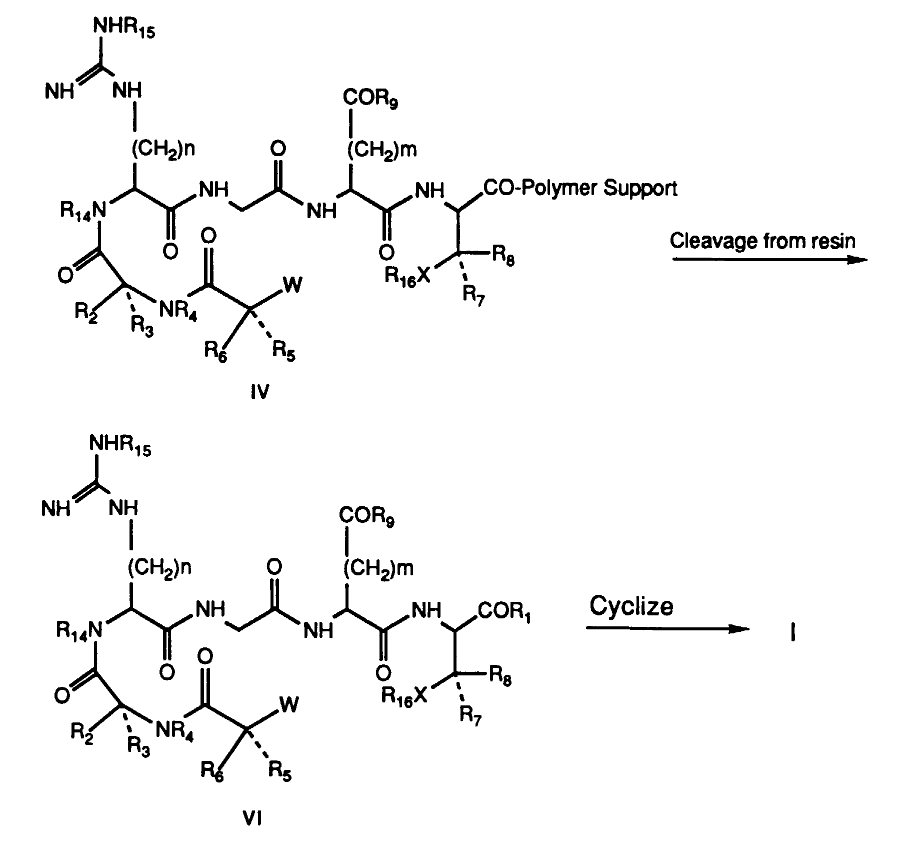

Alternatively, the linear peptide derivative IV, prepared as described above in Method A, may be cleaved from the resin prior to cyclization to yield VI. For example, if IV is synthesized on a polystyrene resin the cleavage can be accomplished using liquid hydrogen flouride. The groups R₉, R₁₅ and R₁₆ may, if desired, be cleaved concurrently under these conditions. If concurrent cleavage is desired, then examples of suitable substituents R₉ are t-butyloxy, benzyloxy or cyclohexyloxy, R₁₅ is p-toluenesulfonyl or 2,2,5,7,8-pentamethylchroman-6-sulfonyl and R₁₆ is triphenylmethyl or p-methylbenzyl if X is either O or S, or t-butxycarbonyl if X is NR₁₃. Cleavage of these groups would result in R₉ being OH and R₁₅ and R₁₆ being hydrogen. The peptide derivative VI may then be cyclized in solution in the presence of a weak base such as ammonium hydroxide. The group W is as described in Method A. The resulting crude I may then be purified as described above in Method A.

The purified I may be further transformed as described in Method A. Additionally and if desired, when X is NR₁₃ and R₁₃ is hydrogen, I may be acylated with, for example, acetyl chloride, acetic anhydride or benzoyl chloride, methanesulfonyl chloride or p-toluenesulfonyl chloride and the like.

### Method C

Intermediate VI may be prepared by the sequential coupling of amino acid derivatives in solution without the use of polymer resin or other solid supports. The methods useful for solution phase peptide synthesis are well documented in the chemical literature and are known to those skilled in the art (Houben-Weyl, Methoden der Organischen Chemie, 4th Edn., Vol. 15, Georg Thieme Verlag, Stuttgart 1974). The attached substituents R₁, R₉, R₁₅ and R₁₆ may be chosen such that they are transformable concurrently or sequentially as described in Methods A and B above. Cyclization of of VI wherein R₁₆ is H under conditions described above in Method B will provide compounds of Formula I.

The starting materials required for the processes described herein are known in the literature or can be prepared using known methods and known starting materials.

### Isomeric Products

In products of Formula I carbon atoms bonded to four nonidentical substituents are asymmetric. Accordingly, the compounds may exist as diastereoisomers, enantiomers or mixtures thereof. The syntheses described above may employ racemates, enantiomers or diastereomers as starting materials or intermediates. Diastereomeric products resulting from such syntheses may be separated by chromatographic or crystallization methods. Likewise, enantiomeric product mixtures may be separated using the same techniques or by other methods known in the art. Each of the asymmetric carbon atoms, when present in compounds of Formula I, may be in one of two configurations (R or S) and both are within the scope of the present invention. The carbon atoms bearing the (CH₂)ₙ sidechain and the (CH₂)ₘ sidechain are generally preferred to have the S configuration. The carbon atom bearing the substituents R₂ and R₃ is generally preferred to have a configuration corresponding to that of a D amino acid. The configuration may be assigned R or S depending on the chemical composition of R₂ and R₃.

The compounds described in this invention may be isolated as the free acid or base or converted to salts of various inorganic and organic acids and bases. Such salts are within the scope of this invention. Examples of such salts include ammonium, metal salts like sodium, potassium, calcium and magnesium; salts with organic bases like dicyclohexylamine, N-methyl-D-glucamine and the like; and salts with amino acids like arginine or lysine. Salts with inorganic and organic acids may be likewise prepared, for example, using hydrochloric, hydrobromic, sulfuric, phosphoric, trifluoroacetic, methanesulfonic, malic, maleic, fumaric and the like. Non-toxic and physiologically compatible salts are particularly useful although other less desirable salts may have use in the processes of isolation and purification.

A number of methods are useful for the preparation of the salts described above and are known to those skilled in the art. For example, reaction of the free acid or free base form of a compound of Formula I with one or more molar equivalents of the desired acid or base in a solvent or solvent mixture in which the salt is insoluble; or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

The compounds described in the present invention inhibit the binding of fibrinogen to its receptor on platelets, GP IIbIIIa, and thus prevent the aggregation of platelets and the formation of platelet plugs, emboli and thrombii in the circulatory system in mammals. Thromboembolic disorders have been shown to be directly related to the susceptibility of blood platelets to aggregate. Mammals exposed to medical procedures such as angioplasty and thrombolytic therapy are particularly susceptible to thrombus formation. The compounds of the present invention can be used to inhibit thrombus formation following angioplasty. They may also be used in combination with thrombolytic agents such as tissue plasminogen activator and its derivatives (US patents 4,752,603; 4,766,075; 4,777,043; EP 199,574; EP 0238,304; EP 228,862; EP 297,860; PCT WO89/04368; PCT WO89/00197), streptokinase and its derivatives, or urokinase and its derivatives to prevent arterial reocclusion following thrombolytic therapy. When used in combination with the above thrombolytic agents, the compounds of the present invention may be administered prior to, simultaneously with, or subsequent to the antithrombolytic agent Mammals exposed to renal dialysis, blood oxygenation, cardiac catheterization and similar medical procedures as well as mammals fitted with certain prosthetic devices are also susceptible to thromboembolic disorders. Physiologic conditions, with or without known cause may also lead to thromboembolic disorders. Thus, the compounds described herein are useful in treating thromboembolic disorders in mammals. The compounds described herein may also be used as adjuncts to anticoagulant therapy, for example in combination with aspirin, heparin or warfarin and other anticoagulant agents. The application of the compounds described herein for these and related disorders will be apparent to those skilled in the art

### Platelet Inhibition Assays

The evaluation of inhibitors of the fibrinogen-platelet interaction is guided by *in vitro* receptor binding assays and *in vitro* platelet aggregation inhibition assays.

In-vitro biological activity of the compounds of Formula I was monitored using a modified fibrinogen-GP IIbIIIa ELISA based on the method of Nachman and Leung (J. *Clin. Invest*. (1982) **69**, 263-269) which measures the inhibition of fibrinogen binding to purified human platelet GP IIbIIIa receptor. Human fibrinogen was prepared by the method of Lipinska, et al. (*J. Lab. Clin. Med.* (1974) **84**, 509-516). Platelet GP llbllla was prepared by the method of Fitzgerald, et al. *(Anal. Biochem.* (1985) **151**, 169-177.

Microtiter plates are coated with fibrinogen (10 µg/ml) and then blocked with TACTS buffer containing 0.5% bovine serum albumin (BSA). (TACTS buffer contains 20mM Tris.HCl, pH 7.5, 0.02% sodium azide, 2 mM calcium chloride, 0.05% Tween 20, 150 mM sodium chloride.) The plate is washed with phosphate buffered saline (PBS) containing 0.01% Tween 20 and the sample to be determined added, followed by addition of solubilized GP IIbIIIa receptor (40 µg/ml) in TACTS, 0.5% BSA. After incubation, the plate is washed and 1 µg/ml of murine anti-platelet monoclonal antibody AP3 (P. J. Newman et al. *Blood* (1985) **65**, 227-232) is added. After another wash a goat anti-mouse IgG conjugated to horseradish peroxidase is added. A final wash is performed and developing reagent buffer (10 mg o-phenylenediamine dihydrochloride, 0.0212% hydrogen peroxide, 0.22 mM citrate, 50 mM phosphate, pH 5.0) is added and then incubated until color develops. The reaction is stopped with 1N sulfuric acid and the absorbance at 492 nm is recorded.

In addition to the GP IIbIIIa ELISA assay, platelet aggregation assays may be performed in human platelet rich plasma (PRP). Fifty milliliters of whole human blood (9 parts) is drawn on 3.6% sodium citrate (1 part) from a donor who has not taken aspirin or related medications for at least two weeks. The blood is centrifuged at 160 x g for 10 min at 22°C and then allowed to stand for 5 min after which the PRP is decanted. Platelet poor plasma (PPP) is isolated from the remaining blood after centrifugation at 2000 x g for 25 min. The platelet count of the PRP was adjusted to ca. 300,000 per microliter with PPP.

A 225 µL aliquot of PRP plus 25 µL of either a dilution of the test sample or a control (PBS) is incubated for 5 min in a Chrono-log Whole Blood Aggregometer at 25°C. An aggregating agent (collagen, 1 mg/ml; U46619, 100 ng/ml; or ADP, 8 µM) is added and the platelet aggregation recorded.

In the management of thromboembolic disorders the compounds of this invention may be utilized in compositions such as tablets, capsules or elixers for oral administration; suppositories for rectal administration; sterile solutions or suspensions for injectable administration, and the like. Animals in need of treatment using compounds of this invention can be administered dosages that will provide optimal efficacy. The dose and method of administration will vary from animal to animal and be dependent upon such factors as weight, diet, concurrent medication and other factors which those skilled in the medical arts will recognize.

### Dosage Fomulations

Dosage formulations of the cyclic polypeptides of the present invention are prepared for storage or administration by mixing the the cyclic polypeptide having the desired degree of purity with physiologically acceptable carriers, excipients, or stabilizers. Such materials are non-toxic to the recipients at the dosages and concentrations employed, and include buffers such as phosphate, citrate, acetate and other organic acid salts; antioxdants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic add, aspartic acid, or arginine; monosaccharides, disaccharides, and other carbohydrates including cellulose or its derivatives, glucose, mannose, or dextrins; chelating agents such as EDTA; sugar alcohols such as mannitol or sobitol; counterions such as sodium and/or nonionic surfactants such as Tween, Pluronics or polyethyleneglycol.

Dosage formulations of the cyclic polypeptides of the present invention to be used for therapeutic administration must be sterile. Sterility is readily accomplished by filtration through sterile filtration membranes such as 0.2 microne membranes. Cyclic polypeptide formulations ordinarily will be stored in lyophilized form or as an aqueous solution. The pH of the cyclic polypeptide preparations typically will be between 3 and 11, more preferably from 5 to 9 and most preferably from 7 to 8. It will be understood that use of certain of the foregoing excipients, carriers, or stabilizers will result in the formation of cyclic polypeptide salts. While the preferred route of administration is by hypodermic injection needle, other methods of administration are also anticipated such as suppositories, aerosols, oral dosage formulations and topical formulations such as ointments, drops and dermal patches.

Therapeutic cyclic polypeptide formulations generally are placed into a container having a sterile access port, for example, an intravenous solution bag or vial having a stopper pierceable by hypodermic injection needle.

Therapeutically effective dosages may be determined by either *in vitro* or *in vivo* methods. One method of evaluating therapeutically effective dosages is illustrated in Example 23 where the cyclic polypeptide cyclo-S-acetyl-Gly-Arg-Gly-Asp-Cys-OH was determined to have a 50% inhibitory concentration (IC₅₀) of 5 nM when inhibiting fibrinogen binding to the GP IIbIIIa platelet receptor. Similarly, in a platelet aggregation assay in Example 24 using the same cyclic peptide, the IC₅₀ was found to be 8.5 µM. Based upon such *in vitro* assay techniques, a therapeutically effective dosage range may be determined. For each particular cyclic polypeptide of the present invention, individual determinations may be made to determine the optimal dosage required. The range of therapeutically effective dosages will naturally be influenced by the route of administration. For injection by hypodermic needle it may be assumed the dosage is delivered into the body's fluids. For other routes of administration, the absorption efficiency must be individually determined for each cyclic polypeptide by methods well known in pharmacology.

The range of therapeutic dosages is from about 0.001 nM to 1.0 mM, more preferably from 0.1 nM to 100 µM, and most preferably from 1.0 nM to 50 µM.

Typical formulation of compounds of Formula I as pharmaceutical compositions are discussed below.

About 0.5 to 500 mg of a compound or mixture of compounds of Formula I, as the free acid or base form or as a pharmaceutically acceptable salt, is compounded with a physiologically acceptable vehicle, carrier, excipient, binder, preservative, stabilizer, flavor, etc., as called for by accepted pharmaceutical practice. The amount of active ingredient in these compositions is such that a suitable dosage in the range indicated is obtained.

Typical adjuvants which may be incorporated into tablets, capsules and the like are a binder such as acacia, corn starch or gelatin; an excipient such as microcrystalline cellulose; a disintegrating agent like corn starch or alginic acid; a lubricant such as magnesium stearate; a sweetening agent such as sucrose or lactose; a flavoring agent such as peppermint wintergreen or cherry. When the dosage form is a capsule, in addition to the above materials it may also contain a liquid carrier such as a fatty oil. Other materials of various types may be used as coatings or as modifiers of the physical form of the dosage unit A syrup or elixer may contain the active compound, a sweetener such as sucrose, preservatives like propyl paraben, a coloring agent and a flavoring agent such as cherry. Sterile compositions for injection can be formulated according to conventional pharmaceutical practice. For example, dissolution or suspension of the active compound in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted phamaceutical practice.

### EXAMPLES

In the following Examples, common α-amino acids may be described by the standard three letter amino acid code when referring to intermediates and final products. By common α-amino acids is meant those amino acids incorporated into proteins under mRNA direction. Standard abbreviations are listed in **The Merck Index,** 10th Edition, pp Misc-2-Misc-3. Unless otherwise designated the common α-amino acids have the natural or "L"-configuration at the alpha carbon atom. If the code is preceded by a "D" this signifies the opposite enantiomer of the common α-amino add. Modified or unusual α-amino acids such as norleucine (Nle) and omithine (Om) are designated as described in U.S. Patent and Trademark Office Official Gazette 1114TMOG, May 15, 1990. If the product or intermediate name is preceded by "cyclo" this shall be taken to mean that the peptide has been cyclized, e.g. compounds of Formula I or V.

### Example 1

### Bromoacetyl-Gly-Arg-Gly-Asp-Cys-OH

The title compound is prepared in protected form by standard solid phase peptide synthesis on 2% cross-linked polystyrene resin (Merrifield resin). Treatment of the resin bound intermediate with liquid hydrogen fluoride induces concomitant cleavage of the protecting groups from the title compound as well as cleavage of the peptide from the resin. The crude peptide is purified by reverse phase high performance liquid chromatography (HPLC) using a 4.6 mm x 250 mm column containing 10 micron, 300 Angstrom pore size C-18 packing. The elution of the column is with an acetontrile/0.1% aqueous trifluoroacetic acid gradient going from 0% - 40% acetonitrile linearly over 80 minutes. The title compound elutes at 14 minutes. FAB mass spectrum: calc. 627; obs. 628 (M+1).

### Example 2

### Cyclo-S-acetyl-Gly-Arg-Gly-Asp-Cys-OH

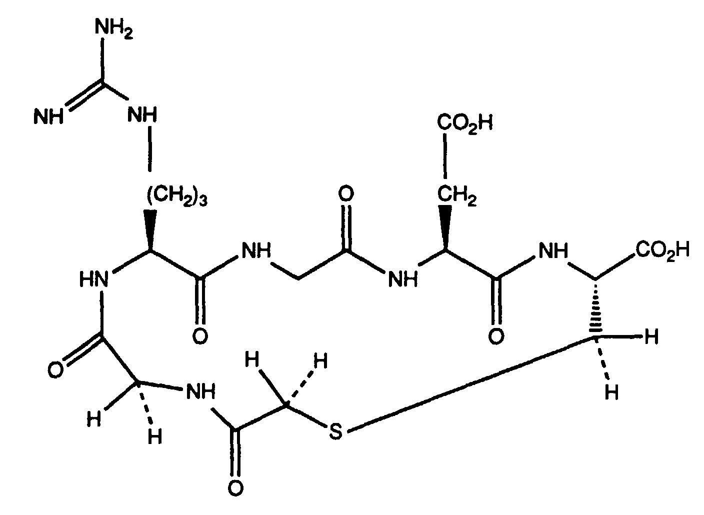

The compound prepared in Example 1 is dissolved in deionized water (1 mg/ml) and the pH of the solution is adjusted to 7.0-8.5 with ammonium hydroxide. After stirring for 4 hr at ambient temperature the reaction solution is acidified to pH 3.0 - 3.5 with trifluoroacetic acid and then lyophilized. The resulting crude product is purified by HPLC using the conditions described in Example 1. The desired title compound elutes after 11 minutes. FAB mass spectrum: calc. 546; obs. 547 (M+1). Amino acid analysis: carboxymethyl-cys, 0.96; Asp, 1.04; Gly, 2.12; Arg, 0.94. 1H NMR(300 MHz D₂O, pH 7): 4.75, m; 4.3-4.4, m; 4.15, d; 4.0, ab q; 3.9, d; 3.4, ab q; 3.2, t; 2.95-3.15, m; 2.7, dq; 1.75-2.05, m; 1.55-1.7,m.

### Example 3

### Cyclo-S-acetyl-Gly-Arg-Gly-Asp-Cys-OH

Bromoacetyl-Gly-Arg(g-2,2,5,7,8-pentamethylchroman-6-sulfonyl)-Gly-Asp(beta-t-butyl)-Cys(S-triphenylmethyl)-O-(polymer resin) is prepared using standard solid phase peptide synthesis utilizing fluorenylmethoxycarbonyl (FMOC) protecting group chemistry on a p-alkoxybenzyl alcohol resin. Repeated treatment of the resin bound peptide with a 1% solution of trifluoroacetic acid in dichloromethane results in cleavage of the S-triphenylmethyl group as evidenced by the bright yellow of the solution. Treatment is continued until dissipation of the yellow color (ca. 1.5 L of the cleavage solution is required per gram of resin bound peptide.) After complete cleavage of the S-triphenylmethyl group, the resin bound peptide is washed several times with a 5% solution of N-methylmorpholine in N,N-dimethylacetamide and then shaken in pure N,N-dimethylacetamide for 12 hr to complete the cyclization. Treatment of the cyclized resin bound peptide with trifluoroacetic acid containing (v/v) 1% phenol, 1% anisole and 1% ethanedithiol effects concomitant cleavage of the remaining protective groups and cleavage of the desired product from the resin. Purification of the crude product as described in Example 2 affords the title compound identical to that described above.

### Example 4

### Synthesis of Compounds of Formula VII

Using the methods described in Examples 1 and 2, the compounds listed in Table I may be prepared. The compounds are depicted by Formula VII wherein m = 1 and n = 3, R₁ and R₉ are OH, R₁₄ is hydrogen and X is S. Crude products are purified using HPLC as described in Example 2.

The following amino acid derivatives may be used in place of Boc-Gly for coupling to the alpha-amine group of Arg to obtain the substituents R₂ and R₃ shown in Table I. When Boc-Glycine is used, R₂ and R₃ are both hydrogen (H).

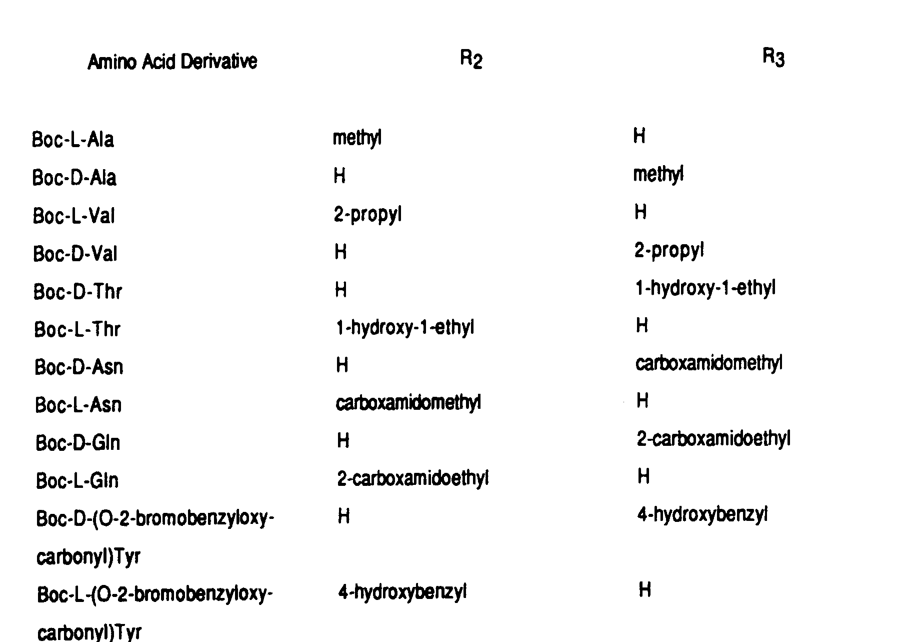

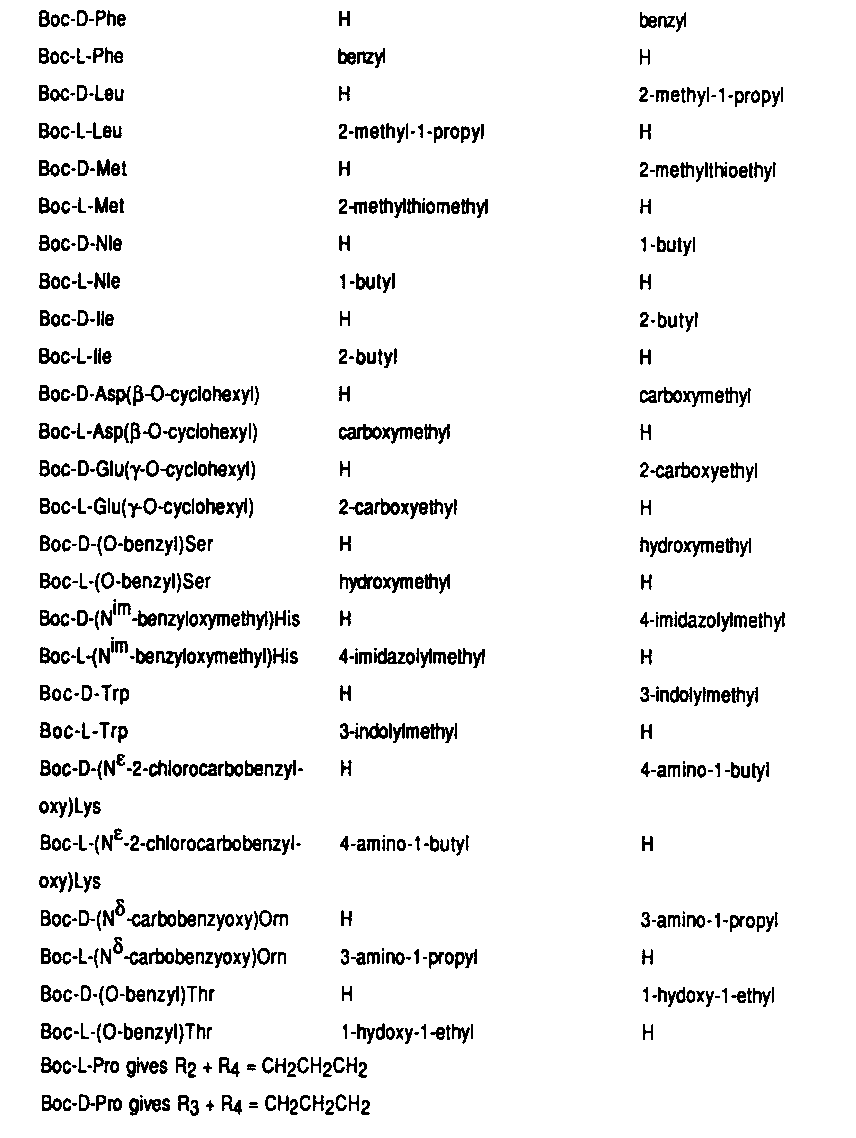

The substituted bromoacetic acids listed below may be used in place of bromoacetic acid in Example 1 and in combination with the amino acid derivatives listed above to provide the compounds shown in Table 1 with variable substitutents at R₅, R₆. When bromoacetic acid is used in combination with the amino acid derivatives listed above R₅ and R₆ are hydrogen (H).
1-naphthyl-α-bromoacetic acid
2-naphthyl-α-bromoacetic acid
phenyl-α-bromoacetic acid
2-trifluromethylphenyl-α-bromoacetic acid
3-trifluromethyfphenyl-α-bromoacetic acid
4-trifluromethyfphenyl-α-bromoacetic acid
4-biphenyl-α-bromoacetic acid
2-bromopropionic acid
2-bromobutyric acid
2-bromopentanoic add

L.Pennicillamine may be substituted for L-cysteine in Example 1 to produce compounds in Table 1 where R₇ and R₈ are methyl.

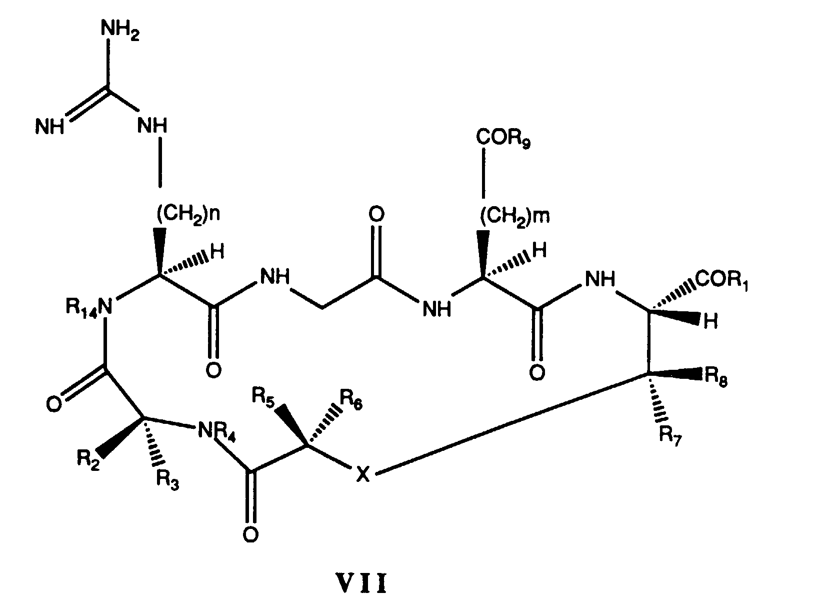

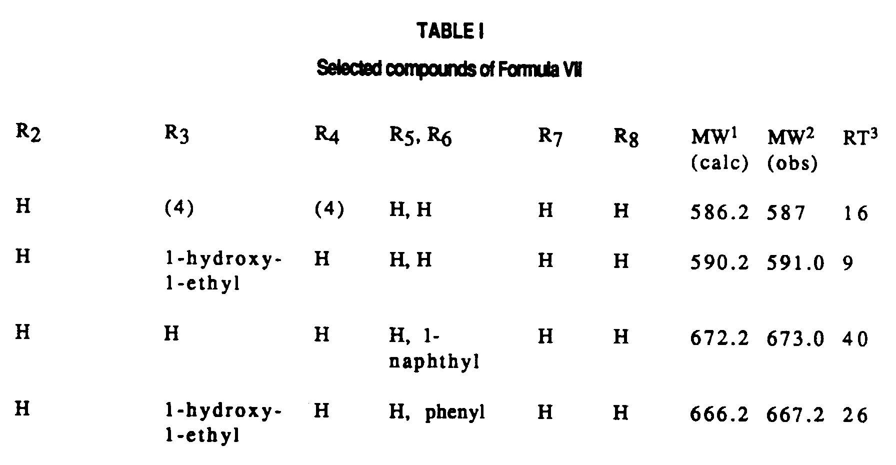

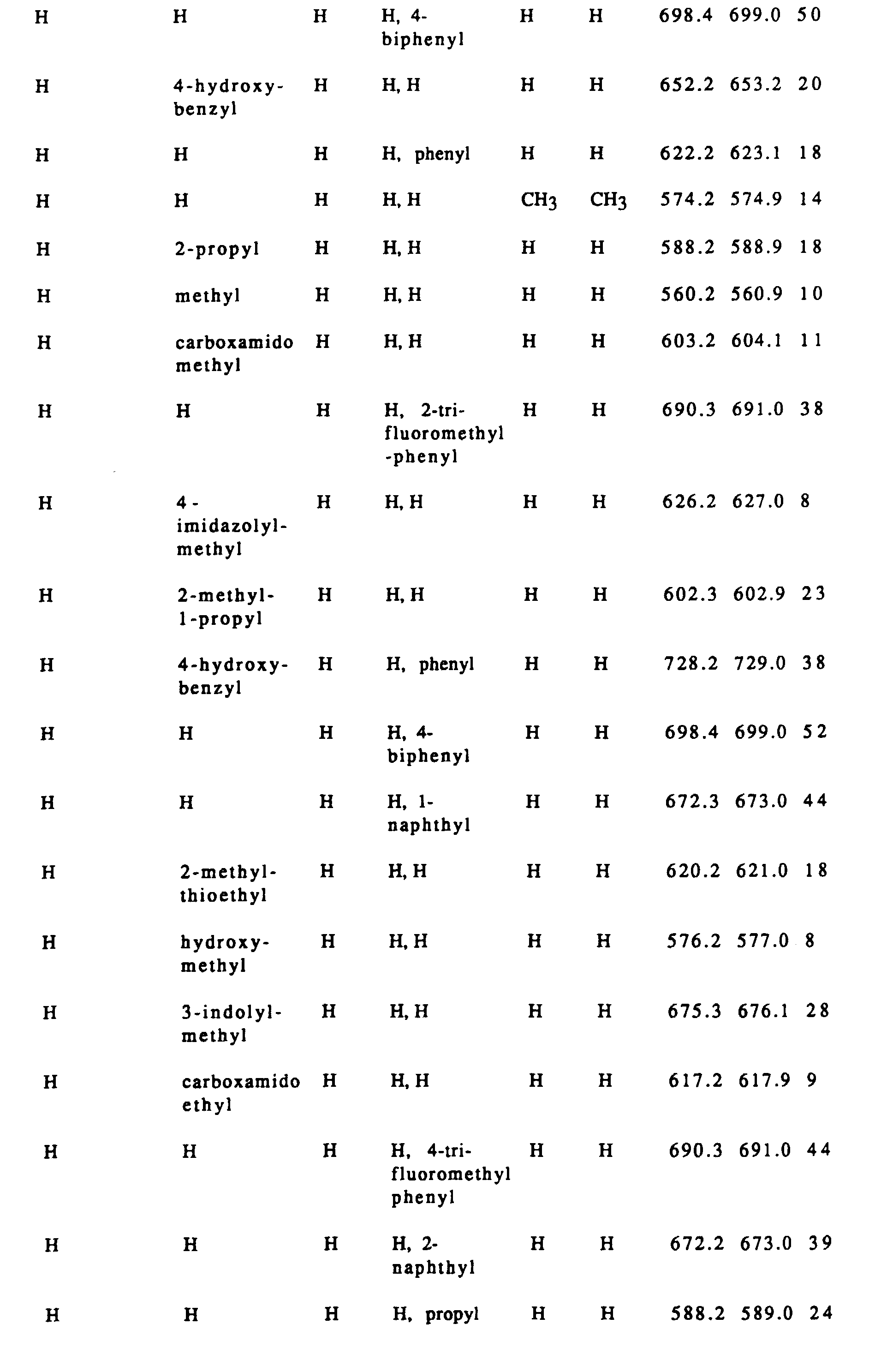

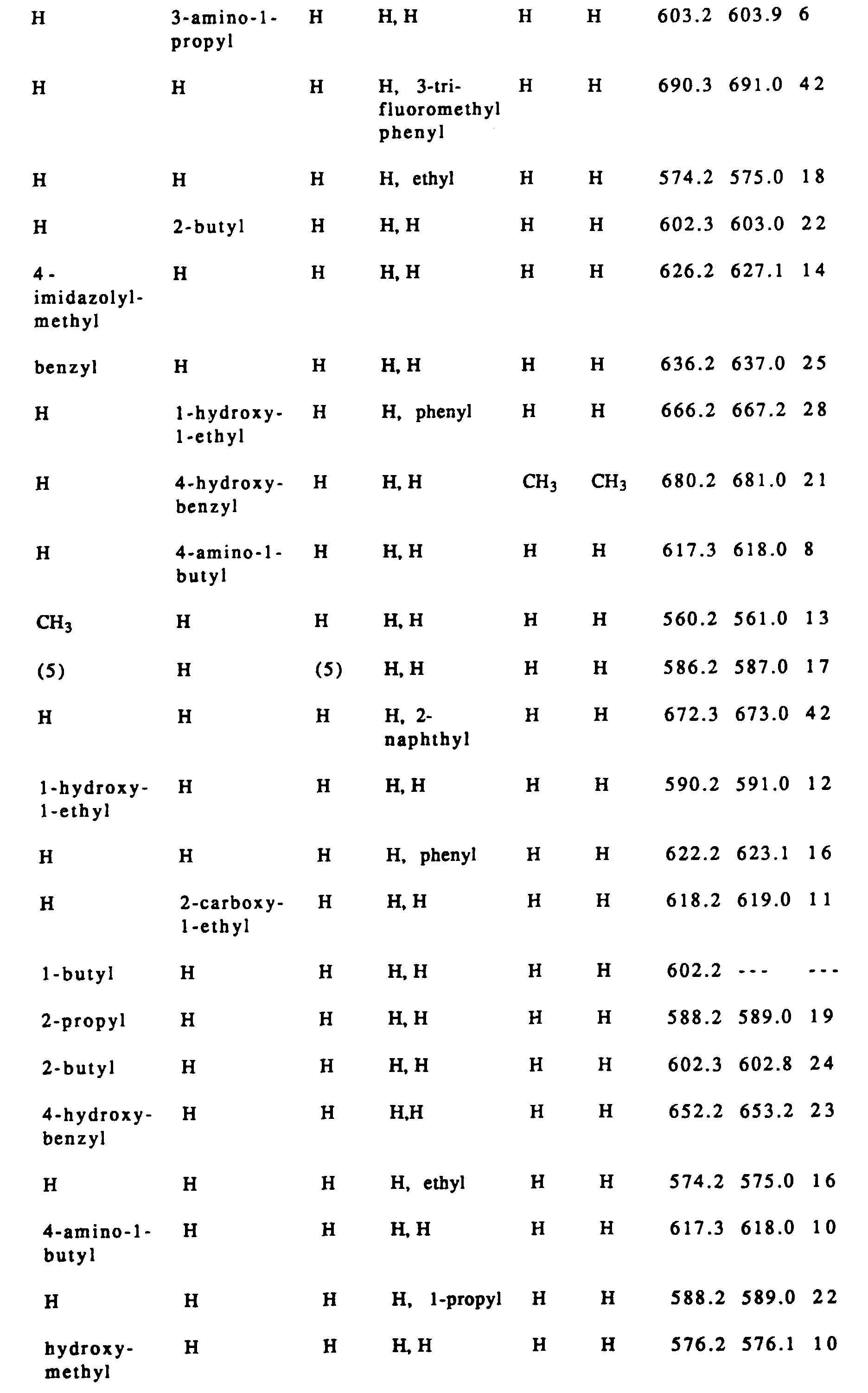

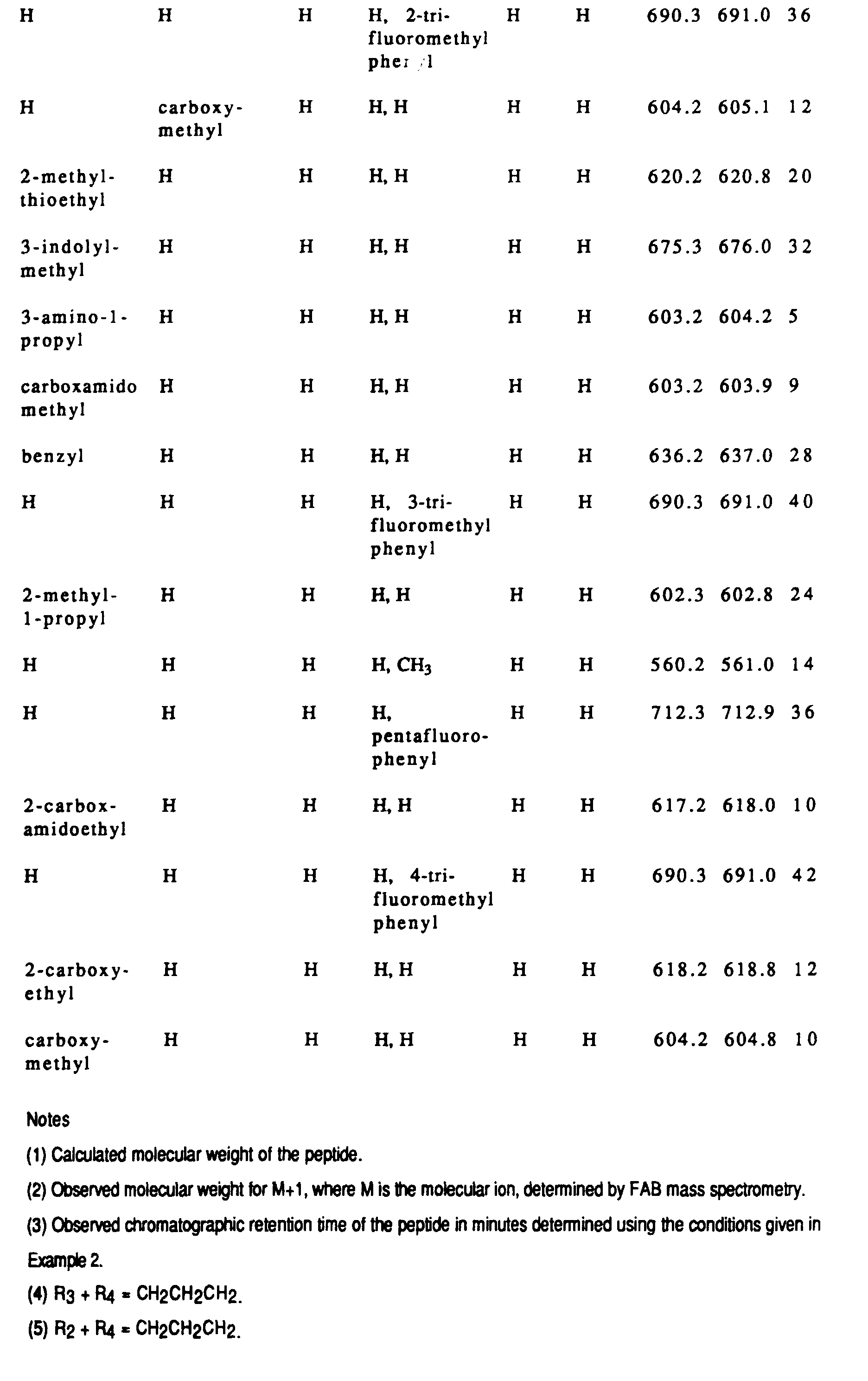

### Example 5

### N-(N-t-Butoxycarbonyl(N^{g}-p-toluenesulfonyl)arginyl)glycine

N-t-Butoxycarbonyl-N^{g}-p-toluenesulfonylarginine (10 mmoles), benzotriazol-1-yloxytris(dimethylamino)phosphonium hexafluorophosphate (BOP, 10 mmoles), ethyl glycinate hydrochloride (13 mmoles) and N-methylmorpholine (50 mmoles) are stirred in a mixture of dichloromethane and N,N-dimethylacetamide (1:1, 50 ml) for 2 hr at room temperature. At the end of the reaction time the mixture is acidified with acetic acid, concentrated in vacuo, and the residue taken up in ethyl acetate. The resulting solution is washed with 10% citric acid (3 times), water (2 times), saturated sodium bicarbonate (3 times), water (2 times) and brine (2 times). The organic solution is dried over magnesium sulfate, filtered, dried and concentrated to afford the crude ethyl ester of the title compound. Crystalline material can be obtained from ethyl acetate-hexane mixtures.

The dipeptide ethyl ester (10 mmoles) is treated with 10.5 mmoles of sodium hydroxide in ethanol and water (4:1, 50 ml) for 1 hr. at room temperature. Water (100 ml) is added and the mixture concentrated to ca 50 ml, acidified with citric acid, and extracted with ethyl acetate. After drying the extract over magnesium sulfate, concentration affords the desired title compound. It may be recrystallized from ethyl acetate-hexane mixtures.

### Example 6

### N-(N-t-buboxycarbonyl(beta-cyclohexyl ester)aspartyl) cysteine (S-4-methylbenzyl)benzyl ester

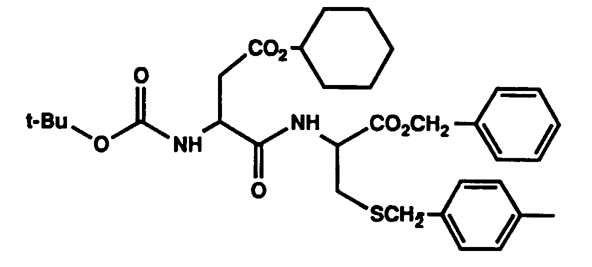

N-t-Butoxycarbonyl(beta-cyclohexyl ester)aspartic acid (10 mmole), BOP reagent (10 mmole), and N-methylmorpholine (15 mmole) are stirred in N,N-dimethylacetamide (50 ml) at room temperature for 10 min. To the resulting solution is added a solution of cysteine(S-4-methylbenzyl) benzyl ester trifluoroacetate (10 mmole) and N-methylmorpholine (15 mmole) in N,N-dimethylacetamide (15 ml). The resulting reaction mixture is stirred overnight at room temperature. The solvent is then removed in vacuo and the reaction residue worked up as described in Example 5 to afford the desired title compound.

### Example 7

### N-(N-(N-t-Butoxycarbonyl(N^{g}-p-toluensulfonyl)arginyl)glycinyl)aspartyl(beta-cyclohexylester))cysteine(S-4-methylbenzyl)benzyl ester

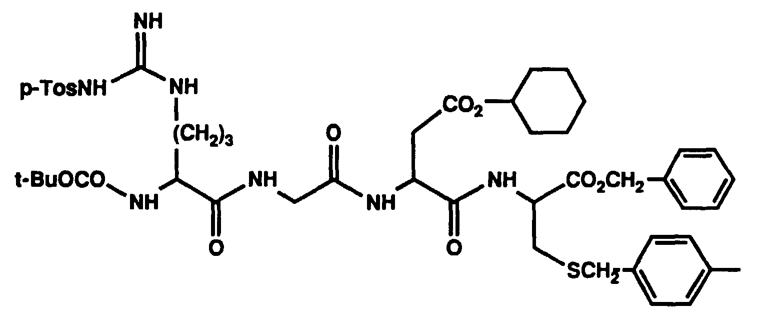

N-(N-t-butoxycarbonyl(beta-cyclohexyl ester)aspartyl) cysteine (S-4-methylbenzyl) benzyl ester from Example 6 (10 mmole) is treated with trifluoroacetic acid in dichloromethane (1:1, 200 ml) for 40 min at room temperature and then concentrated. The resulting solid is washed with ether and dried.

The above solid (10 mmole), BOP reagent (10 mmole), N-methylmorpholine (40 mmole) and N-(N-t-Butoxycarbonyl(N^{g}-p-toluenesulfonyl)arginyl)glycine from Example 22 (10 mmole) are stirred in N,N-dimethylacetamide (50 ml) for 4 hr. After the reaction the solution is concentrated and the residue taken up in ethyl acetate and worked up as described in Example 5. The crude product thus obtained is crystallized from chloroform-hexane.

### Example 8

### N-(N-(N-(N-(N-t-Butoxycarbonyl(O-benzyl)-D-threonyl)(N^{g}-p-toluenesulfonyl)arginyl)glycinyl)aspartyl(beta-cyclohexyl ester))cysteine(S-4-methylbenzyl)benzyl ester

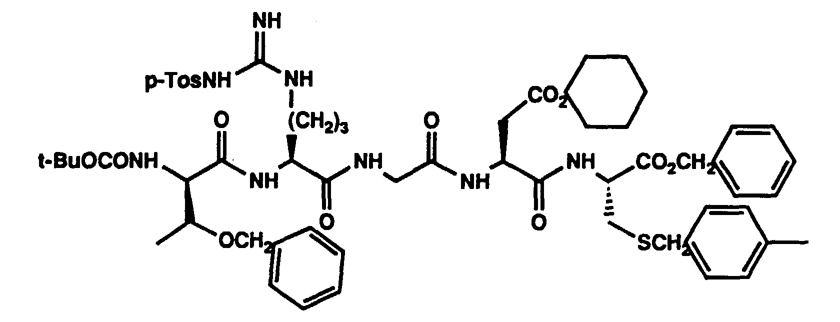

The t-butoxycarbonyl group is removed from N-(N-(N-(N-t-butoxycarbonyl(N^{g}-p-toluenesulfonyl)arginyl)glycinyl)aspartyl(beta-cyclohexyl ester))cysteine(S-4-methylbenzyl) benzyl ester using the procedure described in Example 7 to afford the trifluoroacetate salt of the tetrapeptide.

The above salt is coupled with N-t-butoxycarbonyl(O-benzyl)-D-threonine using the procedure described in Example 7. The desired title compound is isolated after workup as described above.

### Example 9

### Cyclo-S-acetyl-(D-Thr)-Arg-Gly-Asp-Cys-OH

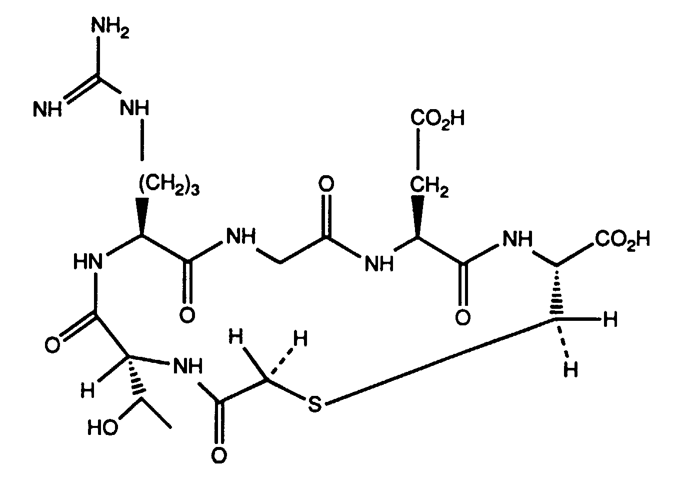

The t-butoxycarbonyl group is removed from N-(N-(N-(N-(N-t-Butoxycarbonyl(O-benzyl)-D-threonyl)(N^{g}-p-toluenesulfonyl)arginyl) glycinyl)aspartyl(beta-cyclohexyl ester))cysteine(S-4-methylbenzyl) benzyl ester using the procedure described in Example 7 to afford the trifluoroacetate salt of the pentapeptide.

Bromoacetic acid (4 mmole) and dicyclohexylcarbodiimide (2 mmole) are stirred together in 5 ml of dichloromethane for 10 min. The solution is filtered and added to the pentapeptide trifluoroacetate prepared above (1 mmole) and N-methylmorpholine (3 mmole) in N,N-dimethylacetamide (5 ml). After stirring for 1 hr at room temperature the reaction is concentrated in vacuo and the residue taken up in ethyl acetate and worked up as described in Example 5. The residue isolated from the ethyl acetate solution is treated directly with a mixture of hydrogen fluoride, anisole and methylethyl sulfide (90:5:5) at 0 C for 1 hr. After removal of the hydrogen fluoride the crude material is dissolved in 10% acetic acid and lyophilized. The isolated powder is then dissolved in water at a concentration of 1 mg/ml and the pH adjusted to 7.3 with ammonium hydroxide. After stirring at ambient temperature for 4 hr the reaction solution is loaded onto a DEAE 52 cellulose column equilibrated in 5 mM ammonium acetate at pH 7.3. The desired title compound is eluted with 45 mM ammonium acetate at pH 7.3. The fractions containing the desired cyclized peptide are desalted immediately by acidification to pH 4 with acetic acid and chromatography over a C-18 reverse phase column. The loaded column is first washed with 0.1% aqueous trifluoroacetic acid followed by linear gradient with 0.1% trifluoroacetic add in acetonitrile. The purified peptide, after lyophilization, is identical with the product described in Example 7.

### Example 10

### Method for Preparation of Other Compounds of Formula VII

Using the methods described in Examples 5-9, the replacement amino acids shown below may be used in place of D-threonine in Example 8 and the replacement bromo acids listed below may be used in place of bromoacetic acid in Example 9 to produce the compounds listed in Table 2. In this example, R₁ and R₉ are OH, R₁₄ is hydrogen, and X is S. The benzyl ester of S-(4-methylbenzyl)-L-pennicillamine may be used in place of the corresponding cysteine derivative in Example 5 to produce compounds in which R₇ and R₈ are methyl.

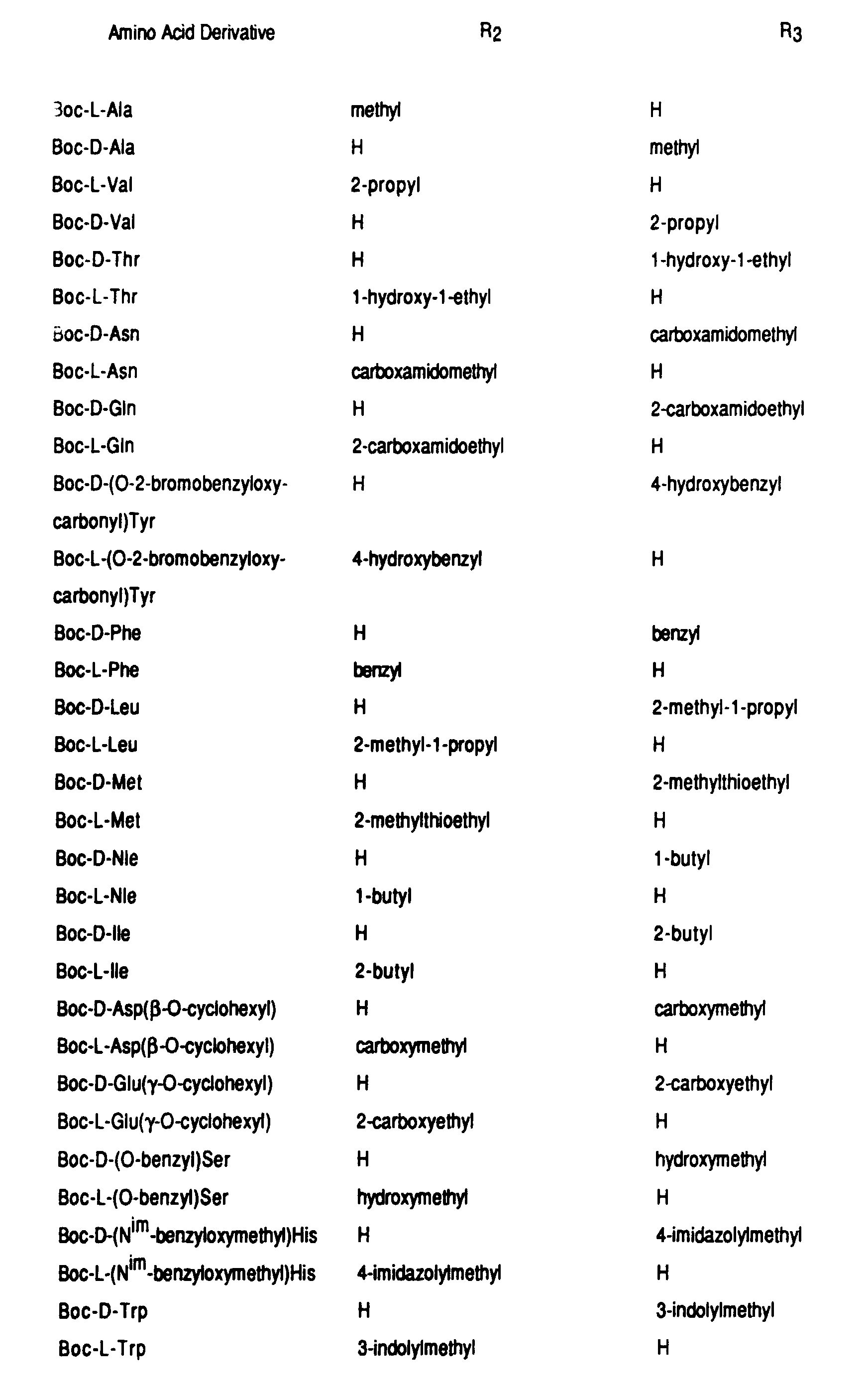

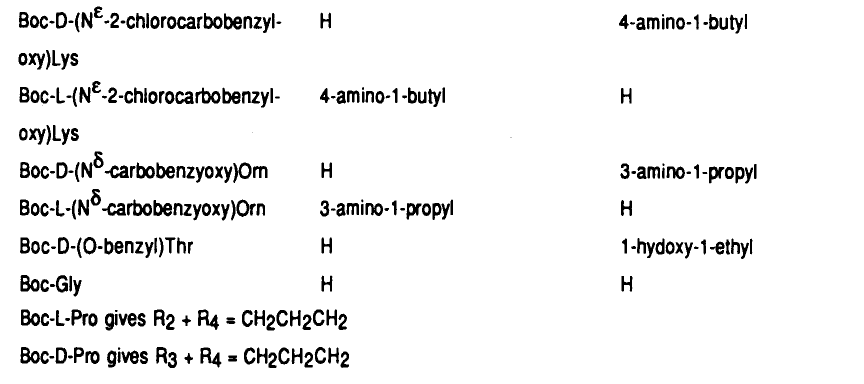

Replacement bromo acids
1-naphthyl-α-bromoacetic acid
2-naphthyl-α-bromoacetic acid
phenyl-α-bromoacetic acid
2-trifluromethylphenyl-α-bromoacetic acid
3-trifluromethylphenyl-α-bomoacetic acid
4-trifluromethylphenyl-α-bromoacetic acid
4-biphenyl-α-bromoacefic acid
2-bromopropionic acid
2-bromobutyric acid
2-bromopentanoic acid
When bromoacetic acid is used in combination with the amino acid derivatives listed above R₅ and R₆ are hydrogen (H).

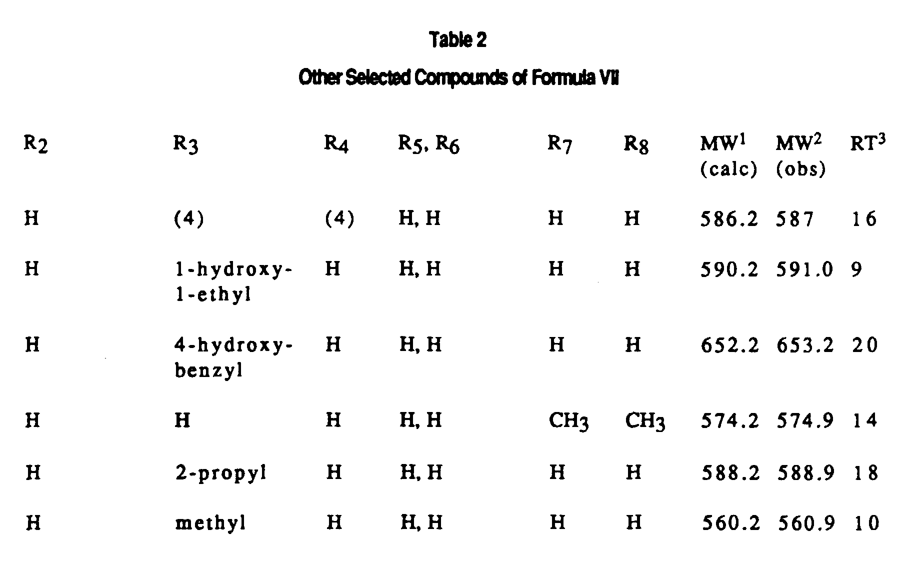

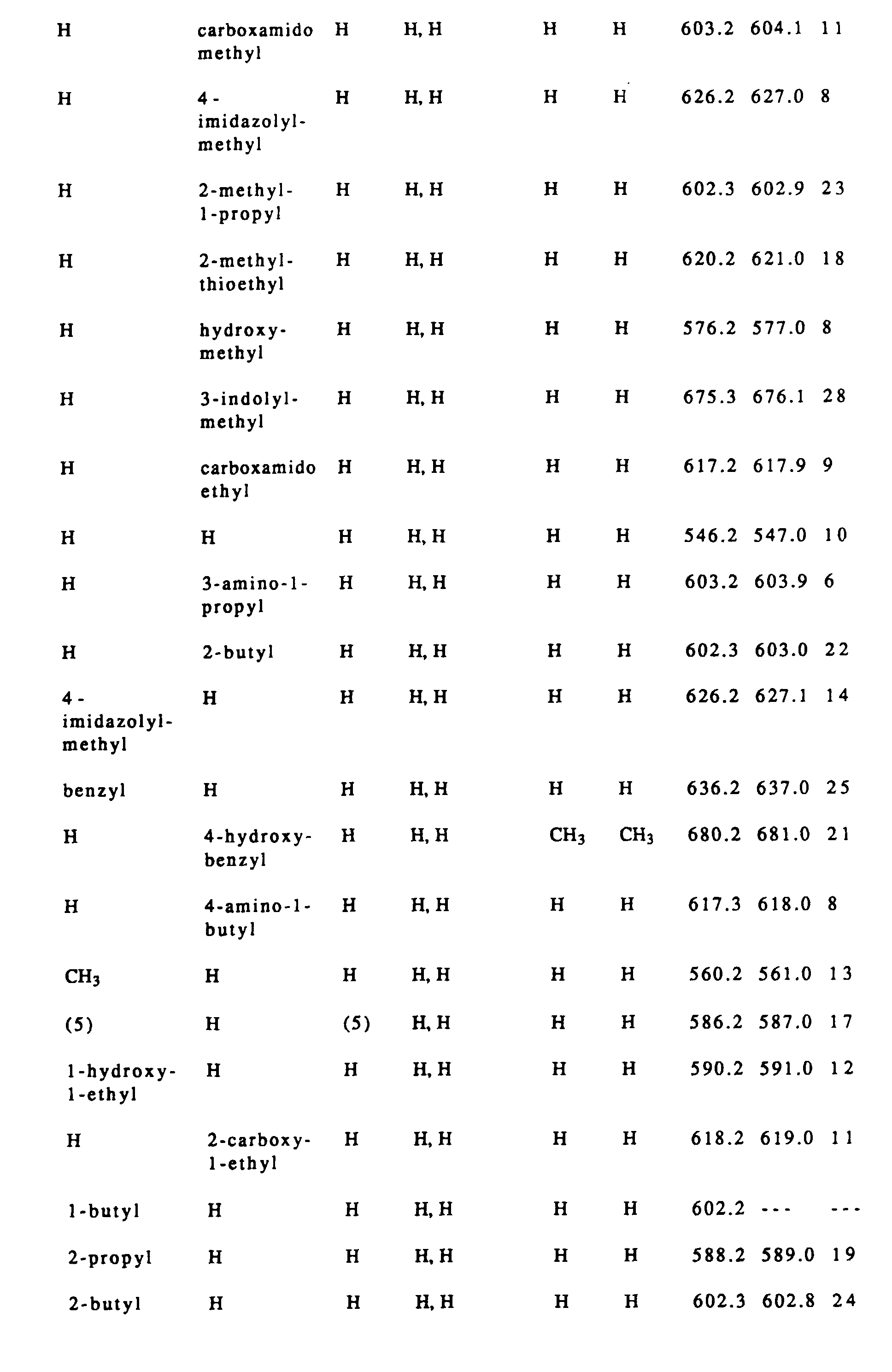

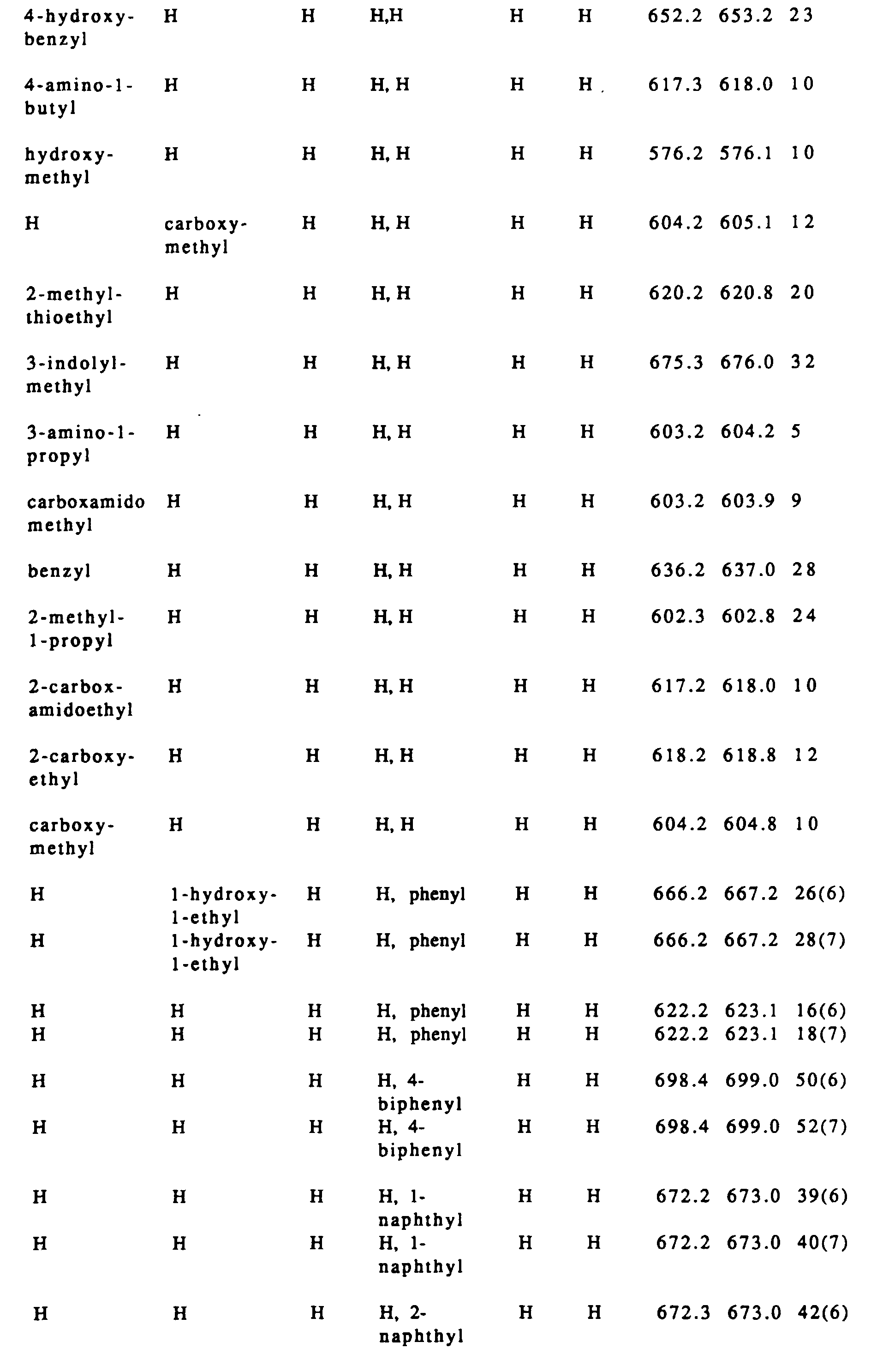

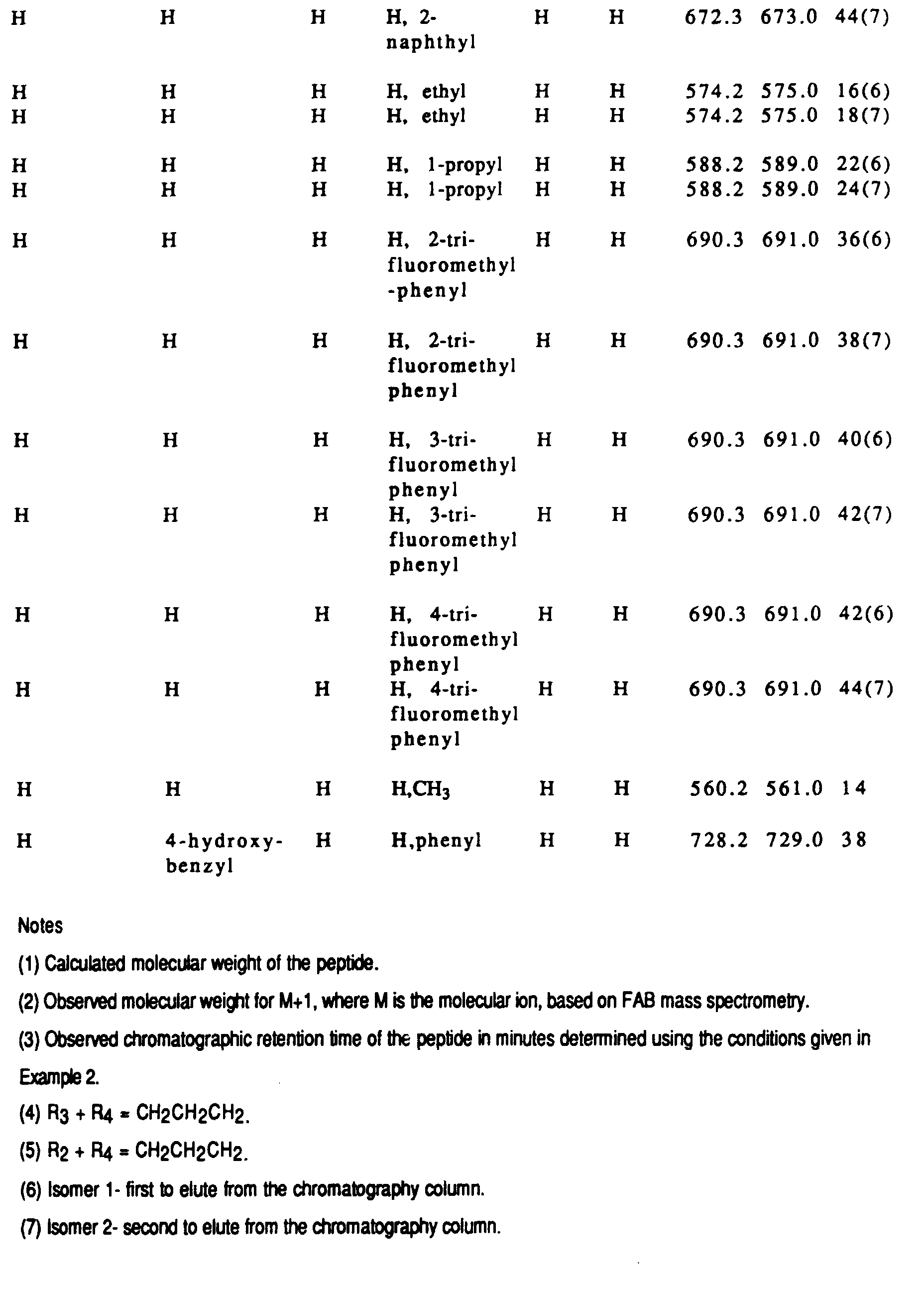

### Example 11

### Cyclo-S-acetyl-(D-Tyr)-Arg-Gly-Asp-Cys-OC₂H₅

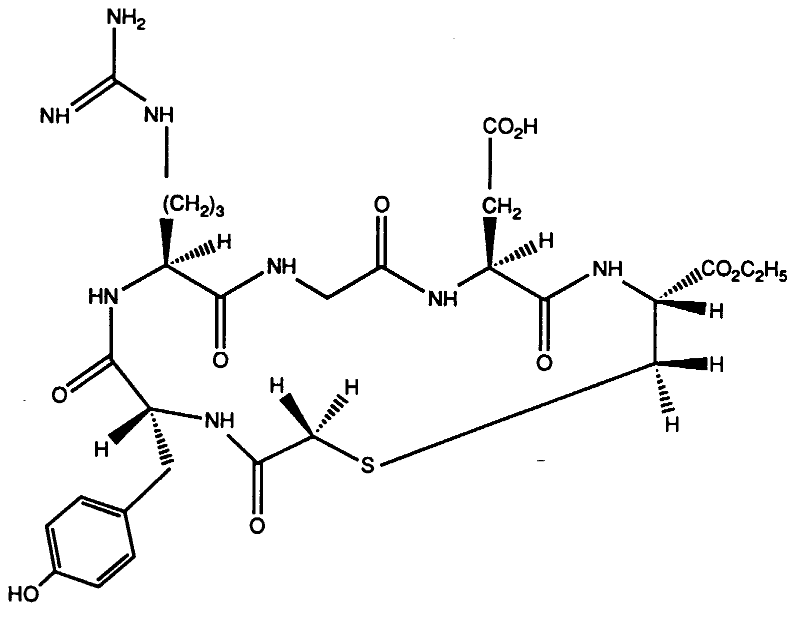

Using the procedures described in Examples 5-9 and replacing cysteine(S-4-methylbenzyl)benzyl ester trifluoroacetate with the corresponding ethyl ester the title compound shown above is obtained. HPLC retention time: 30 min. Molecular weight (calc.): 680.2. Found (FAB): 681.0 (M+1).

### Example 12

### Cyclo-S-acetyl-(D-Tyr)-Arg-Gly-Asp(OC₂H₅)-Cys-OH

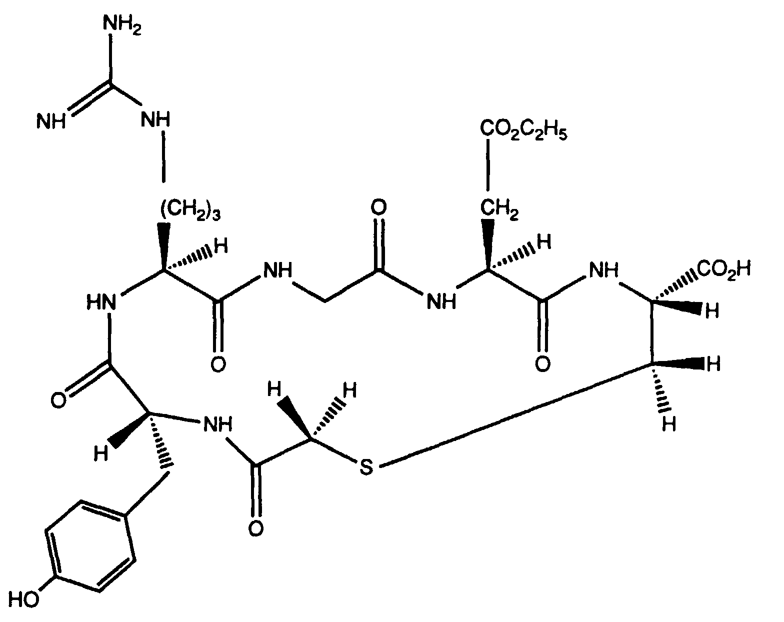

Using the procedures described in Examples 5-9 and replacing N-t-butoxycarbonyl(beta-cyclohexyl ester)aspartic acid with the corresponding beta-ethyl ester the title compound shown above is obtained. HPLC retention time: 26 min. Molecular weight (calc.): 680.2. Found (FAB): 681.2 (M+1).

### Example 13

### Cyclo-S-acetyl-(D-Tyr)-Arg-Gly-Asp(OC₂H₅)-Cys-OC₂H₅

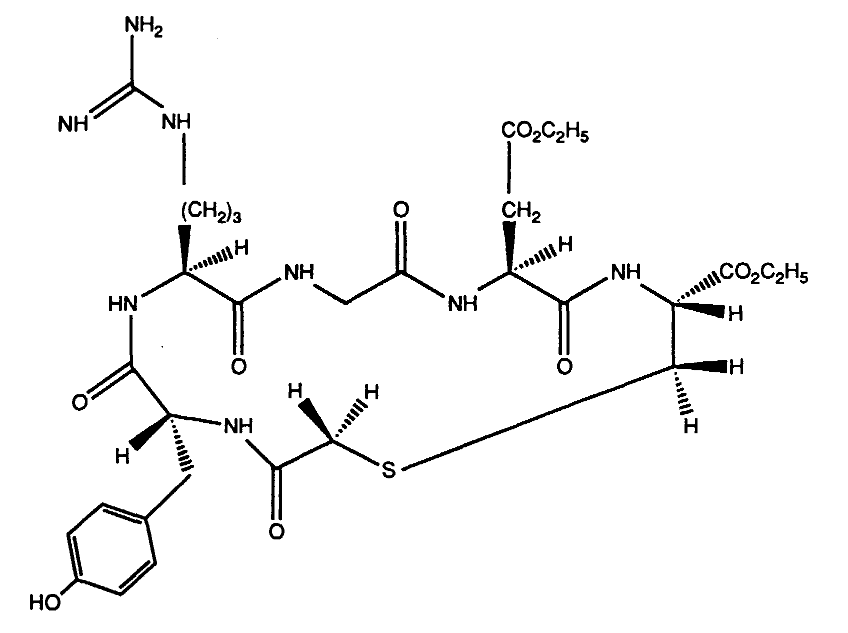

Using the procedures described in Examples 5-9 and replacing cysteine(S-4-methylbenzyl) benzyl ester trifluoroacetate with the corresponding ethyl ester and N-t-butoxycarbonyl(beta-cyclohexyl ester)aspartic acid with the corresponding beta-ethyl ester the title compound shown above is obtained. HPLC retention time: 40 min. Molecular weight (calc.): 709.2. Found (FAB): 710.1 (M+1).

### Example 14

### Preparation of cyclic thioether peptide esters

Using the methods described in Examples 11-13 above, the following cyclized peptide esters are analogously prepared.
Cyclo-S-acetyl-Gly-Arg-Gly-Asp-Cys-OC₂H₅
Cyclo-S-acetyl-Gly-Arg-Gly-Asp(OC₂H₅)-Cys-OH
Cyclo-S-acetyl-Gly-Arg-Gly-Asp(OC₂H₅)-Cys-OC₂H₅
Cyclo-S-acetyl-(D-Ala)-Arg-Gly-Asp-Cys-OC₂H₅
Cyclo-S-acetyl-(D-Ala)-Arg-Gly-Asp(OC₂H₅)-Cys-OH
Cyclo-S-acetyl-(D-Ala)-Arg-Gly-Asp(OC₂H₅)-Cys-OC₂H₅
Cyclo-S-acetyl-(D-Val)-Arg-Gly-Asp-Cys-OC₂H₅
Cyclo-S-acelyl-(D-Val)-Arg-Gly-Asp(OC₂H₅)-Cys-OH
Cyclo-S-acetyl-(D-Val)-Arg-Gly-Asp(OC₂H₅)-Cys-OC₂H₅
Cyclo-S-acetyl-(D-Leu)-Arg-Gly-Asp-Cys-OC₂H₅
Cyclo-S-acetyl-(D-Leu)-Arg-Gly-Asp(OC₂H₅)-Cys-OH
Cyclo-S-acetyl-(D-Leu)-Arg-Gly-Asp(OC₂H₅)-Cys-OC₂H₅
Cyclo-S-acetyl-(D-Ile)-Arg-Gly-Asp-Cys-OC₂H₅
Cyclo-S-acetyl-(D-Ile)-Arg-Gly-Asp(OC₂H₅)-Cys-OH
Cyclo-S-acetyl-(D-Ile)-Arg-Gly-Asp(OC₂H₅)-Cys-OC₂H₅
Cyclo-S-acetyl-(D-Phe)-Arg-Gly-Asp-Cys-OC₂H₅
Cyclo-S-acetyl-(D-Phe)-Arg-Gly-Asp(OC₂H₅)-Cys-OH
Cyclo-S-acetyl-(D-Phe)-Arg-Gly-Asp(OC₂H₅)-Cys-OC₂H₅
Cyclo-S-acetyl-(D-Thr)-Arg-Gly-Asp-Cys-OC₂H₅
Cyclo-S-acetyl-(D-Thr)-Arg-Gly-Asp(OC₂H₅)-Cys-OH
Cyclo-S-acetyl-(D-Thr)-Arg-Gly-Asp(OC₂H₅)-Cys-OC₂H₅
Cyclo-S-acetyl-(D-Pro)-Arg-Gly-Asp-Cys-OC₂H₅
Cyclo-S-acetyl-(D-Pro)-Arg-Gly-Asp(OC₂H₅)-Cys-OH
Cyclo-S-acetyl-(D-Pro)-Arg-Gly-Asp(OC₂H₅)-Cys-OC₂H₅

### Example 15

### Cyclo-S-acetyl-(D-Tyr)-Arg-Gly-Asp-Cys-NH₂

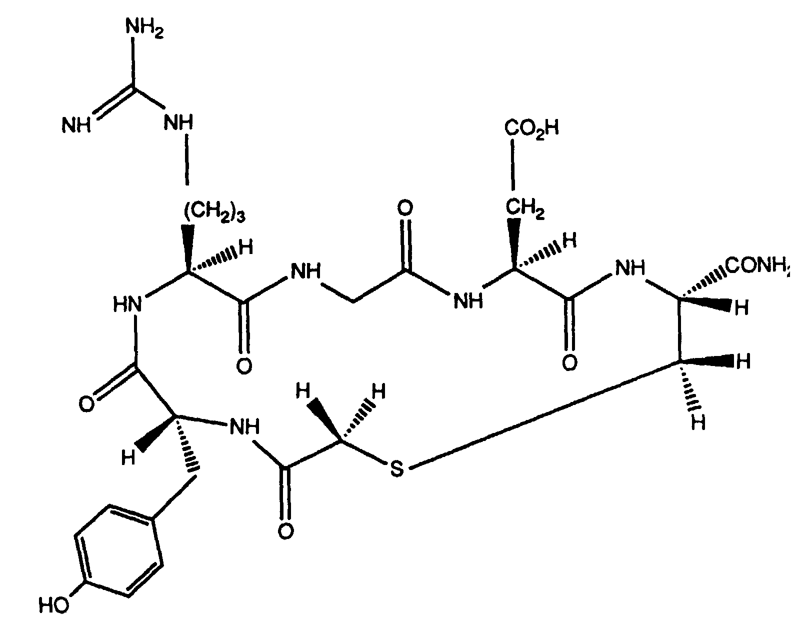

Synthesis of the title compound was accomplished using standard Boc-synthetic protocols on a 4-methylbenzylhydrylamine resin to obtain first a linear peptide as described in Example 1. Cleavage of the linear peptide from the resin with hydrogen fluoride followed by cyclization as described in Example 2 affords the title compound after HPLC purification. HPLC retention time: 18 minutes. Molecular weight. Calc.: 651.2. Obs. (FAB mass spectrum): 652.2 (M+1).

Using this procedure the following compounds are analogously obtained.
Cyclo-S-acetyl-(D-Ala)-Arg-Gly-Asp-Cys-NH₂
Cyclo-S-acetyl-(D-Val)-Arg-Gly-Asp-Cys-NH₂
Cyclo-S-acetyl-(D-Leu)-Arg-Gly-Asp-Cys-NH₂
Cyclo-S-acetyl-(D-Ile)-Arg-Gly-Asp-Cys-NH₂
Cyclo-S-acetyl-(D-Phe)-Arg-Gly-Asp-Cys-NH₂
Cyclo-S-acetyl-(D-Pro)-Arg-Gly-Asp-Cys-NH₂
Cyclo-S-acetyl-Gly-Arg-Gly-Asp-Cys-NH₂

### Example 16

### Cyclo-S-acetyl-Gly-Arg-Gly-Asp-Cys-OH sulfoxide

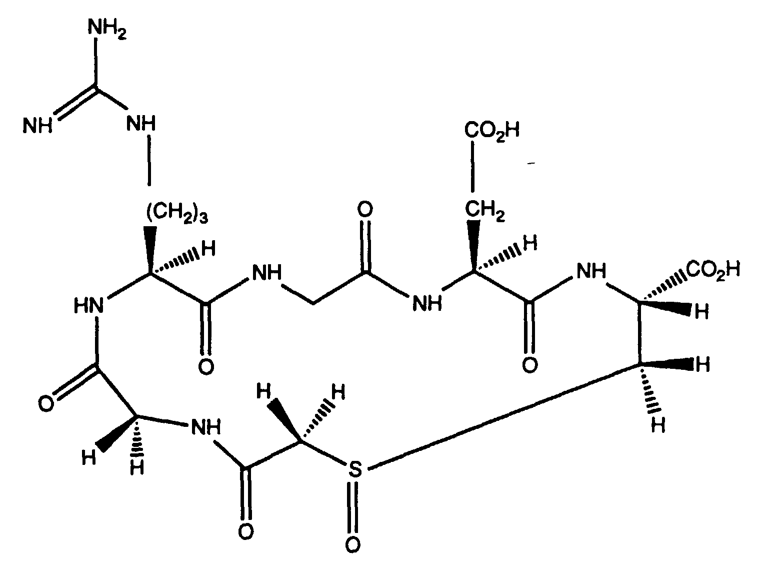

The purified product from Example 2 was dissolved in water at a concentration of 10 mg per mL. The pH of the solution was adjusted to 7. A 50% solution of hydrogen peroxide was added to make a final concentration of 3% hydrogen peroxide and the resulting reaction mixture was stirred overnight at room temperature. The solution was loaded directly onto an octadecylsilyl reverse phase chromatography column. The sulfoxide isomers formed in the reaction were eluted with a linear gradient of acetonitrile in 1% trifluoroacetic acid in water. Isomer 1 eluted at 3.5 minutes. Isomer 2 eluted at 4 minutes.

For both isomers: Molecular weight (calc.) 562.2. Found (FAB mass spectrum): isomer 1: 563.3; isomer 2: 563.3 (M+1).

### Example 17

### Preparation of cyclic thioether sulfoxides

Application of the procedure of Example 16 to thioether peptides prepared in Example 4 and shown in Table 1 and in to peptides prepared by the in Example 15 affords the sulfoxide stereoisomers of Formula VIII listed in Table 3 where R₁₄ is hydrogen, R₉ is OH, and X is SO.

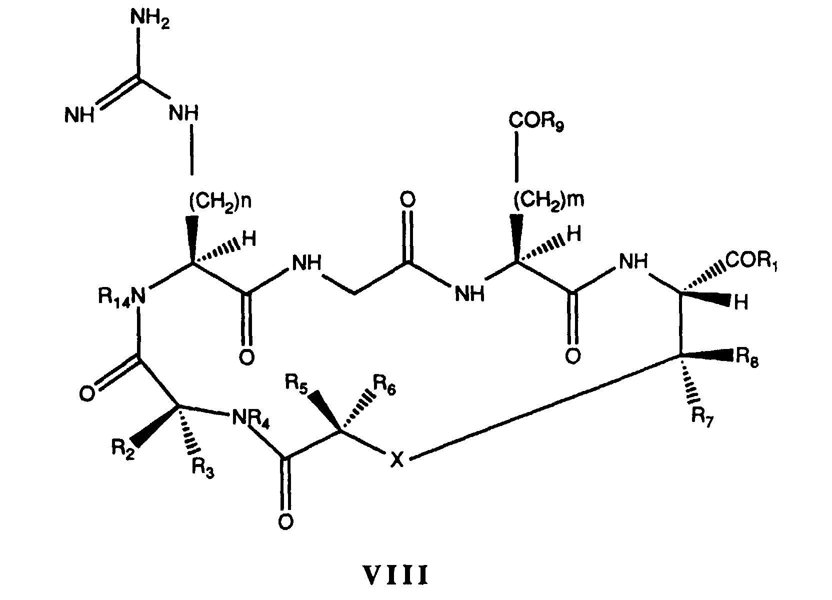

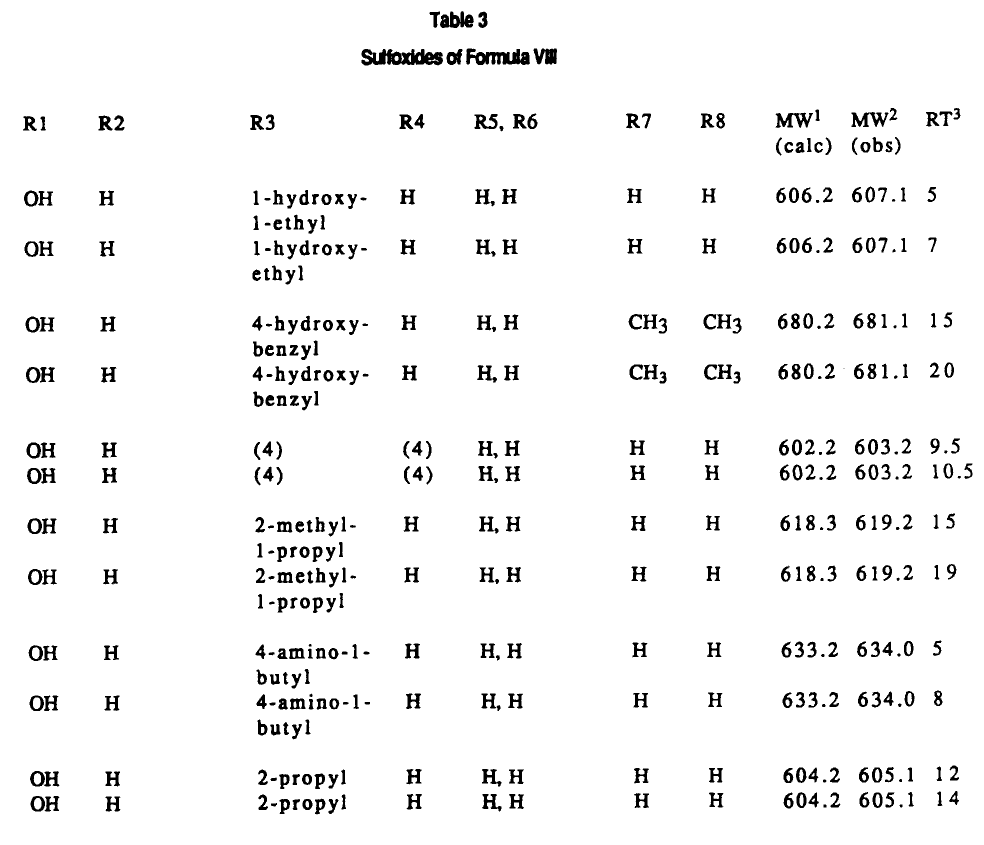

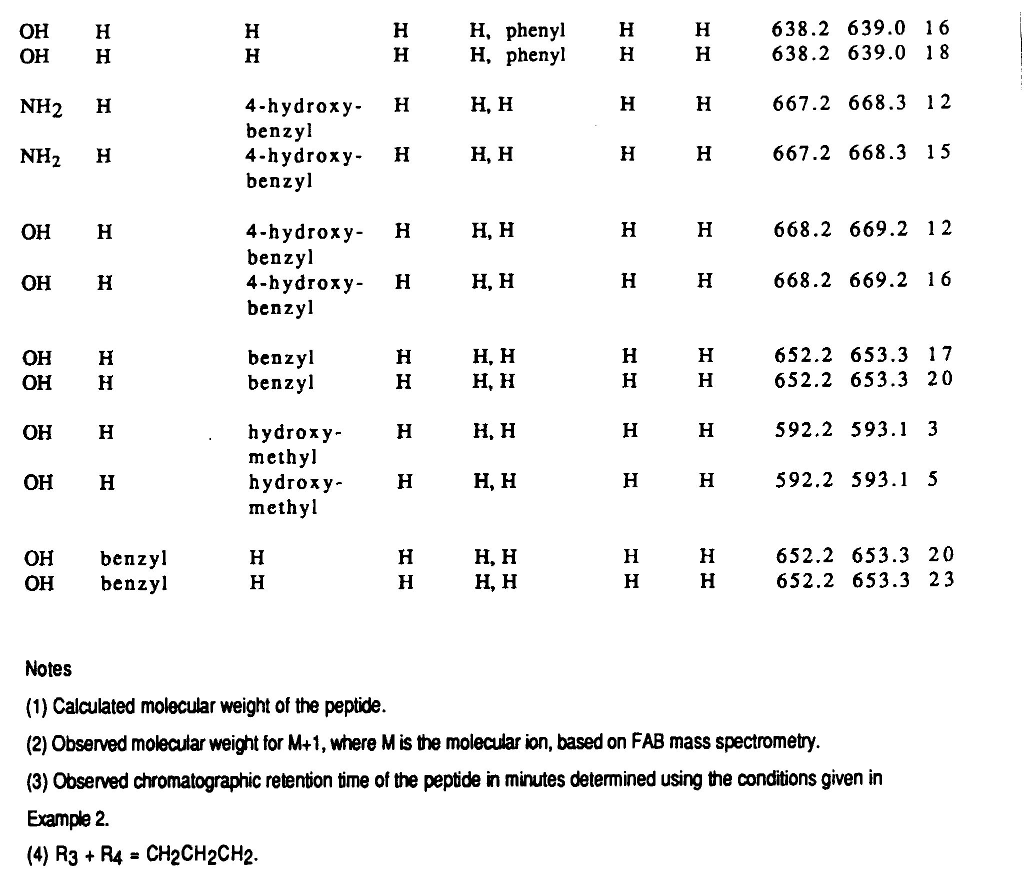

### Example 18

### Synthesis of the compound of Formula IX

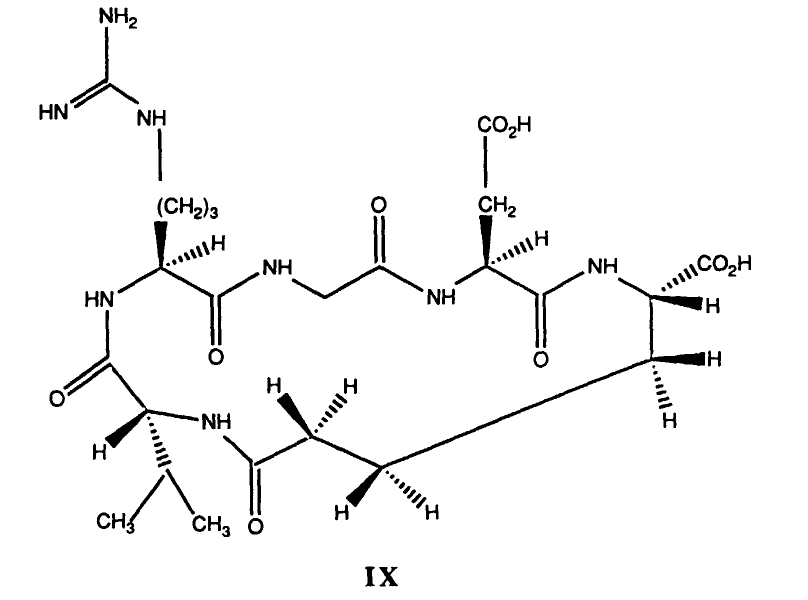

α-FMOC-aminoadipic acid δ-allylester was coupled to Wang resin with diisopropylcarbodiimide and a catalytic amount of 4-dimethylaminopyridine in dichloromethane solvent. The FMOC-group was then removed with 20% piperidine in N,N-dimethylacetamide. Standard FMOC synthesis chemistry was then used to add the Asp, Gly, Arg, and D-Val residues. After the coupling of the D-Val residue, treatment of the resin-bound peptide with 0.3 equivalents of tetrakis(triphenylphosphine)palladium(0) in N,N-dimethylacetamide containing 20% piperidine resulted in removal of the allyl and FMOC groups. The resin-bound peptide was then washed 5 times with 5% N-methylmorpholine in N,N-dimethylacetamide. The peptide was then cyclized with 2 equivalents of BOP reagent. Cleavage of the peptide from the resin was accomplished by treatment with trifluoroacetic acid : triethylsilane (98:2). The peptide was purified by HPLC as described in Example 2. HPLC retention time: 14 minutes. Molecular weight: calc.: 556.3. obs.(FAB mass spectrum): 557.3.

### Example 19

### Synthesis of compounds of Formula VIII derived from glutamic or 2-aminoadipic acid

The compounds of Formula VIII in which R₁ and R₉ are OH, R₅, R₆, R₇, R₈, and R₁₄ are hydrogen, the group X is (CH₂)ₖ where k is 0 or 1, m is 1, and n is 3 are prepared using the procedure of Example 18. In Table 4 k = 0 means glutamic acid and k = 1 means aminoadipic acid.

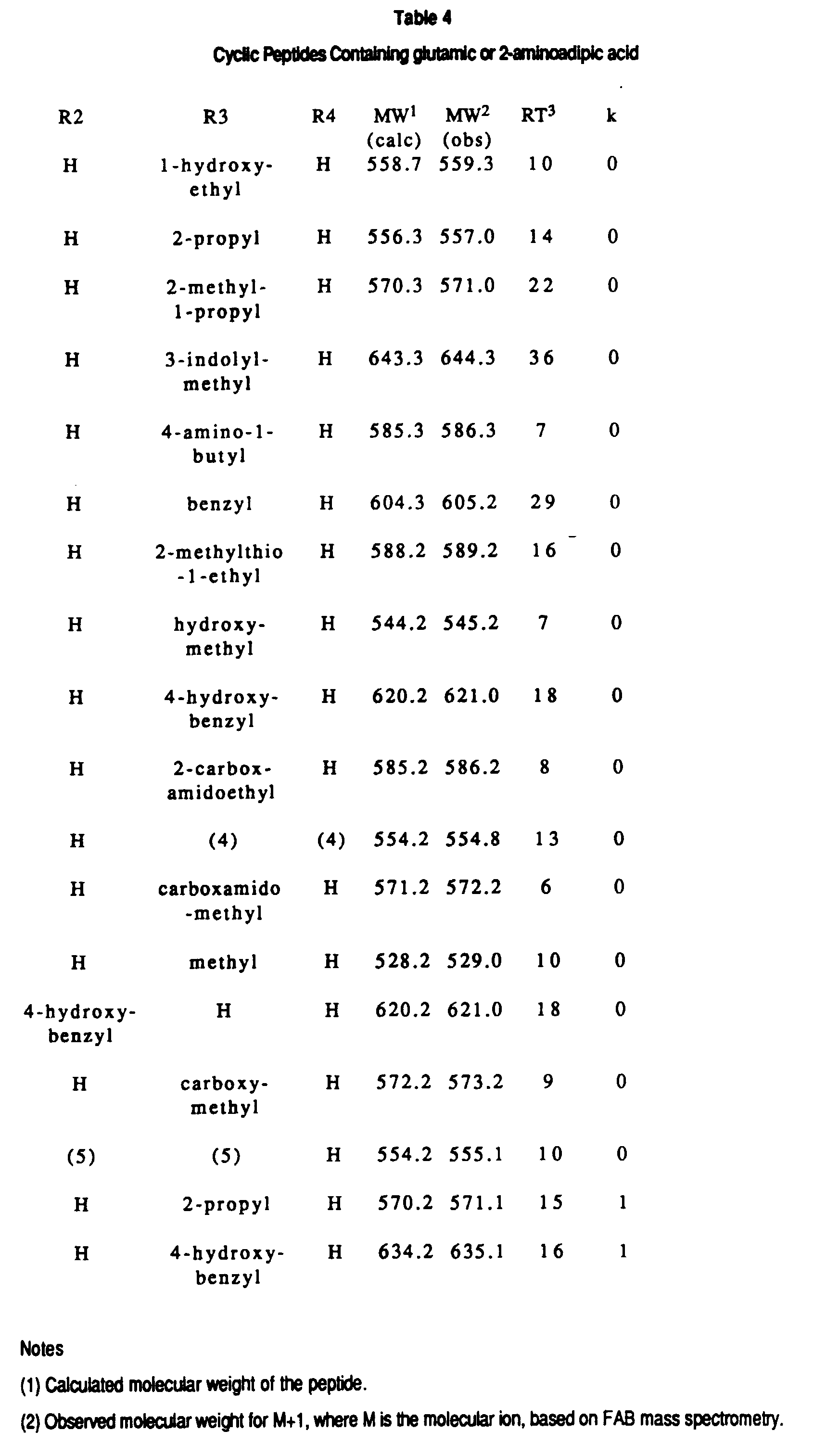

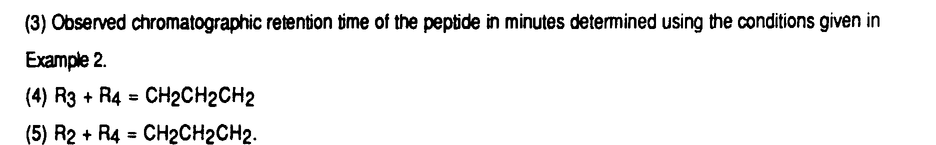

### Example 20

### L-2-FMOC-Amino-3-(N-t-butocycarbonyl-N-allyloxycarbonylmethyl)aminopropionic acid

Slow distillation of a solution of N-CBZ-D-serine methyl ester, dimethoxypropane (5 eq) and a catalytic amount of pyridinium p-toluenesulfonate (0.014 eq) in benzene resulted in the clean formation of the methyl 3-Cbz-2,2-dimethyloxazolidine-4-carboxylate. Reduction of the methyl ester with LiBH₄ (3 eq) in 2:1 ethanol-THF gave 3- Cbz-2,2-dimethyloxazolidine-4-methanol. Swern oxidation with (COCl)₂ (2 eq), DMSO (4 eq) and Et₃N (5 eq) in CH₂Cl₂ gave the 3-Cbz-2,2-dimethyloxazolidine-4-carboxyaldehyde which was reductively aminated with glycine benzyl ester hydrochloride (5 eq) and NaBH₃CN (1 eq) in methanol to give 4- (N-benzyloxycarbonylmethyl)-aminomethyl-3- Cbz-2,2-dimethyloxazolidine. The resulting amine was protected as the Boc derivative using Boc₂O (1.25 eq) and NaHCO₃ (1.4 eq) in 2:1 THF-H₂O. Hydrogenation of a methanolic solution of 4- (N-t-butyloxycarbonyl-N-benzyloxycarbonylmethyl)-aminomethyl-3- Cbz-2,2-dimethyloxazolidine, obtained in the previous step, at 50 psi in the presence of 10% Pd-C, the CBZ and benzyl ester groups were cleaved and the oxazolidine ring hydrolyzed, giving L-2-amino-3-(N-t-butoxycarbonyl-N-carboxymethyl)amino-1-propanol. The FMOC group was appended to the 2-amino function using N-(9-fluorenylmethoxycarbonyloxy)succinimide (1.15 eq) and NaHCO₃ (2.5 eq) in 1:1 dioxane-H₂O. Allyl esterification of the ω-carboxyl group using allyl bromide (7 eq) and NaHCO₃ (2.5 eq) in DMF followed by Jones oxidation (3 eq) of the primary alcohol gave the desired title compound.

### Example 21

### N-FMOC-O-allyloxycarbonylmethyl-L-serine

O-allylation of N-CBZ-L-serine in DMF solution was achieved by sequential treatment with NaH (2.2 eq) and allyl bromide (1.1 eq). The carboxylic acid was converted to the *tert-*butyl ester using isobutylene and H₂SO₄ in methylene chloride to afford O-allyl-N-Cbz-L-serine t-butyl ester. Cleavage of the terminal olefin of the allyl group by ozonolysis in methanol with a dimethylsulfide workup gave the aldehyde which, without isolation, was reduced to O-(2-hydroxy-1-ethyl)-N-Cbz-L-serine t-butyl ester with NaBH₄ (1 eq). The FMOC group was introduced by first reductively clearing the CBZ group at 50 psi H2 in methanol over 10% Pd-C catalyst, then reprotecting the resulting free amine with fluorenylmethylchloroformate (1.15 eq) and NaHCO₃ (1.8 eq) in 2:1 THF-H₂O. Jones oxidation (3 eq) of the alcohol on the hydroxyethyl group gave FMOC-O-carboxymethyl-L-serine-t-butyl ester, which was treated with allyl bromide (3 eq) and NaHCO₃ (1.5 eq) in DMF to give FMOC-O-allyloxycarbonylmethyl-L-serine-t-butyl ester. Finally, the t-butyl ester was removed with trifluoroacetic acid giving the desired N-FMOC-O-allyloxycarbonylmethyl-L.serine.

### Example 22

### Synthesis of cyclic peptides of Formula VIII derived from O-carboxymethyl-L-serine and 3-(N-carboxymethyl)-2,3-diaminopropionic acid

The protected amino acids from Examples 20 and 21 were coupled to Wang resin using diisopropylcarbodiimide and catalytic 4-dimethylaminopyridine. Standard FMOC solid phase synthesis protocol was used to couple the Asp, Gly, Arg and selected D-amino acid residues. Thus, coupling steps were carried out using BOP and N-methyl morpholine in N,N-dimethylacetamide. Deprotections were performed with 20% piperidine in N,N-dimethylacetamide. Cleavage of the allyl ester groups was effected with (Ph₃P)₄Pd in 20% piperidine/N,N-dimethylacetamide. The peptide was cyclized while still attached to the resin using diisopropylcarbodiimide and a catalytic amount of N-hydroxybenztriazole in dichloromethane to effect cyclization. The cydized peptides were cleaved from the resin with trifluoroacetic acid and purified on reverse phase HPLC. The peptides prepared by this route are derivatives of Formula VIII wherein R₁ and R₉ are OH, R₂, R₄, R₅, R₆, R₇, R₈, and R₁₄ are hydrogen, n is 3 and m is 1 and the remaining substituents are as shown.

| R₃ | X | MW(calc)¹ | MW(obs)² | RT³ |
|---|---|---|---|---|
| 2-propyl | NH | 571.2 | 572.3 | 8 |
| 4-hydroxybenzyl | NH | 635.2 | 636.3 | 25 |
| 4-hydroxybenzyl | O | 636.2 | 637.2 | 18 |

| | | | | |
|---|---|---|---|---|
| Notes (1) Calculated molecular weight of the peptide. | | | | |
| (2) Observed molecular weight for M+1, where M is the molecular ion, based on FAB mass spectrometry. | | | | |
| (3) Observed chromatographic retention time of the peptide in minutes determined using the conditions given in Example 2. | | | | |

### Example 23

### Inhibition of fibrinogen binding to GP IIbIIIa

Microtiter plates are coated with fibrinogen (10 µg/ml) and then blocked with TACTS buffer containing 0.5% BSA. (TACTS buffer contains 20mM Tris.HCl, pH 7.5, 0.02% sodium azide, 2 mM calcium chloride, 0.05% Tween 20, 150 mM sodium chloride.) The plate is washed with phosphate buffered saline containing 0.01% Tween 20 and a dilution of the sample to be determined added, followed by addition of solubilized IIbIIIa receptor (40 µg/ml) in TACTS, 05% BSA. After incubation, the plate is washed and murine monodonal anti-platelet antibody AP3 (1 µg/ml) added. After another wash goat and anti-mouse IgG conjugated to horseradish peroxidase is added. A final wash is performed and developing reagent buffer (10 mg o-phenylenediamine dihydrochloride, 0.0212% hydrogen peroxide, 0.22 mM citrate, 50 mM phosphate, pH 5.0) is added and then incubated until color developed. The reaction is stopped with 1N sulfuric acid and the absorbance at 492 nm is recorded and the IC₅₀ values determined. The results are recorded in Table 5.

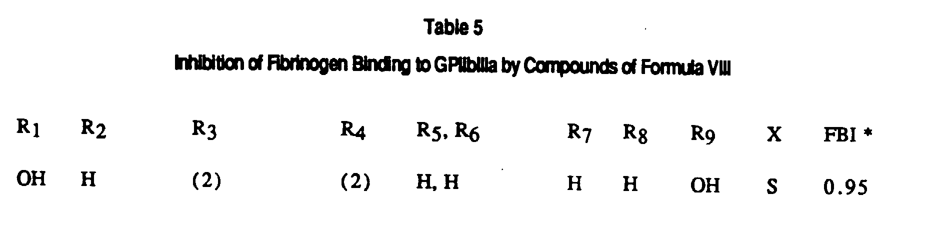

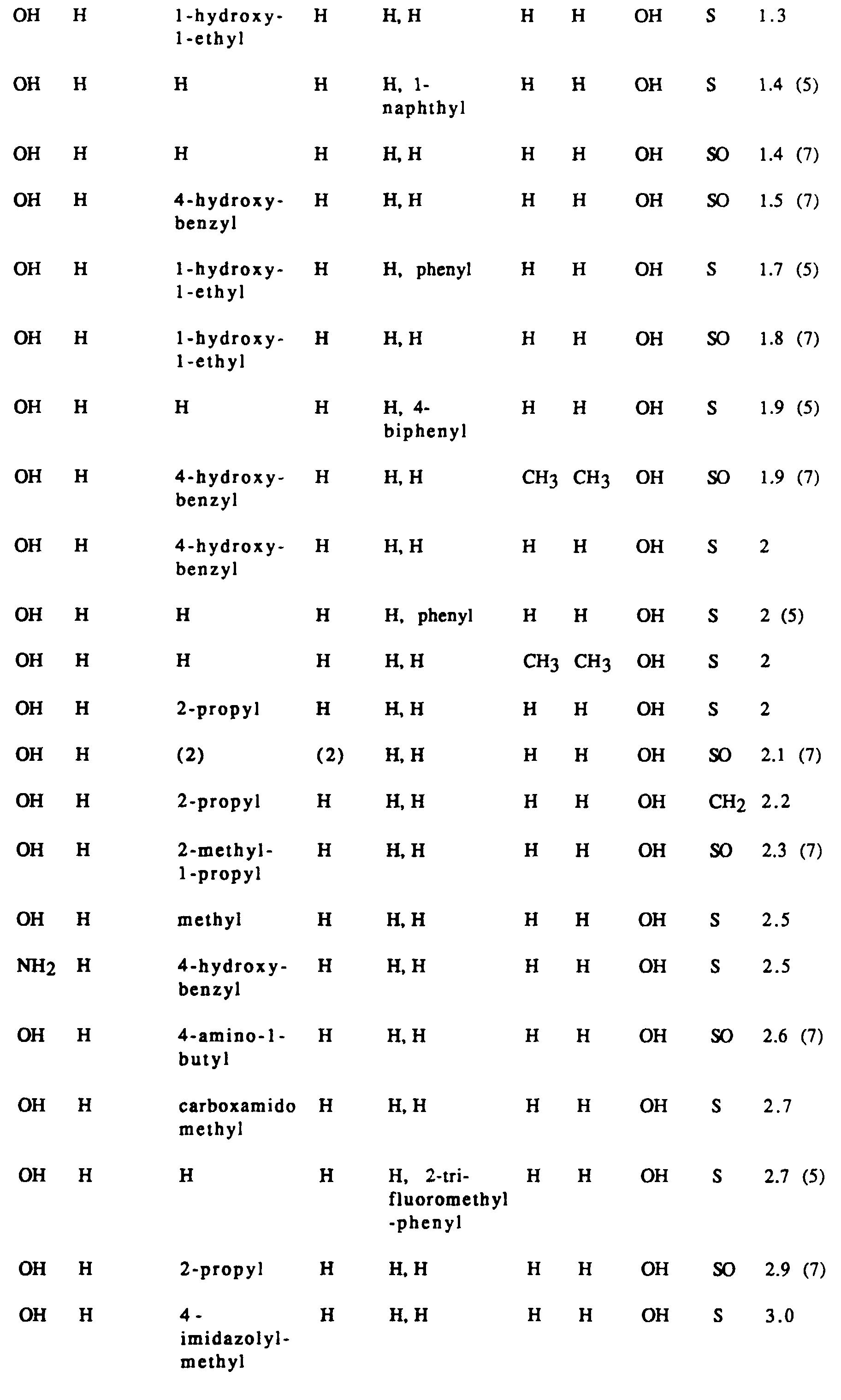

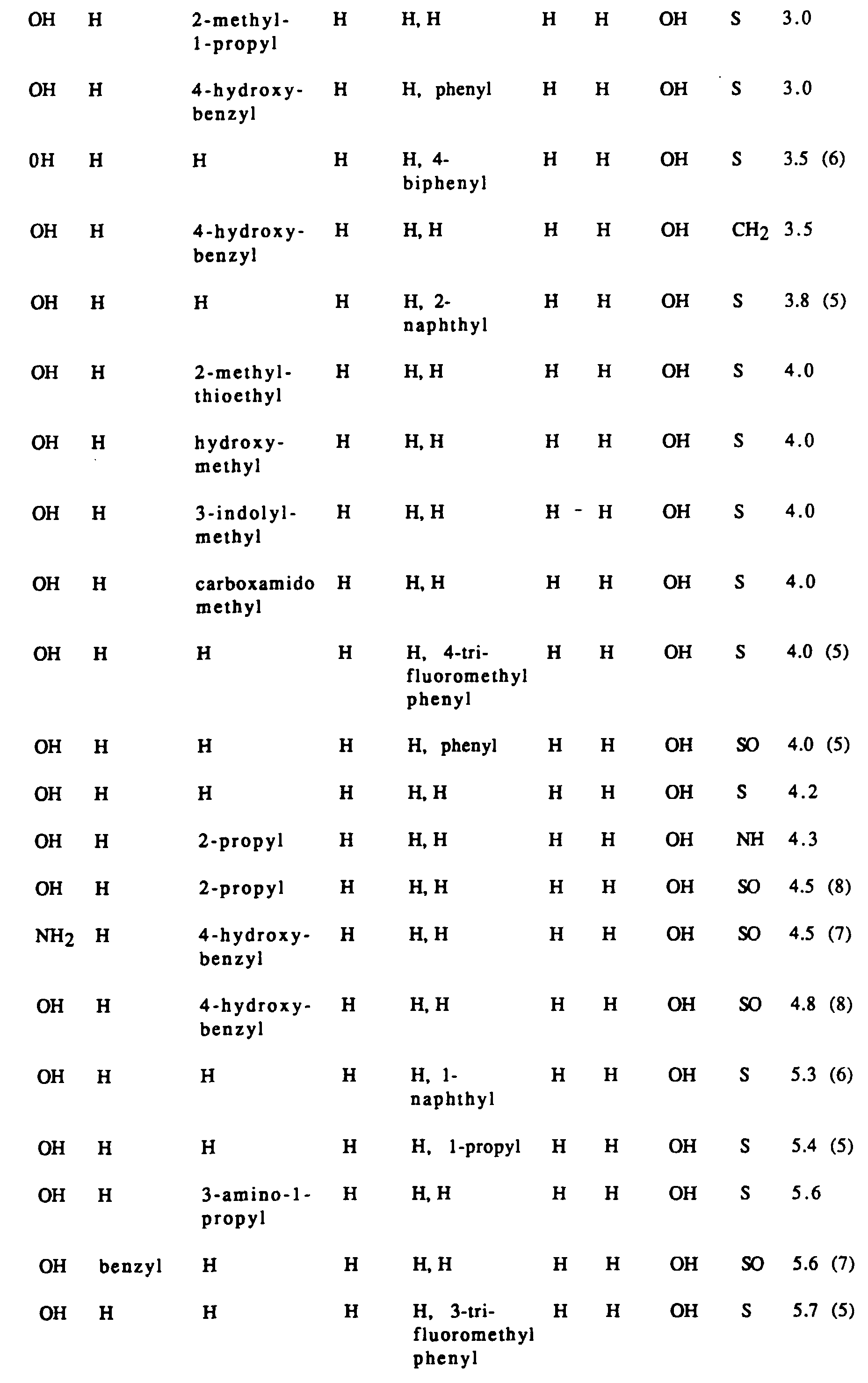

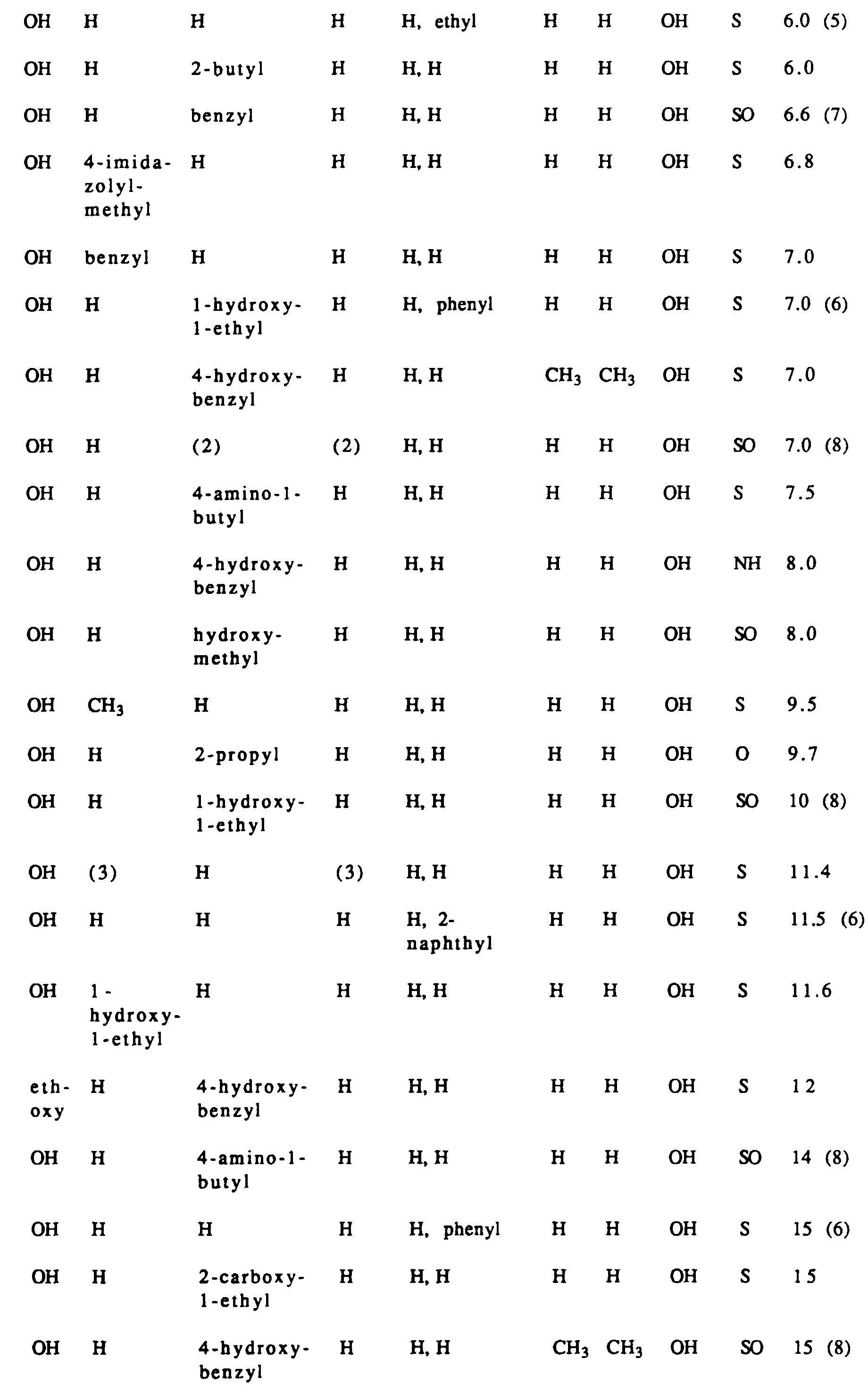

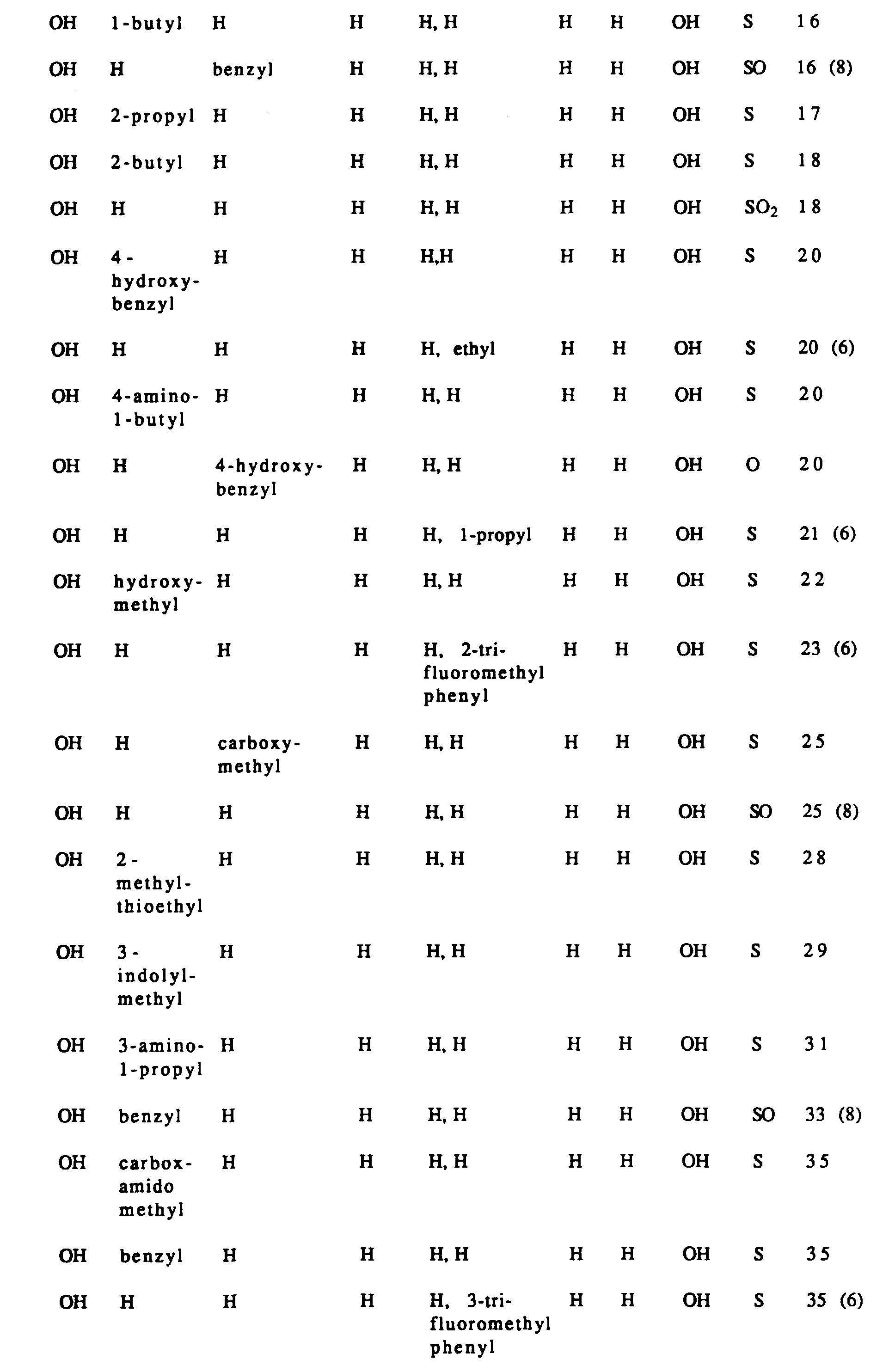

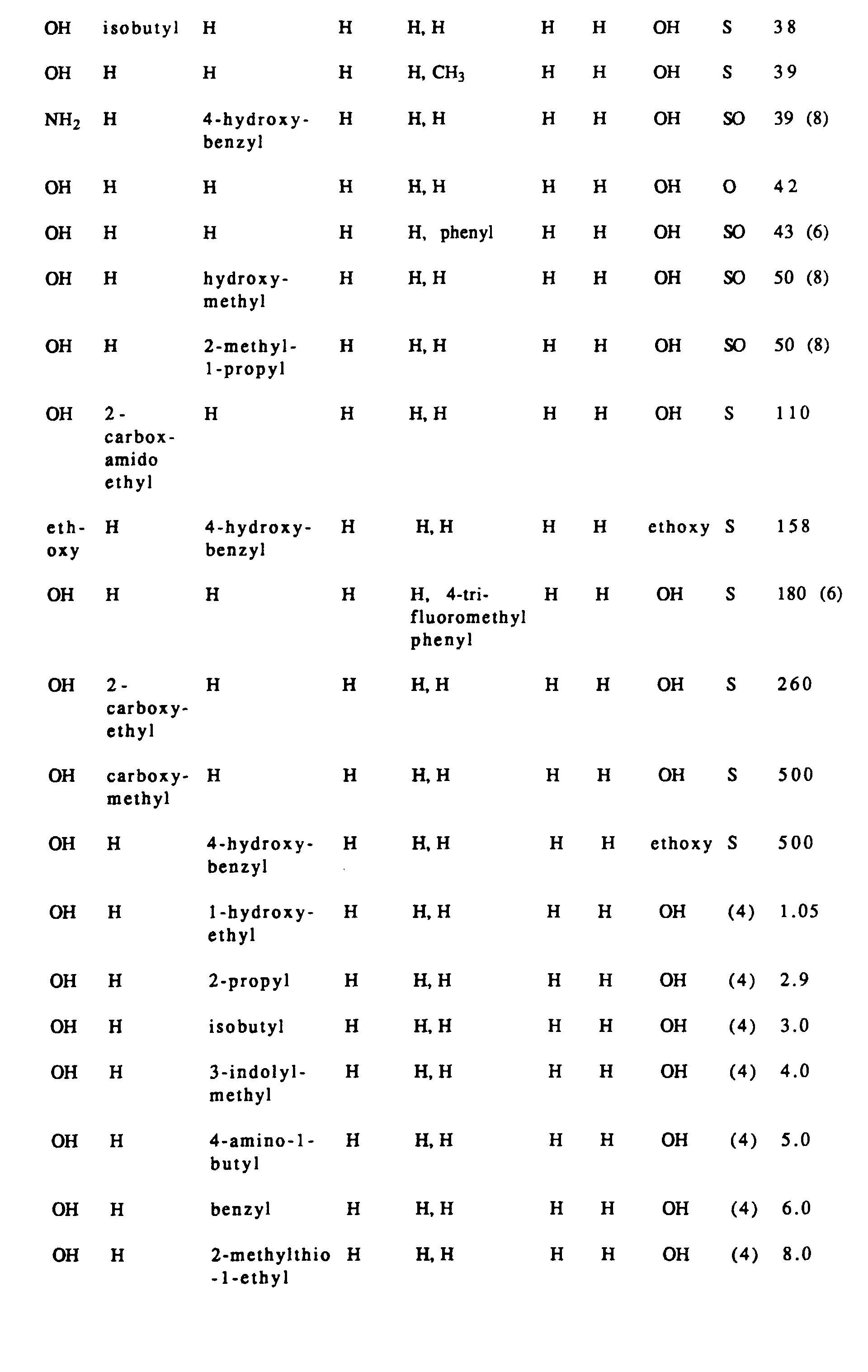

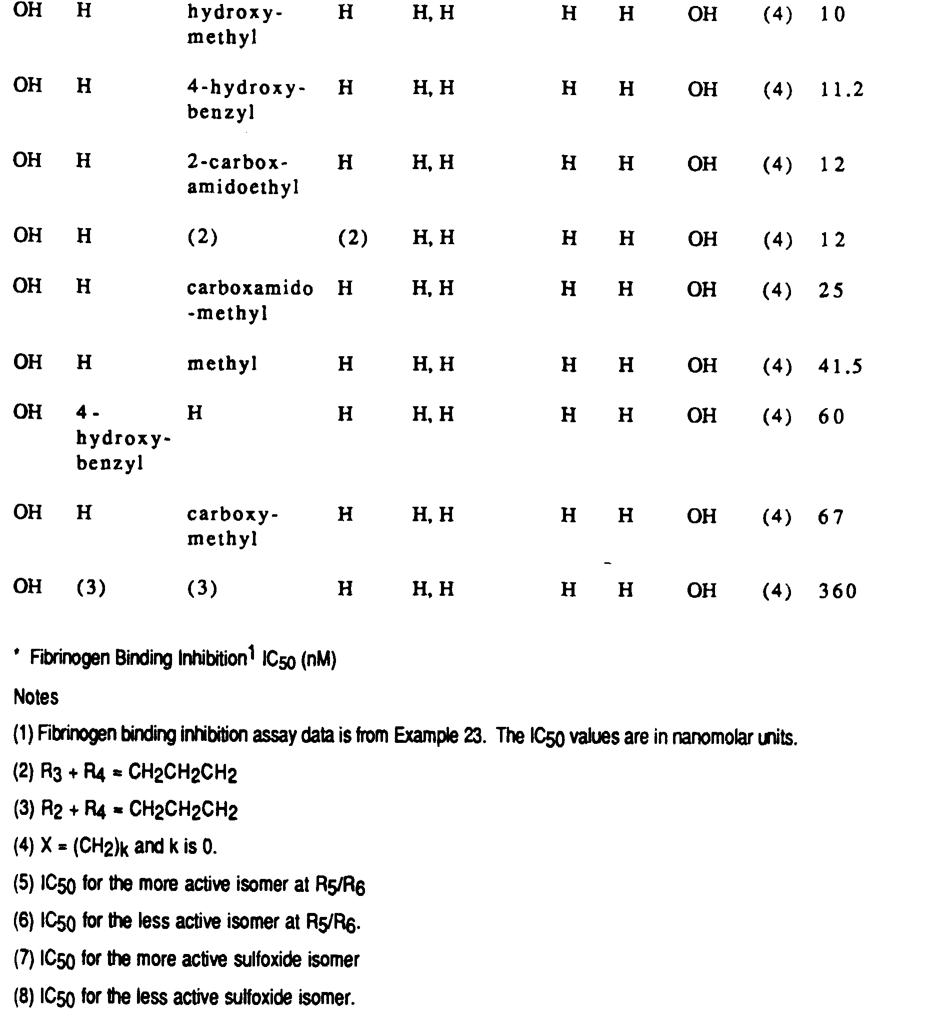

### Example 24

### Inhibition of platelet aggregation

Fifty milliliters of whole human blood (9 parts) is drawn on 3.6% sodium citrate (1 part) from a donor who has not taken aspirin or related medications for at least two weeks. The blood is centrifuged at 160 x g for 10 min at 22° C and then allowed to stand for 5 min after which the PRP is decanted. Platelet poor plasma (PPP) is isolated from the remaining blood after centrifugation at 2000 x g for 25 min. The platelet count of the PRP was diluted to ca. 300000 per microliter with PPP.

A 225 µL aliquot of PRP plus 25 µL of either a dilution of the test sample or a control (PBS) is incubated for 5 min in a Chrono-log Whole Blood Aggregometer at 25° C. Adenosine diphosphate (ADP, 8 µM) is added and the platelet aggregation recorded. The results of this test are recorded in Table 6. Where the possibility of stereoisomers exists, the values in Table 6 are for the most active stereoisomers.

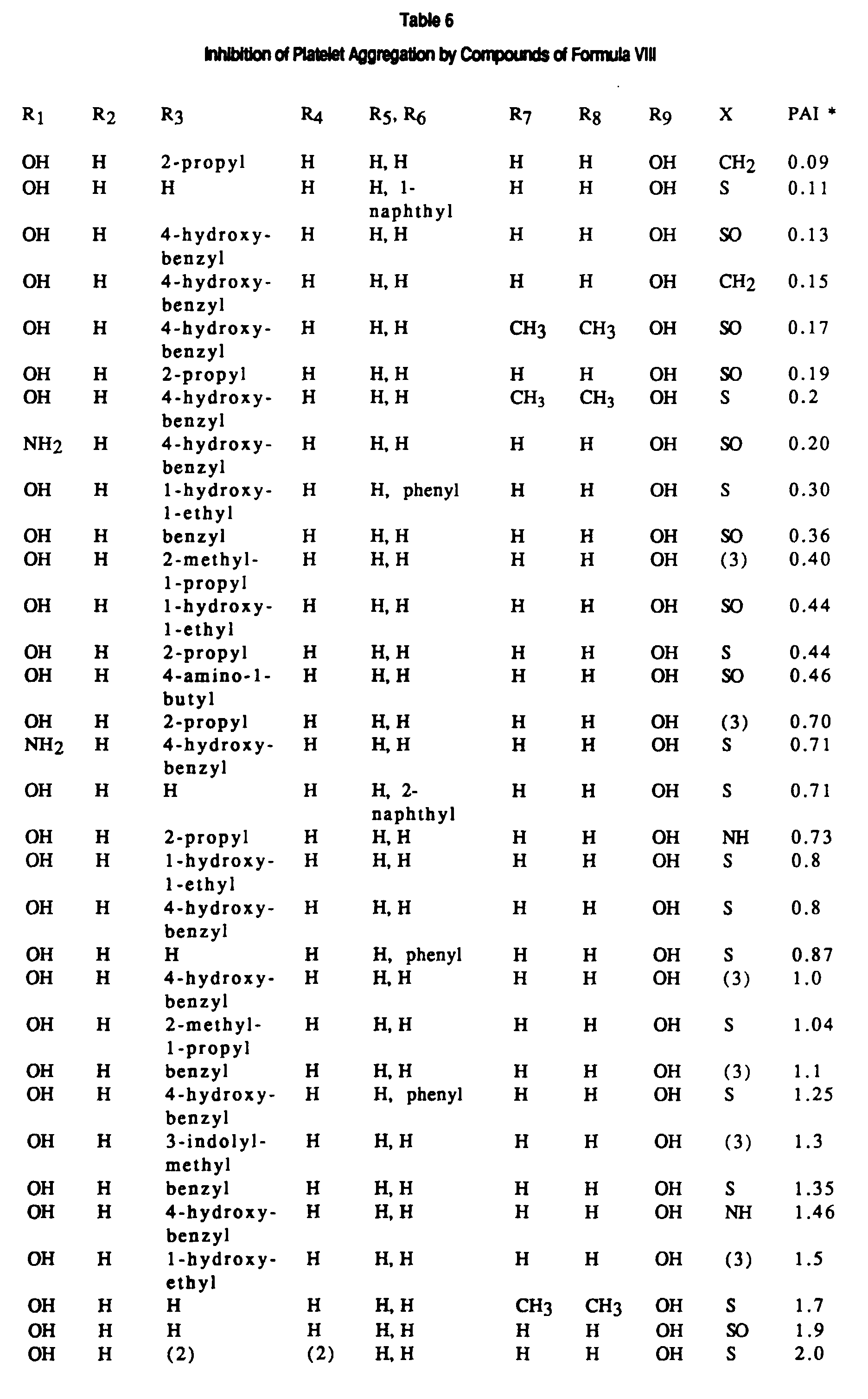

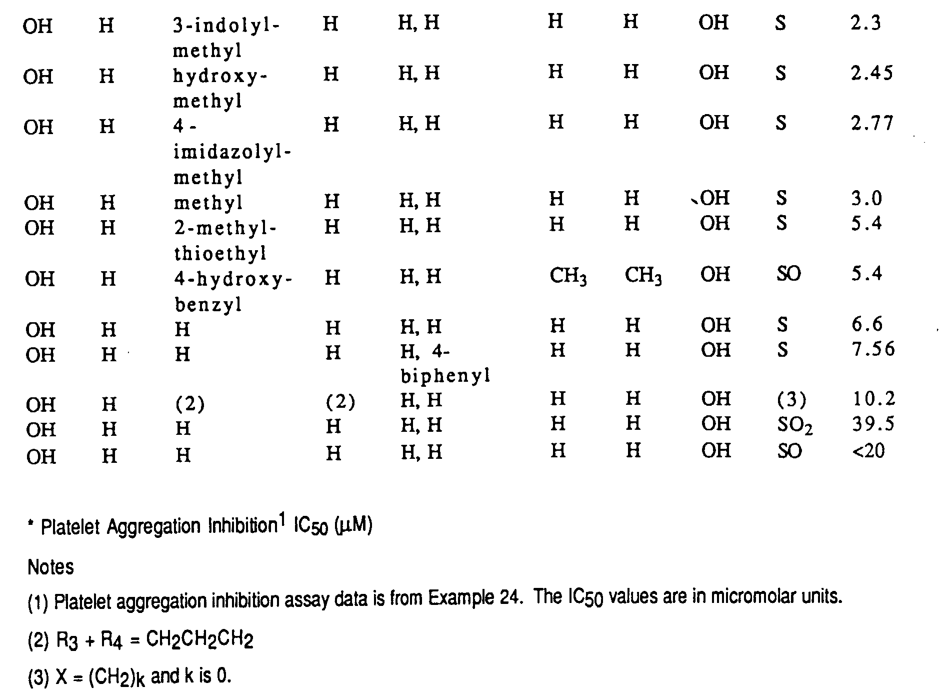

In view of the efficacy of these cydic polypeptides as inhibitors of fibrinogen binding to GP IIbIIIa, and the feasibility as demonstrated herein of producing these cyclic polypeptides, the present invention may have application in the treatment of a large group of disorders associated with, or characterized by, a hyperthrombotic state. Representative of such disorders are genetic or acquired deficiencies of factors which normally prevent a hyperthrombotic state; medical procedures such as angioplasty and thrombolytic therapy; mechanical obstructions to blood flow, such as tumor masses, prosthetic synthetic cardiac valves, and extracorporeal perfusion devices; atherosclerosis; and coronary artery disease.

## Claims

1. A compound of the formula: 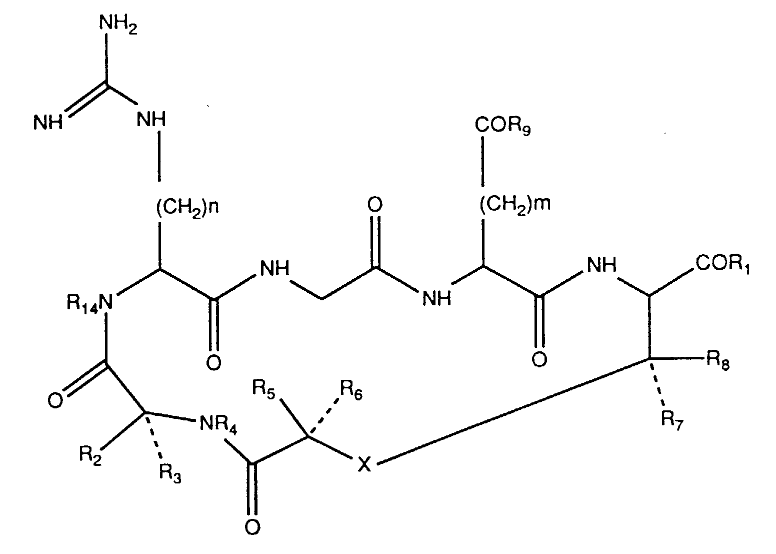 wherein
R₁ and R₉ are the same or different and are selected from
hydroxy,
C₁-C₈ alkoxy,
C₃-C₁₂ alkenoxy,
C₆-C₁₂ aryloxy,
di-C₁-C₈ alkylamino-C₁-C₈-alkoxy,
acylamino-C₁-C₈-alkoxy selected from the group acetylaminoethoxy, nicotinoylaminoethoxy, and succinamidoethoxy,
pivaloyloxyethoxy,
C₆-C₁₂ aryl-C₁-C₈-alkoxy where the aryl group is unsubstituted or substituted with one or more of the groups nitro, halo (F, Cl, Br, I), C₁-C₄-alkoxy, and amino,
hydroxy-C₂-C₈-alkoxy,
dihydroxy-C₃-C₈-alkoxy, and
NR₁₀R₁₁ wherein R₁₀ and R₁₁ are the same or different and are hydrogen, C₁-C₈-alkyl, C₃-C₈-alkenyl, C₆-C₁₂-aryl where the aryl group is unsubstituted or substituted with one or more of the groups
nitro, halo (F, Cl, Br, I), C₁-C₄-alkoxy, and amino, C₆-C₁₂-aryl-C₁-C₈-alkyl where the aryl group is unsubstituted or substituted by one or more of the groups nitro,
halo (F, Cl, Br, I), C₁-C₄-alkoxy, and amino;
R₂, R₃, R₅, R₆, R₇, R₈ are the same or different and are selected from
hydrogen,
C₆-C₁₂ aryl where the aryl group is unsubstituted or substituted by one or more of the groups nitro, hydroxy, halo (F, Cl, Br, I), C₁-C₈ alkyl, halo-C₁-C₈ alkyl, C₁-C₈-alkoxy, amino, phenyloxy, phenyl, acetamido, benzamido, di-C₁-C₈ alkylamino, C₁-C₈ alkylamino, C₆-C₁₂ aroyl, C₁-C₈ alkanoyl, and hydroxy-C₁-C₈ alkyl,
C₁-C₁₂ alkyl either substituted or unsubstituted, branched or straight chain where the substituents are selected from halo (F, Cl, Br, I),
C₁-C₈ alkoxy,
C₆-C₁₂ aryloxy where the aryl group is unsubstituted or substituted by one or more of the groups nitro, hydroxy, halo (F, Cl, Br, I), C₁-C₈ alkyl, C₁-C₈-alkoxy, amino, phenyloxy, acetamido, benzamido, di-C₁-C₈ alkylamino, C₁-C₈ alkylamino, C₆-C₁₂ aroyl, and C₁-C₈ alkanoyl,
isothioureido,
C₃-C₇ cycloalkyl,
ureido,
amino,
C₁-C₈ alkylamino,
di-C₁-C₈ alkylamino,
hydroxy,
amino-C₂-C₈ alkylthio,
amino-C₂-C₈ alkoxy,
acetamido,
benzamido wherein the phenyl ring is unsubstituted or substituted by one or more of the groups nitro, hydroxy, halo (F, Cl, Br, I), C₁-C₈ alkyl, C₁-C₈-alkoxy, amino, phenyloxy, acetamido, benzamido, di-C₁-C₈ alkylamino, C₁-C₈ alkylamino, C₆-C₁₂ aroyl, and C₁-C₈ alkanoyl,
C₆-C₁₂ arylamino wherein the aryl group is unsubstituted or substituted by one or more of the groups nitro, hydroxy, halo, C₁-C₈ alkyl, C₁-C₈-alkoxy, amino, phenyloxy, acetamido, benzamido, di-C₁-C₈ alkylamino, C₁-C₈ alkylamino, C₆-C₁₂ aroyl, and C₁-C₈ alkanoyl,
guanidino,
phthalimido,
mercapto,
C₁-C₈ alkylthio,
C₆-C₁₂ arylthio,
carboxy,
carboxamide,
carbo-C₁-C₈ alkoxy,
C₆-C₁₂ aryl wherein the aryl group is unsubstituted or substituted by one or more of the groups nitro, hydroxy, halo, C₁-C₈ alkyl, C₁-C₈-alkoxy, amino, phenyloxy, acetamido, benzamido, di-C₁-C₈ alkylamino, C₁-C₈ alkylamino, hydroxy-C₁-C₈ alkyl, C₆-C₁₂ aroyl, and C₁-C₈ alkanoyl, and
aromatic heterocyclic wherein the heterocyclic groups have 5-10 ring atoms and contain up to two O, N, or S heteroatoms;
R₂ and R₃, R₅ and R₆, or R₇ and R₈ may optionally and independently be joined together to form a carbocyclic or heterocyclic ring of from four to seven atoms where the heteroatoms are selected from O, S or NR₁₂ where R₁₂ is selected from
hydrogen, C₁-C₈-alkyl, C₃-C₈-alkenyl, C₆-C₁₂-aryl, C₆-C₁₂-aryl-C₁-C₈-alkyl, C₁-C₈ alkanoyl, and C₆-C₁₂ aroyl,
R₄ is selected from
hydrogen,
C₁-C₈ alkyl,
C₃-C₁₀ cycloalkyl,
C₆-C₁₂ aryl, and
C₆-C₁₂ aryl-C₁-C₈-alkyl;
R₂ or R₃ may be optionally joined with R₄ to form a piperidine, pyrrolidine or thiazolidine ring; R₁₄ is selected from
hydrogen, C₁-C₈-alkyl, C₃-C₈-alkenyl, C₆-C₁₂-aryl, and C₆-C₁₂ aryl-C₁-C₈-alkyl;
X is selected from
an O or S atom,
an S atom bearing one or two O atoms,
NR₁₃ wherein R₁₃ is hydrogen, C₁-C₈-alkyl, C₃-C₈-alkenyl, C₆-C₁₂-aryl, C₆-C₁₂-aryl-C₁-C₈-alkyl, C₁-C₈ alkanoyl, and C₆-C₁₂ aroyl, and
C₆-C₁₂ aryl,
C₁-C₈ alkanoyl,
(CH₂)ₖ where k is an integer from 0 to 5;
n is an integer from 1 to 6;
m is an integer from 0 to 4; and
pharmaceutically acceptable salts thereof.

2. A compound of the formula 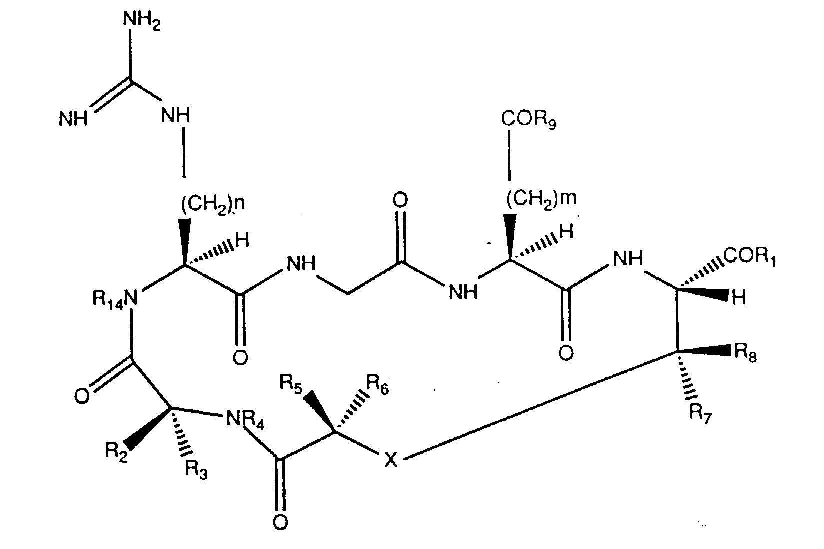 wherein
R₁ and R₉ are the same or different and are hydroxy, NH₂, C₁-C₄ alkoxy or benzyloxy;
R₂ is hydrogen
R₃ is selected from
hydrogen,
C₁-C₆ alkyl branched or unbranched, unsubstituted or substituted with substituents selected from amino, hydroxy, mercapto, methylthio, carboxy, carboxamide, guanidino, phenyl, 4-hydroxyphenyl, 4-methoxyphenyl, 3-indolyl, and 4-imidazolyl,
phenyl either unsubstituted or substituted with one to three substituents that may be independently nitro, hydroxy, halo (F, Cl, Br, I), C₁-C₄ alkyl, C₁-C₄-alkoxy, amino, phenyloxy, phenyl, acetamido, benzamido, di-C₁-C₄ alkylamino, C₁-C₄ alkylamino, halo-C₁-C₄ alkyl, C₆-C₁₂ aroyl, and C₁-C₄ alkanoyl,
1-naphthyl,
2-naphthyl,
2-thienyl,
2-pyridyl,
3-pyridyl, and
4-pyridyl;
R₅ and R₆ are independently selected from
hydrogen,
C₁-C₆ alkyl either branched or unbranched, unsubstituted or substituted with substituents selected from amino, hydroxy, mercapto, carboxy, carboxamide, guanidino, phenyl or 4-hydroxyphenyl, 4-methoxyphenyl, 3-indolyl, and 4-imidazolyl,
phenyl either unsubstituted or substituted with one to three substituents that may be independently selected from nitro, hydroxy, halo (F, Cl, Br, I), C₁-C₄ alkyl, C₁-C₄-alkoxy, amino, phenyloxy, phenyl, acetamido, benzamido, di-C₁-C₄-alkylamino, C₁-C₄ alkylamino, halo-C₁-C₄ alkyl, C₆-C₁₂ aroyl, and C₁-C₄ alkanoyl,
1-naphthyl,
2-naphthyl,
2-thienyl,
2-pyridyl,
3-pyridyl, and
4-pyridyl;
R₇ or R₈ are the same or different and are selected from
hydrogen,
C₁-C₄ alkyl,
phenyl either unsubstituted or substituted with from one to three substituents independently selected from hydroxy, halo (F, Cl, Br, I), C₁-C₄ alkyl, and C₁-C₄-alkoxy;
R₄ is hydrogen or may be joined with R₃ to form a heterocyclic ring selected from piperidine, pyrrolidine or thiazolidine;
R₁₄ is hydrogen or methyl ;
X is selected from
an O or S atom,
an S atom bearing one or two O atoms,
NR₁₃ where R₁₃ is selected from hydrogen, C₁-C₄ alkyl, benzyl, phenyl, C₁-C₄ alkanoyl, benzoyl and
(CH₂)ₖ, where k is 0 to 5;
n is 3 or 4;
m is 1; and
pharmaceutically acceptable salts thereof.

3. A compound of the formula 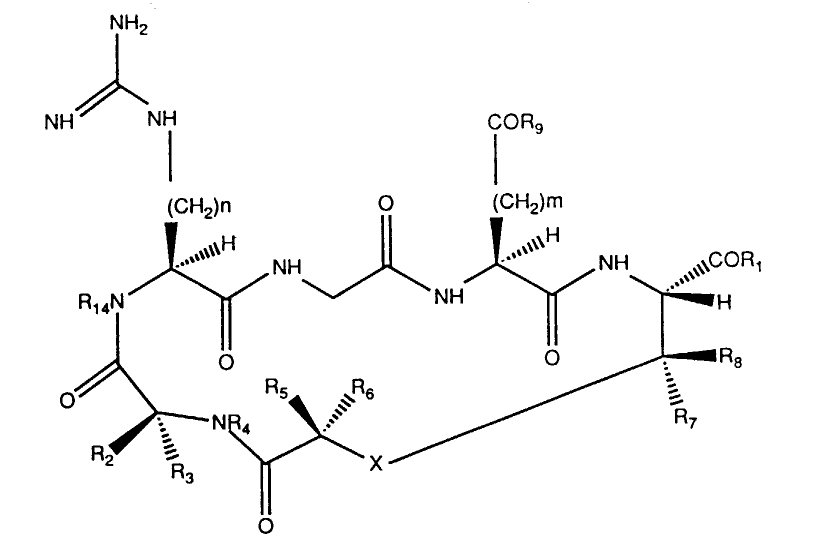 wherein
R₁ and R₉ are the same or different and are hydroxy, NH₂, C₁-C₄ alkoxy or benzyloxy;
R₃ is hydrogen
R₂ is selected from
hydrogen,
C₁-C₆ alkyl branched or unbranched, unsubstituted or substituted with substituents selected from amino, hydroxy, mercapto, methylthio, carboxy, carboxamide, guanidino, phenyl, 4-hydroxyphenyl, 4-methoxyphenyl, 3-indolyl, and 4-imidazolyl,
phenyl either unsubstituted or substituted with one to three substituents that may be independently nitro, hydroxy, halo (F, Cl, Br, I), C₁-C₄ alkyl, C₁-C₄-alkoxy, amino, phenyloxy, phenyl, acetamido, benzamido, di-C₁-C₄ alkylamino, C₁-C₄ alkylamino, halo-C₁-C₄ alkyl, C₆-C₁₂ aroyl, and C₁-C₄ alkanoyl,
1-naphthyl,
2-naphthyl,
2-thienyl,
2-pyridyl,
3-pyridyl, and
4-pyridyl ;
R₅ and R₆ are independently selected from
hydrogen,
C₁-C₆ alkyl either branched or unbranched, unsubstituted or substituted with substituents selected from amino, hydroxy, mercapto, carboxy, carboxamide, guanidino, phenyl or 4-hydroxyphenyl, 4-methoxyphenyl, 3-indolyl, and 4-imidazolyl,
phenyl either unsubstituted or substituted with one to three substituents that may be independently selected from nitro, hydroxy, halo (F, Cl, Br, I), C₁-C₄ alkyl, C₁-C₄-alkoxy, amino, phenyloxy, phenyl, acetamido, benzamido, di-C₁-C₄ alkylamino, C₁-C₄ alkylamino, halo-C₁-C₄ alkyl, C₆-C₁₂ aroyl, and C₁-C₄ alkanoyl,
1-naphthyl,
2-naphthyl,
2-thienyl,
2-pyridyl,
3-pyridyl, and
4-pyridyl;
R₇ or R₈ are the same or different and are selected from
hydrogen,
C₁-C₄ alkyl,
phenyl either unsubstituted or substituted with from one to three substituents independently selected from hydroxy, halo (F, Cl, Br, I), C₁-C₄ alkyl, and C₁-C₄-alkoxy;
R₄ is hydrogen or may be joined with R₃ to form a heterocyclic ring selected from piperidine, pyrrolidine or thiazolidine;
R₁₄ is hydrogen or methyl;
X is selected from
an O or S atom,
an S atom bearing one or two O atoms,
NR₁₃ where R₁₃ is selected from hydrogen, C₁-C₄ alkyl, benzyl, phenyl, C₁-C₄ alkanoyl, benzoyl and
(CH₂)ₖ, where k is 0 to 5;
n is 3 or 4;
m is 1; and
pharmaceutically acceptable salts thereof.

4. A compound of the formula: 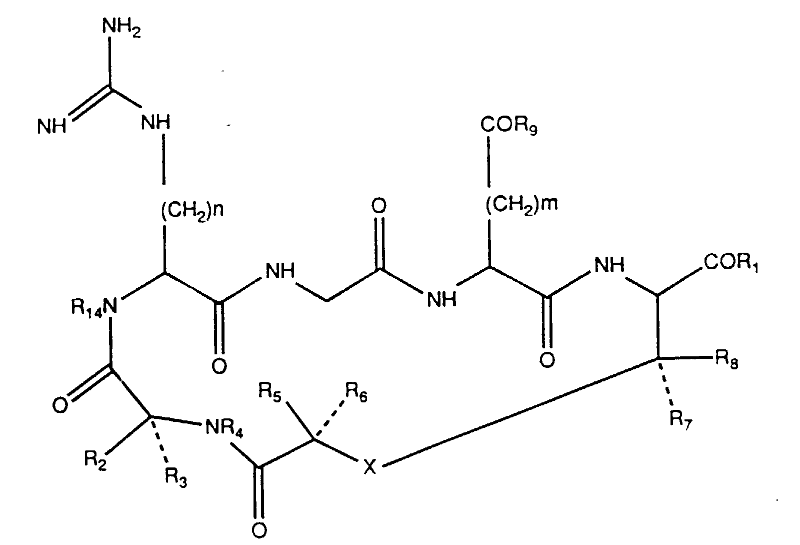 selected
(i) wherein R₁ and R₉ are both OH; R₂, R_{4,} R₅, R₆, R₇ and R₈ are all H; R₃ is 2-propyl; and X is CH₂
(ii) wherein R₁ and R₉ are both OH; R₂, R₃, R₄, R₅, R₇ and R₈ are all H; R₆ is 1-naphthyl; and X is S.
(iii) wherein R₁ and R₉ are both OH; R_{2,} R₄, R₅, R₆, R₇ and R₈ are all H; R₃ is a 4-hydroxybenzyl; and X is SO
(iv) wherein R₁ and R₉ are both OH; R₂, R₄, R₅, R₆, R₇ and R₈ are all H; R₃ is 4-hydroxybenzyl; and X is CH₂
(v) wherein R₁ and R₉ are both OH; R₂, R₄, R₅, and R₆ are all H; R₃ is 4-hydroxybenzyl; R₇ and R₈ are both CH₃; and X is SO
(vi) wherein R₁ and R₉ are both OH; R₂, R₄, R₅, R₆, R₇ and R₈ are all H; R₃ is 2-propyl and X is SO.
(vii) wherein R₁ and R₉ are both OH; R₂, R₄, R₅, and R₆ are all H; R₃ is 4-hydroxybenzyl; R₇ and R₈ are both CH₃; and X is S
(viii) wherein R₁ is NH₂; R₂, R₄, R_{5,} R₆, R₇ and R₈ are all H; R₃ is 4-hydroxybenzyl, R₉ is OH; and X is SO
(ix) wherein R₁ and R₉ are both OH; R₂, R₄, R₅, R₇ and R₈ are all H; R₃ is 1-hydroxy-1-ethyl, R₆ is phenyl; and X is S
(x) wherein R₁ and R₉ are both OH; R₂, R₄, R₅, R₆, R₇ and R₈ are all H; R₃ is benzyl; and X is SO
(xi) wherein R₁ and R₉ are both OH; R₂, R₄, R₅, R₆, R₇ and R₈ are all H; R₃ is 2-methyl-1-propyl; and X is (CH₂)_{K} and K is O
(xii) wherein R₁ and R₉ are both OH; R₂, R₄, R₅, R₆, R₇ and R₈ are all H; R₃ is 1-hydroxy-1-ethyl; and X is SO
(xiii) wherein R₁ and R₉ are both OH; R_{2,} R_{4,} R_{5,} R₆, R₇ and R₈ are all H; R₃ is 2-propyl and X is S
(xiv) wherein R₁ and R₉ are both OH; R₂, R₄, R₅, R₆, R₇ and R₈ are all H; R₃ is 4-amino-1-butyl; and X is SO
(xv) wherein R₁ and R₉ are both OH; R₂, R₄, R₅, R₆, R₇ and R₈ are all H; R₃ is 2-propyl and X is (CH₂)_{K} and K is O
(xvi) wherein R₁ is NH₂; R₂, R₄, R₅, R₆, R₇ and R₈ are all H; R₃ is 4-hydroxy-benzyl; R₉ is OH; and X is S
(xvii) wherein R₁ and R₉ are both OH; R₂, R₃, R₄, R₅, R₇ and R₈ are all H; R₆ is 2-naphthyl and X is S
(xviii) wherein R₁ and R₉ are both OH; R₂, R₄, R₅, R₆, R₇ and R₈ are all H; R₃ is 2-propyl; and X is NH
(xix) wherein R₁ and R₉ are both OH; R₂, R_{4,} R₅, R₆, R₇ and R₈ are all H; R₃ is 1-hydroxy-1-ethyl; and X is S
(xx) wherein R₁ and R₉ are both OH; R₂, R_{4,} R₅, R₆, R₇ and R₈ are all H; R₃ is 4-hydroxy-benzyl; and X is S
(xxi ) wherein R₁ and R₉ are both OH; R₂, R₃, R₄, R₅, R₇ and R₈ are all H; R₆ is phenyl; and X is S
(xxii) wherein R₁ and R₉ are both OH; R₂, R₄, R₅, R₆, R₇ and R₈ are all H; R₃ is 4-hydroxy-benzyl; and X is (CH₂)_{K} and K is O
(xxiii) wherein R₁ and R₉ are both OH; R₂, R₄, R₅, R₆, R₇ and R₈ are all H; R₃ is 2-methyl-1-propyl; and X is S
(xxiv) wherein R₁ and R₉ are both OH; R₂, R₄, R₅, R₆, R₇ and R₈ are all H; R₃ is benzyl and X is (CH₂)_{K} and K is O
(xxv) wherein R₁ and R₉ are both OH; R₂, R₄, R₅, R₇ and R₈ are all H; R₃ is 4-hydroxy-benzyl, R₆ is phenyl and X is S
(xxvi) wherein R₁ and R₉ are both OH; R₂, R₄, R₅, R₆, R₇ and R₈ are all H; R₃ is 3-indolyl-methyl; and X is (CH₂)_{K} and K is O
(xxvii) wherein R₁ and R₉ are both OH; R₂, R₄, R₅, R₆, R₇ and R₈ are all H; R₃ is benzyl and X is S
(xxviii) wherein R₁ and R₉ are both OH; R₂, R₄, R₅, R₆, R₇ and R₈ are all H; R₃ is 4-hydroxy-benzyl and X is NH
(xxix) wherein R₁ and R₉ are both OH; R₂, R₄, R₅, R₆, R₇ and R₈ are all H; R₃ is 1-hydroxy-ethyl and X is (CH₂)_{K} and K is O
(xxx) wherein R₁ and R₉ are both OH; R₂, R₃, R₄, R₅ and R₆ are all H; R₇ and R₈8 are CH₃ and X is S
(xxxi) wherein R₁ and R₉ are both OH; R₂, R₃, R₄, R₅, R₆, R₇ and R₈ are all H; and X is SO
(xxxii) wherein R₁ and R₉ are both OH; R₂, R₅, R₆, R₇ and R₈ are all H; R₃ and R₄ is CH₂CH₂CH₂; and X is S
(xxxiii) wherein R₁ and R₉ are both OH; R₂, R₄, R₅, R₆, R₇ and R₈ are all H; R₃ is 3-indolylpmethyl and X is S
(xxxiv) wherein R₁ and R₉ are both OH; R₂, R₄, R₅, R₆, R₇ and R₈ are all H; R₃ is hydroxymethyl and X is S
(xxxv) wherein R₁ and R₉ are both OH; R₂, R₄, R₅, R₆, R₇ and R₈ are all H; R₃ is 4-imidazolyl-methyl and X is S
(xxxvi) wherein R₁ and R₉ are both OH; R₂, R₄, R₅, R₆, R₇ and R₈ are all H; R₃ is methyl and X is S,

5. A compound of claim 2 selected from compounds wherein
(i) R₁ and R₉ are OH;
R₂, R₄, R₅, R₆, R₇, R₈, and R₁₄ are hydrogen;
R₃ is selected from
hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is S; and
n is 3 or 4;
(ii) R₁ and R₉ are OH;
R₂, R₄, R₅, R₆, R₇, R₈, and R₁₄ are hydrogen;
R₃ is selected from
hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is SO; and
n is 3 or 4
(iii) R₁ and R₉ are OH;
R₂, R₃, R₄, R₇, R₈, and R₁₄ are hydrogen;
R₅ and R₆ are independently selected from hydrogen, methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphtyl, 4-biphenyl, and pentafluorophenyl;
X is S; and
n is 3 or 4;
(iv) R₁ and R₉ are OH;
R₂, R₃, R₄, R₇, R₈, and R₁₄ are hydrogen;
R₅ and R₆ are independently selected from hydrogen methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphthyl, 4-biphenyl, and pentafluorophenyl;
X is SO; and
n is 3 or 4;
(v) R₁ and R₉ are OH;
R₂, R₃, R₄, R₅, R₆, and R₁₄ are hydrogen;
R₇ and R₈ are independently selected from hydrogen, methyl and phenyl;
X is S; and
n is 3 or 4;
(vi) R₁ and R₉ are OH;
R₂, R₃, R₄, R₅, R₆, and R₁₄ are hydrogen;
R₇ and R₈ are independently selected from hydrogen, methyl and phenyl;
X is SO; and
n is 3 or 4;
(vii) R₁ and R₉ are OH;
R₂, R₄, R₇, R₈, and R₁₄ are hydrogen;
R₃ is selected from 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
R₅ and R₆ are independently selected from hydrogen, methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphthyl, 4-biphenyl, and pentafluorophenyl;
X is S; and
n is 3 or 4;
(viii) R₁ and R₉ are OH;
R₂, R₄, R₇, R₈, and R₁₄ are hydrogen;
R₃ is selected from 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
R₅ and R₆ are independently selected from hydrogen, methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphthyl, 4-biphenyl, and pentafluorophenyl;
X is SO; and
n is 3 or 4;
(ix) R₁ and R₉ are OH;
R₂, R₄, R₅, R₆, and R₁₄ are hydrogen;
R₃ is selected from 4-amino-1-butyl, 3-amino-1-propyl, hydroxymethyl, 2-hydroxy-1-propyl, methyl ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamiadoethyl;
R₇ and R₈ are independently selected from hydrogen, methyl and phenyl;
X is S; and
n is 3 or 4;
(x) R₁ and R₉ are OH;
R₂, R₄, R₅, R₆, and R₁₄ are hydrogen;
R₃ is selected from 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
R₇ and R₈ are independently selected from hydrogen, methyl and phenyl;
X is SO; and
n is 3 or 4;
(xi) R₁ and R₉ are OH;
R₂, R₃, R₄, and R₁₄ are hydrogen;
R₅ and R₆ are independently selected from hydrogen, methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphthyl, 4-biphenyl, and pentafluorophenyl;
R₇ and R₈ are independently selected from hydrogen, methyl and phenyl;
X is S; and
n is 3 or 4;
(xii) R₁ and R₉ are OH;
R₂, R₃, R₄, and R₁₄ are hydrogen;
R₅ and R₆ are independently selected from hydrogen, methyl, ethyl, propyl, isopropyl, butyl, isobutyl, benzyl, phenyl, 2-trifluoromethylphenyl, 3-trifluoromethylphenyl, 4-trifluoromethylphenyl, 1-naphthyl, 2-naphthyl, 4-biphenyl, and pentafluorophenyl;
R₇ and R₈ are independently selected from hydrogen, methyl and phenyl;
X is SO; and
n is 3 or 4;
(xiii) R₁ and R₉ are OH:
R₂, R₄, R_{5,} R₆, R₇, R_{8,} and R₁₄ are hydrogen:
R₃ is selected from hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is O; and
n is 3 or 4;
(xiv) R₁ and R₉ are OH:
R₂, R₄, R₅, R₆, R₇, R₈, and R₁₄ are hydrogen;
R₃ is selected from hydrogen, 4-amino-1-butyl, 3-ammo-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is NR₁₃ where R₁₃ is selected from hydrogen, C₁-C₄ alkyl, C₁-C₄ alkanoyl, benzoyl and benzyl; and
n is 3 or 4;
(xv) R₁ and R₉ are OH;
R₂, R₄, R₅, R₆, R₇, R₈, and R₁₄ are hydrogen;
R₃ is 4-hydroxybenzyl;
X is S; and
n is 3;
(xvi) R₁ and R₉ are OH;
R₂, R₄, R₅, R₆, R₇, R₈, and R₁₄ are hydrogen;
R₃ is 4-hydroxybenzyl;
X is SO; and
n is 3;
(xvii) R₁ and R₉ are OH;
R₂, R₄, R₅, R₆, R₇, R₈, and R₁₄ are hydrogen;
R₃ is isopropyl;
X is S; and
n is 3;
(xviii) R₁ and R₉ are OH;
R₂, R₄, R₅, R₆, R₇, R₈, and R₁₄ are hydrogen;
R₃ is isopropyl;
X is SO; and
n is 3;
(xix) R₁ and R₉ are OH;
R₂, R₄, R₅, R₆, R₇, R₈, and R₁₄ are hydrogen;
R₃ is 2-butyl;
X is S; and
n is 3;
(xx) R₁ and R₉ are OH;
R₂, R₄, R₅, R₆, R₇, R₈, and R₁₄ are hydrogen;
R₃ is 2-butyl;
X is SO; and
n is 3;
(xxi) R₁ and R₉ are OH;
R₂, R₅, R₆, R₇, R₈, and R₁₄ are hydrogen;
R₃ and R₄ are joined together to form a pyrrolidine ring;
X is S; and
n is 3;
(xxii) R₁ and R₉ are OH;
R₂, R₅, R₆, R₇, R₈, and R₁₄ are hydrogen;
R₃ and R₄ are joined together to form a pyrrolidine ring;
X is SO; and
n is 3;
(xxiii) R₁ and R₉ are OH;
R₂, R₄, R₅, R₆, and R₁₄ are hydrogen;
R₃ is 4-hydroxybenzyl;
R₇ and R₈ are methyl;
X is S; and
n is 3;
(xxiv) R₁ and R₉ are OH;
R₂, R₄, R₅, R₆, and R₁₄ are hydrogen;
R₃ is 4-hydroxybenzyl;
R₇ and R₈ are methyl;
X is SO; and
n is 3;
(xxv) R₁ and R₉ are OH;
R₂, R₄, R₅, R₆, and R₁₄ are hydrogen;
R₃ is isopropyl;
R₇ and R₈ are methyl;
X is S; and
n is 3;
(xxvi) R₁ and R₉ are OH;
R₂, R₄, R₅, R₆, and R₁₄ are hydrogen;
R₃ is isopropyl;
R₇ and R₈ are methyl;
X is SO; and
n is 3;
(xxvii) R₁ and R₉ are OH;
R₂, R₃, R₄, R₅, R₆, and R₁₄ are hydrogen;
R₇ and R₈ are methyl;
X is S; and
n is 3;
(xxviii) R₁ and R₉ are OH;
R₂, R₃, R₄, R₅, R₆, and R₁₄ are hydrogen;
R₇ and R₈ are methyl;
X is SO; and
n is 3;
(xxix) R₁ is NH₂;
R₉ is OH;
R₂, R₄, R₅, R₆, R₇, R₈, and R₁₄ are hydrogen;
R₃ is selected from hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is S; and
n is 3 or 4;
(xxx) R₁ is NH₂;
R₉ is OH;
R₂, R₄, R₅, R₆, R₇, R₈, and R₁₄ are hydrogen;
R₃ is selected from hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is SO; and
n is 3 or 4;
(xxxi) R₁ and R₉ are OH;
R₂, R₄, R₅, R₆, R₇, R₈, and R₁₄ are hydrogen;
R₃ is selected from hydrogen, 4-amino-1-butyl, 3-amino-1-propyl, hydroxymethyl, 2-hydroxy-1-propyl, methyl, ethyl, 2-propyl, isobutyl, n-propyl, n-butyl, 2-butyl, methylthioethyl, benzyl, 4-hydroxybenzyl, 4-methoxybenzyl, 3-indolylmethyl, 4-imidazolylmethyl, phenyl, carboxymethyl, carboxyethyl, carboxamidomethyl, and carboxamidoethyl;
X is (CH₂)ₖ where k is 0,1 or 2; and
n is 3 or 4;
(xxxii) R₁ and R₉ are OH;
R₂, R₄, R₅, R₆, R₇, R₈, and R₁₄ are hydrogen;
R₃ is 4-hydroxybenzyl;
X is CH₂; and
n is 3;
(xxxiii) R₁ and R₉ are OH;
R₂, R₄, R₅, R₆, R₇, R₈, and R₁₄ are hydrogen;
R₃ is isopropyl;
X is CH₂; and
n is 3;
(xxxiv) R₁ and R₉ are OH;
R₂, R₄, R₅, R₆, R₇, R₈, and R₁₄ are hydrogen;
R₃ is 2-butyl;
X is CH₂; and
n is 3;
(xxxv) R₁ and R₉ are OH:
R₂, R₅, R₆, R₇, R₈, and R₁₄ are hydrogen;
R₃ and R₄ are joined together to form a pyrrolidine ring;
X is CH₂; and
n is 3.

6. A process for preparing the compound of claim 2 which comprises:
(a) cyclizing an intermediate of Fomula IV 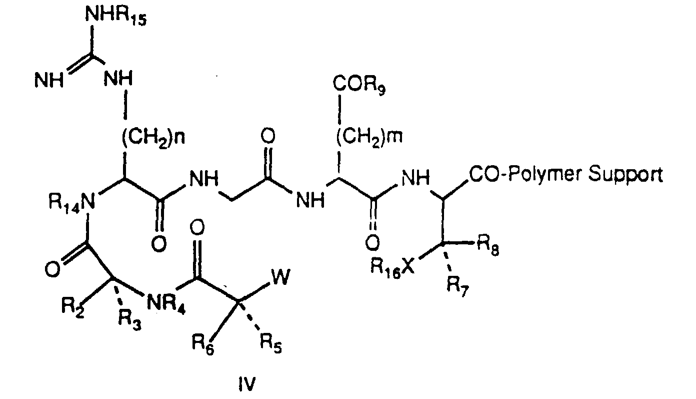 wherein
R₁-R₉, n and m are as defined in claim 2,
W is Br, Cl, or I,
R₁₄ is selected from hydrogen, C₁-C₆ alkyl, and C₆-C₁₂ aryl
R₁₅ is 2,2,5,7,8-pentamethylchroman-6-sulfonyl,
R₁₆ is hydrogen, and
X is S;
(b) cleaving the desired product from the resin;
(c) cleaving any protecting groups; and
(d) purifying and isolating the more biologically active isomer.

7. A process for preparing the compound of claim 2 which comprises:
(a) cyclizing an intermediate of Formula VI 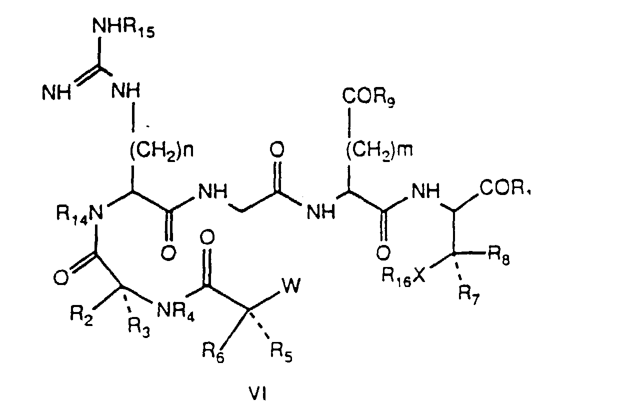 wherein
R₁-R₉, n and m are as defined in claim 2,
W is Br, Cl, or I,
R₁₄ is selected from hydrogen, C₁-C₆ alkyl, and C₆-C₁₂ aryl
R₁₅ is 2,2,5,7,8-pentamethylchroman-6-sulfonyl,
R₁₆ is H, and
X is S;
(b) cleaving any protecting groups; and
(c) purifying and isolating the more biologically active isomer.

8. A pharmaceutical composition comprising a pharmaceutically acceptable excipient and the compound of claim 1.

9. Use of a compound according to claim 1 in the manufacture of a medicament for inhibiting platelet aggregation.

10. Use of a compound according to claim 1 in the manufacture of a medicament for administration to a mammal having an increased propensity for thrombus formation.

11. A composition comprising a compound of claim 1 for any one of:
(a) reducing platelet aggregation;
(b) treating a mammal who has an increased propensity for thrombus formation;
(c) inhibiting fibrinogen binding to platelets in a mammal.

12. A composition comprising a compound of claim 1 wherein R₁ is C₁-C₆ alkoxy which includes branched and unsaturated alkyl groups.

13. A composition comprising a compund of claim 1 wherein R₉ is C₁-C₆ alkoxy which includes branched and unsaturated alkyl groups.

14. A composition comprising a compound of claim 1 wherein R₁ and R₉ are both C₁-C₆ alkoxy which includes branched and unsaturated alkyl groups.

15. Use of a composition of any one of claims 11 to 13 in the manufacture of a medicament for treating a mammal who has increased propensity for thrombus formation wherein the R₁ and R₉ alkoxy groups are hydrolysable following administration to said mammal.

16. Use of a compound of claim 1 in combination with a thrombolytic agent in the manufacture of a medicament for treating a mammal who has an increased propensity for thrombus formation.

17. Use of a compound of claim 1 in combination with an anticoagulant in the manufacture of a medicament for treating a mammal who has increased propensity for thrombus formation.

18. A use according to claim 10 wherein the medicament is for administration following angioplasty.

19. A cyclic peptide containing the tripeptide sequence Arg-Gly-Asp and a thioether linkage in the cycle.

20. The cyclic peptide of claim 19 wherein the cyclic peptide contains 5 amino acids in the cycle.

21. The cyclic peptide of claim 19 wherein the cycle contains 17 or 18 atoms in a ring.

22. The cyclic peptide of claim 21 wherein the cyclic peptide contains at least one D-α-amino acid.

23. The cyclic peptide of claim 22 wherein the D-amino acid is in any position in the cycle except the Arg-Gly-Asp sequence.

24. The cyclic peptide of claim 20 wherein the sulfur of the thioether linkage is bonded to at least one oxygen.

25. A cyclic pentapeptide containing the tripeptide sequence Arg-Gly-Asp wherein the cycle comprises a peptide linkage through an amino acid sidechain.

26. The cyclic pentapeptide of claim 25 wherein the peptide linkage is through the 6 carboxyl of 2-amino-1,6-hexanedioic acid.

27. The cyclic pentapeptide of claim 26 wherein the cycle contains 17 or 18 atoms in a ring.

## Patentansprüche

1. Verbindung der Formel: 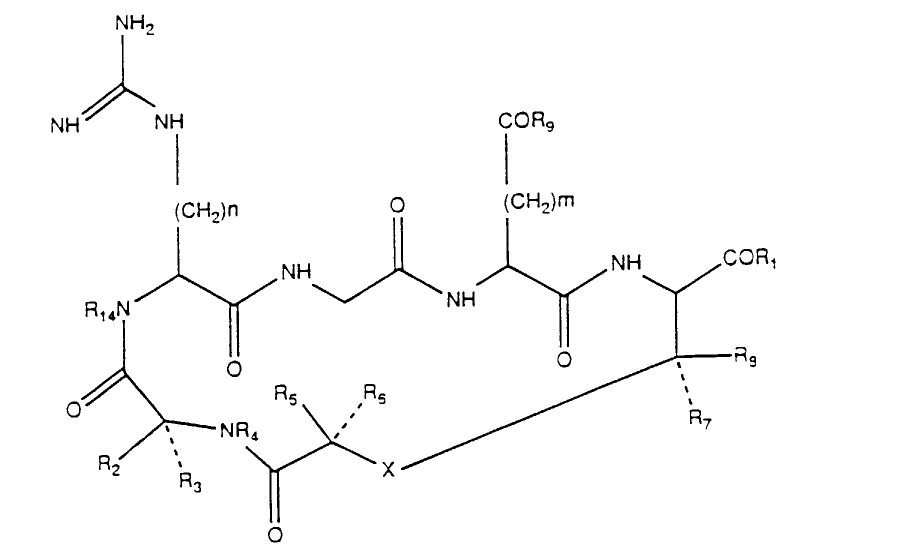 worin
R₁ und R₉ gleich oder unterschiedlich sind und ausgewählt sind aus
Hydroxy,
C₁-C₈-Alkoxy_{,}
C₃-C₁₂-Alkenoxy,
C₆-C₁₂-Aryloxy
Di-C₁-C₈-alkylamino-C₁-C₈-alkoxy,
Acylamino-C₁-C₈-alkoxy, ausgewählt aus der Gruppe Acetylaminoethoxy, Nicotinoylaminoethoxy und Succinamidethoxy,
Pivaloyloxyethoxy,
C₆-C₁₂-Aryl-C₁-C₈-alkoxy, worin die Aryl-Gruppe unsubstituiert oder substituiert ist mit einer oder mehreren der Gruppen Nitro, Halogen (F, Cl, Br, I), C₁-C₄-Alkoxy, und Amino,
Hydroxy-C₂-C₈-alkoxy,
Dihydroxy-C₃-C₈-alkoxy, und
NR₁₀R₁₁ worin R₁₀ und R₁₁ gleich oder unterschiedlich sind und Wasserstoff, C₁-C₈-Alkyl, C₃-C₈-Alkenyl, C₆-C₁₂-Aryl, worin die Aryl-Gruppe unsubstituiert oder substituiert ist mit einer oder mehreren der Gruppen Nitro, Halogen (F, Cl, Br, I), C₁-C₄-Alkoxy, und Amino, C₆-C₁₂-Aryl-C₁-C₈-alkyl, worin die Aryl-Gruppe unsubstituiert oder substituiert ist mit einer oder mehreren der Gruppen Nitro, Halogen (F, Cl, Br, I), C₁-C₄-Alkoxy, und Amino, sind;
R₁, R₃, R₅, R₆, R₇, R₈ gleich oder unterschiedlich sind und ausgewählt sind aus Wasserstoff,
C₆-C₁₂-Aryl, worin die Aryl-Gruppe unsubstituiert oder substituiert ist mit einer oder mehreren der Gruppen Nitro, Hydroxy, Halogen (F, Cl, Br, I), C₁-C₈-Alkyl, Halogen-C₁-C₈-Alkyl, C₁-C₈-alkoxy, Amino, Phenyloxy, Phenyl, Acetamido, Benzamido, Di-C₁-C₈-alkylamino, C₁-C₈-Alkylamino, C₆-C₁₂-Aroyl, C₁-C₈-Alkanoyl, und Hydroxy-C₁-C₈-alkyl,
C₁-C₁₂-Alkyl, entweder substituiert oder unsubstituiert, verzweigt oder geradkettig, worin die Substituenten ausgewählt sind aus Halogen (F, Cl, Br, I),
C₁-C₈-Alkoxy,
C₆-C₁₂-Aryloxy, worin die Aryl-Gruppe unsubstituiert oder substituiert ist mit einer oder mehreren der Gruppen Nitro, Hydroxy, Halogen (F, Cl,Br, I), C₁-C₈-Alkyl, C₁-C₈-Alkoxy, Amino, Phenyloxy, Acetamido, Benzamido, Di-C₁-C₈-alkylamino, C₁-C₈-Alkylamino, C₆-C₁₂-Aroyl, und C₁-C₈-Alkanoyl,
Isothioureido,
C₃-C₇-Cycloalkyl,
Ureido,
Amino,
C₁-C₈-Alkylamino,
Di-C₁-C₈-alkylamino,
Hydroxy,
Amino-C₂-C₈-alkylthio,
Amino-C₂-C₈ alkoxy,
Acetamido,
Benzamido, worin der Phenylring unsubstituiert oder substituiert ist mit einer oder mehreren der Gruppen Nitro, Hydroxy, Halogen (F, Cl, Br, I), C₁-C₈-Alkyl, C₁-C₈-Alkoxy, Amino, Phenyloxy, Acetamido, Benzamido, Di-C₁-C₈-alkylamino, C₁-C₈-Alkylamino, C₆-C₁₂-Aroyl, und C₁-C₈-Alkanoyl,
C₆-C₁₂-Arylamino, worin die Arylgruppe unsubstituiert oder substituiert ist mit einer oder mehreren der Gruppen Nitro, Hydroxy, Halogen, C₁-C₈-Alkyl, C₁-C₈-Alkoxy, Amino, Phenyloxy, Acetamido, Benzamido, Di-C₁-C₈-alkylamino, C₁-C₈-Alkylamino, C₆-C₁₂-Aroyl, und C₁-C₈-Alkanoyl,
Guanidino,
Phthalimido,
Mercapto,
C₁-C₈-Alkylthio,
C₆-C₁₂-Arylthio,
Carboxy,
Carboxamide,
Carbo-C₁-C₈-Alkoxy,
C₆-C₁₂-Aryl, worin die Aryl-Gruppe unsubstituiert oder substituiert ist mit einer oder mehreren der Gruppen Nitro, Hydroxy, Halogen, C₁-C₈-Alkyl, C₁-C₈-Alkoxy, Amino, Phenyloxy, Acetamido, Benzamido, Di-C₁-C₈-alkylamino, C₁-C₈-Alkylamino, Hydroxy-C₁-C₈-alkyl, C₆-C₁₂-Aroyl, und C₁-C₈-Alkanoyl, und
aromatischer Heterozyklus, worin die heterocyclischen Gruppen 5-10 Ringatome aufweisen und bis hinauf zu zwei O, N, oder S Heteroatome enthalten;
R₂ und R₃, R₅ und R₆, oder R₇ und R₈ unabhängig gegebenenfalls jeweils miteinander verbunden sind, um einen carbozyklischen oder heterozyklischen Ring mit vier bis sieben Atomen zu bilden, worin die Heteroatome ausgewählt sind aus O, S oder NR₁₂,
worin R₁₂ ausgewählt ist aus
Wasserstoff, C₁-C₈-Alkyl, C₃-C₈-Alkenyl, C₆-C₁₂-Aryl, C₆-C₁₂-Aryl-C₁-C₈-alkyl, C₁-C₈-Alkanoyl, und C₆-C₁₂-Aroyl,
R₄ ausgewählt ist aus
Wasserstoff,
C₁-C₈-Alkyl,
C₃-C₁₀-Cycloalkyl,
C₆-C₁₂-Aryl, und
C₆-C₁₂-Aryl-C₁-C₈-alkyl;
R₂ oder R₃ gegebenenfalls mit R₄ verbunden sind, um einen Piperidin-, Pyrrolidin- oder Thiazolidinring zu bilden;
R₁₄ ausgewählt ist aus
Wasserstoff, C₁-C₈-Alkyl, C₃-C₈-Alkenyl, C₆-C₁₂-Aryl, und C₆-C₁₂-Aryl-C₁-C₈-alkyl;
X ausgewählt ist aus
einem 0- oder S-Atom,
einem S-Atom, das ein oder zwei O-Atom aufweist,
NR₁₃ worin R₁₃ Wasserstoff, C₁-C₈-Alkyl, C₃-C₈-Alkenyl, C₆-C₁₂-Aryl, C₆-C₁₂-Aryl-C₁-C₈-alkyl, C₁-C₈-Alkanoyl und C₆-C₁₂-Aroyl ist, und
C₆-C₁₂-Aryl,
C₁-C₈-Alkanoyl,
(CH₂)ₖ, worin k eine ganze Zahl von 0 bis 5 ist;
n eine ganze Zahl von 1 bis 6 ist;
m eine ganze Zahl 0 bis 4 ist; und
pharmazeutisch akzeptable Salze davon.

2. Verbindung der Formel: 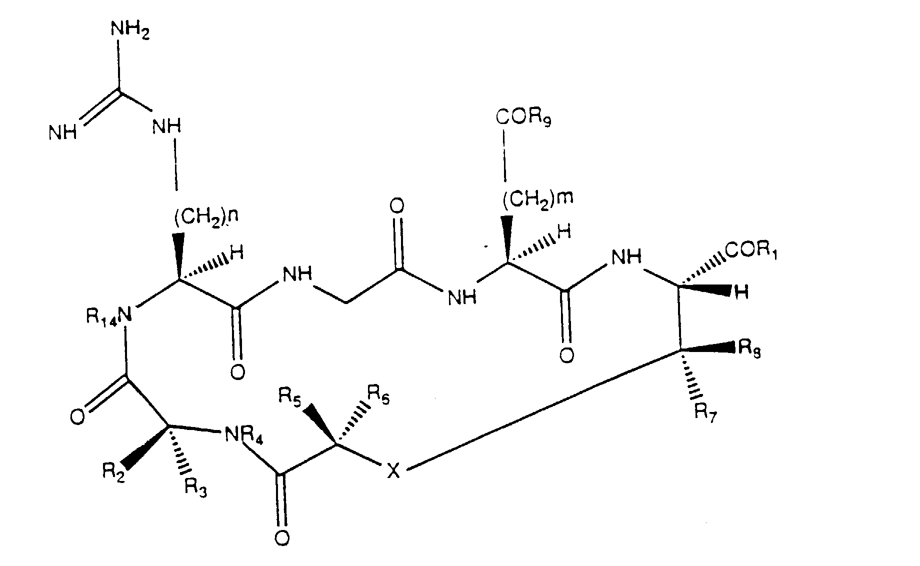 worin
R₁ und R₉ gleich oder unterschiedlich sind und Hydroxy, NH₂, C₁-C₄-Alkoxy oder Benzyloxy sind;
R₂ Wasserstoff ist
R₃ ausgewählt ist aus
Wasserstoff,
C₁-C₆-Alkyl, verzweigt oder unverzweigt, unsubstituiert oder substituiert mit Substituenten, ausgewählt aus Amino, Hydroxy, Mercapto, Methylthio, Carboxy, Carboxamide, Guanidino, Phenyl, 4-Hydroxyphenyl, 4-Methoxyphenyl, 3-Indolyl, und 4-imidazolyl,
Phenyl, entweder unsubstituiert oder substituiert mit einem bis drei Substituenten, die unabhängig Nitro, Hydroxy, Halogen (F, Cl, Br, I), C₁-C₄-Alkyl, C₁-C₄-Alkoxy, Amino, Phenyloxy, Phenyl, Acetamido, Benzamido, Di-C₁-C₄-alkylamino, C₁-C₄-Alkylamino, Halogen-C₁-C₄-alkyl, C₆-C₁₂-Aroyl, und C₁-C₄-Alkanoyl sein können,
1-Naphthyl,
2-Naphthyl,
2-Thienyl,
2-Pyridyl,
3-Pyridyl, und
4-Pyridyl;
R₅ und R₆ unabhängig ausgewählt sind aus
Wasserstoff,
C₁-C₆-Alkyl, entweder verzweigt oder unverzweigt, unsubstituiert oder substituiert mit Substituenten, die ausgewählt sind aus Amino, Hydroxy, Mercapto, Carboxy, Carboxamid, Guanidino, Phenyl oder 4-Hydroxyphenyl, 4-Methoxyphenyl, 3-Indolyl, und 4-Imidazolyl,
Phenyl, entweder unsubstituiert oder substituiert mit ein bis drei Substituenten, die unabhängig ausgewählt sind aus Nitro, Hydroxy, Halogen (F, Cl, Br, I), C₁-C₄-Alkyl, C₁-C₄-Alkoxy, Amino, Phenyloxy, Phenyl, Acetamido, Benzamido, Di-C₁-C₄-alkylamino, C₁-C₄-Alkylamino, Halogen-C₁-C₄-alkyl, C₆-C₁₂-Aroyl, und C₁-C₄-Alkanoyl,
1-Naphthyl,
2-Naphthyl,
2-Thienyl,
2-Pyridyl,
3-Pyridyl, und
4-Pyridyl;
R₇ oder R₈ gleich oder unterschiedlich sind und ausgewählt sind aus
Wasserstoff,
C₁-C₄-Alkyl,
Phenyl, entweder unsubstituiert oder substituiert mit ein bis drei Substituenten, die unabhängig ausgewählt sind aus Hydroxy, Halogen (F, Cl, Br, I), C₁-C₄-Alkyl, und C₁-C₄-Alkoxy;
R₄ Wasserstoff ist oder mit R₃ verbunden sein kann, um einen heterozyklischen Ring zu bilden, der ausgewählt ist aus Piperidin, Pyrrolidin oder Thiazolidin;
R₁₄ Wasserstoff oder Methyl ist;
X ausgewählt ist aus
einem O oder S atom,
einem S-Atom, das ein oder zwei O-Atome aufweist,
NR₁₃, worin R₁₃ ausgewählt ist aus Wasserstoff, C₁-C₄-Alkyl, Benzyl, Phenyl, C₁-C₄-Alkanoyl, Benzoyl und
(CH₂)ₖ, worin k 0 bis 5 ist;
n 3 oder 4 ist;
m 1 ist; und
pharmazeutisch akzeptable Salze davon.

3. Verbindung der Formel: 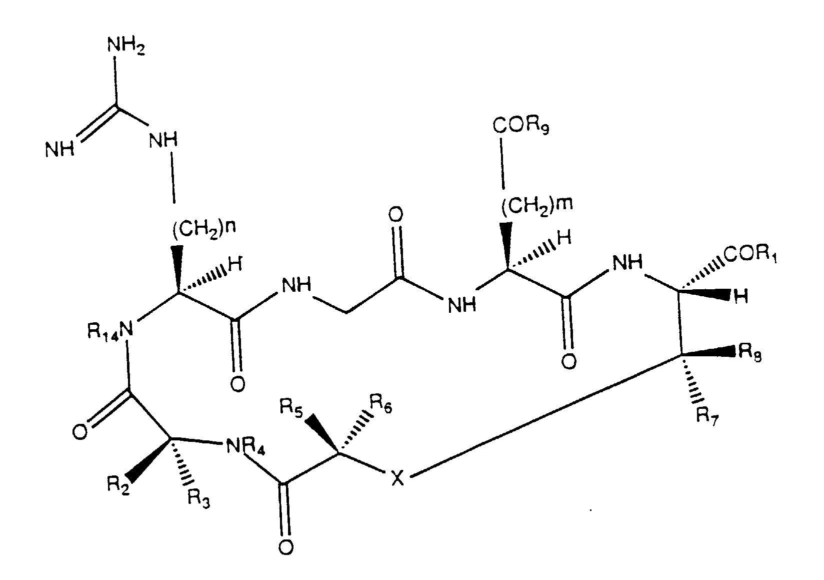 worin
R₁ und R₉ gleich oder unterschiedlich sind und Hydroxy, NH₂, C₁-C₄-Alkoxy oder Benzyloxy sind;
R₃ Wasserstoff ist,
R₂ ausgewählt ist aus
Wasserstoff,
C₁-C₆-Alkyl, verzweigt oder unverzweigt, unsubstituiert oder substituiert mit Substituenten, die ausgewählt sind aus Amino, Hydroxy, Mercapto, Methylthio, Carboxy, Carboxamid, Guanidino, Phenyl, 4-Hydroxyphenyl, 4-Methoxyphenyl, 3-Indolyl und 4-Imidazolyl,
Phenyl, entweder unsubstituiert oder substituiert mit ein bis drei Substituenten, die unabhängig Nitro, Hydroxy, Halogen (F, Cl, Br, I), C₁-C₄-Alkyl, C₁-C₄-alkoxy, Amino, Phenyloxy, Phenyl, Acetamido, Benzamido, Di-C₁-C₄-alkylamino, C₁-C₄-Alkylamino, Halogen-C₁-C₄-alkyl, C₆-C₁₂-Aroyl und C₁-C₄-Alkanoyl sein können,
1-Naphthyl,
2-Naphthyl,
2-Thienyl,
2-Pyridyl,
3-Pyridyl, und
4-Pyridyl;
R₅ und R₆ unabhängig ausgewählt sind aus
Wasserstoff,
C₁-C₆-Alkyl, entweder verzweigt oder unverzweigt, unsubstituiert oder substituiert mit Substituenten, die ausgewählt sind aus Amino, Hydroxy, Mercapto, Carboxy, Carboxamid, Guanidino, Phenyl oder 4-Hydroxyphenyl, 4-Methoxyphenyl, 3-Indolyl, und 4-Imidazolyl,
Phenyl, entweder unsubstituiert oder substitulert mit ein bis drei Substituenten, die unabhängig ausgewählt sind aus Nitro, Hydroxy, Halogen (F, Cl, Br, I), C₁-C₄-Alkyl, C₁-C₄-Alkoxy, Amino, Phenyloxy, Phenyl, Acetamido, Benzamido, Di-C₁-C₄-alkylamino, C₁-C₄-Alkylamino, Halogen-C₁-C₄-alkyl, C₆-C₁₂-Aroyl, und C₁-C₄-Alkanoyl,
1-Naphthyl,
2-Naphthyl
2-Thienyl,
2-Pyridyl,
3-Pyridyl, und
4-Pyridyl;
R₇ oder R₈ gleich oder unterschiedlich sind und ausgewählt sind aus
Wasserstoff,
C₁-C₄-Alkyl,
Phenyl, entweder unsubstituiert oder substituiert with von ein bis drei Substituenten, die unabhängig ausgewählt sind aus Hydroxy, Halogen (F, Cl, Br, I), C₁-C₄-Alkyl, und C₁-C₄-Alkoxy;
R₄ Wasserstoff ist oder mit R₃ verbunden sein kann, um einen heterozyklischen Ring zu bilden, der ausgewählt ist aus Piperidin, Pyrrolidin oder Thiazolidin;
R₁₄ Wasserstoff oder Methyl ist;
X ausgewählt ist aus
einem O oder S-Atom,
einem S-Atom, das ein oder zwei O-Atome aufweist,
NR₁₃ , worin R₁₃ ausgewählt ist aus Wasserstoff, C₁-C₄-Alkyl, Benzyl, Phenyl, C₁-C₄-Alkanoyl, Benzoyl und
(CH₂)ₖ, worin k 0 bis 5 ist;
n 3 oder 4 ist:
m 1 ist; und
pharmazeutisch akzeptable Salze davon.

4. Verbindung der Formel: 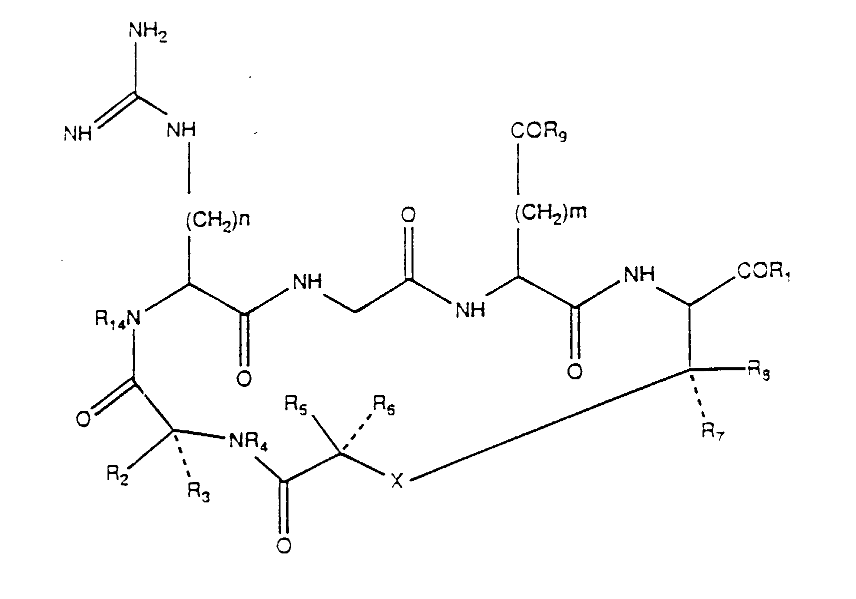 ausgewählt aus
(i) worin R₁ und R₉ OH sind; R₂, R₄, R₅, R₆, R₇ und R₈ alle H sind; R₃ 2-Propyl ist; und X CH₂ ist
(ii) worin R₁ und R₉ OH sind; R₂, R₃, R₄, R₅, R₇ und R₈ alle H sind; R₆ 1-Naphthyl ist; und X S ist.
(iii) worin R₁ und R₉ OH sind; R₂, R₄, R₅, R₆, R₇, und R₈ alle H sind; R₃ ein 4-Hydroxybenzyl ist; und X SO ist
(iv) worin R₁ und R₉ OH sind; R₂, R₄, R₅, R₆, R₇ und R₆ alle H sind; R₃ 4-Hydroxybenzyl ist; und X CH₂ ist
(v) worin R₁ und R₉ OH sind; R₂, R₄, R₅ und R₆ alle H sind; R₃ 4-Hydroxybenzyl ist; R₇ und R₈ CH₃ sind; und X SO ist
(vi) worin R₁ und R₉ OH sind; R₂, R₄, R₅, R₆, R₇ und R₈ alle H sind; R₃ 2-Propyl ist und X SO ist
(vii) worin R₁ und R₉ OH sind; R₂, R₄, R₅ und R₆ alle H sind; R₃ 4-Hydroxybenzyl ist; R₇ und R₈ CH₃ sind; und X S ist
(viii) worin R₁ NH₂ ist; R₂, R₄, R₅, R₆, R₇ und R₈ alle H sind; R₃ 4-Hydroxybenzyl ist, R₉ OH ist; und X SO ist
(ix) worin R₁ und R₉ OH sind; R₂, R₄, R₅, R₇ und R₈ alle H sind; R₃ 1-Hydroxy-1-Ethyl ist, R₆ Phenyl ist; und X S ist
(x) worin R₁ und R₉ OH sind; R₂, R₄, R₅, R₆, R₇ und R₈ alle H sind; R₃ Benzyl ist; und X SO ist
(xi) worin R₁ und R₉ OH sind; R₂, R₄, R₅, R₆, R₇ und R₈ alle H sind; R₃ 2-Methyl-1-propyl ist; und X (CH₂)_{K} und K 0 ist
(xii) worin R₁ und R₉ OH sind; R₂, R₄, R₅, R₆, R₇ und R₈ alle H sind; R₃ 1-Hydroxy1-Ethyl ist; und X SO ist
(xiii) worin R₁ und R₉ OH sind; R₂, R₄, R₅, R₆, R₇ und R₈ alle H sind; R₃ 2-Propyl ist und X S ist
(xiv) worin R₁ und R₉ OH sind; R₂, R₄, R₅, R₆, R₇ und R₈ alle H sind; R₃ 4-Amino-1-butyl ist; und X SO ist
(xv) worin R₁ und R₉ OH sind; R₂, R₄, R₅, R₆, R₇ und R₈ alle H sind; R₃ 2-Propyl ist und X (CH₂)_{K} und K 0 ist
(xvi) worin R₁ ist NH₂; R₂, R₄, R₅, R₆, R₇ und R₈ alle H sind; R₃ 4-Hydroxy-Benzyl ist; R₉ OH ist, und X S ist
(xvii) worin R₁ und R₉ OH sind; R₂, R₃, R₄, R₅, R7 und R8 alle H sind; R₆ 2-Naphthyl ist und X S ist
(xviii) worin R₁ und R₉ OH sind; R₂, R₄, R₅, R₆, R₇ und R₈ alle H sind; R₃ 2-Propyl ist; und X NH ist
(xix) worin R₁ und R₉ OH sind; R₂, R₄, R₅, R₆, R₇ und R₈ alle H sind; R₃ 1-Hydroxy1-Ethyl ist; und X S ist
(xx) worin R₁ und R₉ OH sind; R₂, R₄, R₅, R₆, R₇ und R₈ alle H sind; R₃ 4-Hydroxy-Benzyl ist; und X S ist
(xxi) worin R₁ und R₉ OH sind; R₂, R₃, R₄, R₅, R₇ und R₈ alle H sind; R₆ Phenyl ist; und X S ist
(xxii) worin R₁ und R₉ OH sind; R₂, R₄, R₅, R₆, R₇ und R₈ alle H sind; R₃ 4-Hydroxy-Benzyl ist; und X (CH₂)_{K} und K 0 ist
(xxiii) worin R₁ und R₉ OH sind; R₂, R₄, R₅, R₆, R₇ und R₈ alle H sind; R₃ 2-Methyl-1-Propyl ist; und X S ist
(xxiv) worin R₁ und R₉ OH sind; R₂, R₄, R₅, R₆, R₇ und R₈ alle H sind; R₃ Benzyl ist und X (CH₂)_{K} und K 0 ist
(xxv) worin R₁ und R₉ OH sind; R₂, R₄, R₅, R₇ und R₈ alle H sind; R₃ 4-Hydroxy-Benzyl ist; R₆ Phenyl ist und X S ist
(xxvi) worin R₁ und R₉ OH sind; R₂, R₄, R₅, R₆, R₇ und R₈ alle H sind; R₃ 3-Indolylmethyl ist; und X (CH₂)_{K} und K 0 ist
(xxvii) worin R₁ und R₉ OH sind; R₂, R₄, R₅, R₆, R₇ und R₈ alle H sind; R₃ Benzyl ist und X S ist
(xxviii) worin R₁ und R₉ OH sind; R₂, R₄, R₅, R₆, R₇ und R₈ alle H sind; R3 4-Hydroxy-Benzyl ist und X NH ist
(xxix) worin R₁ und R₉ OH sind; R₂, R₄, R₅, R₆, R₇ und R₈ alle H sind; R₃ 1-Hydroxy-Ethyl ist und X (CH₂)_{K} und K 0 ist
(xxx) worin R₁ und R₉ OH sind; R₂, R₃ R₄, R₅, R₆ alle H sind; R₇ und R₈ CH₃ sind und X S ist
(xxxi) worin R₁ und R₉ OH sind; R₂, R₃ R₄, R₅, R₆, R₇ und R₈ alle H sind; und X SO ist
(xxxii) worin R₁ und R₉ OH sind; R₂, R₅ R₆, R₇ und R₈ alle H sind; R₃ und R₄ CH₂CH₂CH₂ sind; und X S ist
(xxxiii) worin R₁ und R₉ OH sind; R₂, R₄, R₅ R₆, R₇ und R₈ alle H; R₃ 3-Indolylmethyl ist und X S ist
(xxxiv) worin R₁ und R₉ OH sind; R₂, R₄, R₅ R₆, R₇ und R₈ alle H sind; R₃ Hydroxymethyl ist und X S ist
(xxxv) worin R₁ und R₉ OH sind; R₂, R₄, R₅ R₆, R₇ und R₈ alle H sind; R₃ 4-Imidazoiyimethyl ist und X S ist
(xxxvi) worin R₁ und R₉ OH sind; R₂, R₄, R₅ R₆, R₇ und R₈ alle H sind; R₃ Methyl ist und X S ist.

5. Verbindung nach Anspruch 2 ausgewählt aus Verbindungen, worin
(i) R₁ und R₉ OH sind:
R₁, R₄, R₅ R₆, R₇ und R₈ und R₁₄ Wasserstoff sind;
R₃ ausgewählt ist aus
Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl und Carboxamidoethyl;
X S ist; und
n 3 oder 4 ist;
(ii) R₁ und R₉ OH sind;
R₂, R₄, R₅ R₆, R₇ und R₈ und R₁₄ Wasserstoff sind;
R₃ ausgewählt ist aus
Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl,4-Methoxybenzyl, 3-Indolylmethyl, 4-Imidazolylmethyl, Phenyl, Carboxymethyl, Carboxyethyl, Carboxamidomethyl, und Carboxamidoethyl;
X SO ist; und
n 3 oder 4 ist
(iii) R₁ und R₉ OH sind ;
R₂, R₃, R₄, R₇, R₈, und R₁₄ Wasserstoff sind;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4-Trifluormethylphenyl, 1-Naphthyl, 2-Naphthyl, 4-Biphenyl, und Pentafluorphenyl;
X S ist; und
n 3 oder 4 ist;
(iv) R₁ und R₉ OH sind;
R₂, R₃, R₄, R₇, R₈, und R₁₄ Wasserstoff sind;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4-Trifluormethylphenyl, 1-Naphthyl, 2-Naphthyl, 4-Biphenyl, und Pentafluorphenyl;
X SO ist und
n 3 oder 4 ist;
(v) R₁ und R₉ OH sind;
R₂, R₃, R₄, R₅, R₆, und R₁₄ Wasserstoff sind:
R₇ und R₈ unabhängig ausgewählt sind aus Wasserstoff, Methyl, und Phenyl;
X S ist; und
n 3 oder 4 ist;
(vi) R₁ und R₉ OH sind;
R₂, R₃, R₄, R₅, R₆, und R₁₄ Wasserstoff sind;
R₇ und R₈ unabhängig ausgewählt sind aus Wasserstoff, Methyl und Phenyl;
X SO ist, und
n 3 oder 4 ist;
(vii) R₁ und R₉ OH sind;
R₂, R₄, R₇, R₈, und R₁₄ Wasserstoff sind;
R₃ ausgewählt ist aus 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4-Trifluormethylphenyl, 1-Naphthyl, 2-Naphthyl, 4-Biphenyl, und Pentafluorphenyl;
X S ist; und
n 3 oder 4 ist;
(viii) R₁ und R₉ OH sind;
R₂, R₄, R₇, R₈, und R₁₄ Wasserstoff sind;
R₃ ausgewählt ist aus 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl und Carboxamidoethyl;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4-Trifluormethylphenyl, 1-Naphthyl, 2-Naphthyl, 4-Biphenyl und Pentafluorphenyl;
X SO ist; und
n 3 oder 4 ist;
(ix) R₁ und R₉ OH sind;
R₂, R₄, R₅, R₆, und R₁₄ Wasserstoff sind;
R₃ ausgewählt ist aus 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-imidazolylmethyl, Phenyl, Carboxymethyl, Carboxyethyl, Carboxamidomethyl, und Carboxamidoethyl;
R₇ und R₈ unabhängig ausgewählt sind aus Wasserstoff, Methyl, und Phenyl;
X S ist; und
n 3 oder 4 ist;
(x) R₁ und R₉ OH sind;
R₂, R₄, R₅, R₆, und R₁₄ Wasserstoff sind;
R₃ ausgewählt ist aus 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
R₇ und R₈ unabhängig ausgewählt sind aus Wasserstoff, Methyl und Phenyl;
X SO ist; und
n 3 oder 4 ist;
(xi) R₁ und R₉ OH sind;
R₂, R₃, R₄, und R₁₄ Wasserstoff sind;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4-Trifluormethylphenyl, 1-Naphthyl, 2-Naphthyl, 4-Biphenyl, und Pentafluorphenyl;
R₇ und R₈ unabhängig ausgewählt sind aus Wasserstoff, Methyl und Phenyl;
X S ist; und
n 3 oder 4 ist;
(xii) R₁ und R₉ OH sind;
R₂, R₃, R₄, und R₁₄ Wasserstoff sind;
R₅ und R₆ unabhängig ausgewählt sind aus Wasserstoff, Methyl, Ethyl, Propyl, Isopropyl, Butyl, Isobutyl, Benzyl, Phenyl, 2-Trifluormethylphenyl, 3-Trifluormethylphenyl, 4- Trifluormethylphenyl, 1-Naphthyl, 2-Naphthyl, 4-Biphenyl, und Pentafluorphenyl;
R₇ und R₈ unabhängig ausgewählt sind aus Wasserstoff, Methyl und Phenyl;
X SO ist; und
n 3 oder 4 ist;
(xiii) R₁ und R₉ OH sind;
R₂, R₄, R₅, R₆, R₇, R₈ und R₁₄ Wasserstoff sind;
R₃ ausgewählt ist aus Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methyithioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
X 0 ist; und
n 3 oder 4 ist;
(xiv) R₁ und R₉ OH sind;
R₂, R₄, R₅, R₆, R₇, R₈, und R₁₄ Wasserstoff sind;
R₃ ausgewählt ist aus Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
X NR₁₃ ist, worin R₁₃ ausgewählt ist aus Wasserstoff, C₁-C₄-Alkyl, C₁-C₄-Alkanoyl, Benzoyl und Benzyl; und
n 3 oder 4 ist;
(xv) R₁ und R₉ OH sind;
R₂, R₄, R₅, R₆, R₇, R₈, und R₁₄ Wasserstoff sind;
R₃ 4-Hydroxybenzyl und;
X S ist; und
n 3 ist;
(xvi) R₁ und R₉ OH sind;
R₂, R₄, R₅, R₆, R₇, R₈, und R₁₄ Wasserstoff sind;
R₃ 4-Hydroxybenzyl ist;
X SO ist; und
n 3 ist;
(xvii) R₁ und R₉ OH sind;
R₂, R₄, R₅, R₆, R₇, R₈, und R₁₄ Wasserstoff sind;
R₃ Isopropyl ist;
X S ist; und
n 3 ist;
(xviii) R₁ und R₉ OH sind;
R₂, R₄, R₅, R₆, R₇, R₈, und R₁₄ Wasserstoff sind;
R₃ Isopropyl ist;
X SO ist; und
n 3 ist;
(xix) R₁ und R₉ OH sind;
R₂, R₄, R₅, R₆, R₇, R₈, und R₁₄ Wasserstoff sind;
R₃ 2-Butyl ist;
X S ist; und
n 3 ist;
(xx) R₁ und R₉ OH sind;
R₂, R₄, R₅, R₆, R₇, R₈, und R₁₄ Wasserstoff sind;
R₃ 2-Butyl ist;
X SO ist; und
n 3 ist;
(xxi) R₁ und R₉ OH sind;
R₂, R₅, R₆, R₇, R₈, und R₁₄ Wasserstoff sind;
R₃ und R₄ miteinander verbunden sind, um einen Pyrrolidinring zu bilden;
X S ist; und
n 3 ist;
(xxii) R₁ und R₉ OH sind;
R₂, R₅, R₆, R₇, R₈, und R₁₄ Wasserstoff sind;
R₃ und R₄ miteinander verbunden sind, um einen Pyrrolidinring zu bilden;
X SO ist; und
n 3 ist;
(xxiii) R₁ und R₉ OH sind;
R₂, R₄, R₅, R₆, und R₁₄ Wasserstoff sind;
R₃ 4-Hydroybenzyl ist;
R₇ und R₈ Methyl sind;
X S ist; und
n 3 ist;
(xxiv) R₁ und R₉ OH sind;
R₂, R₄, R₅, R₆, und R₁₄ Wasserstoff sind;
R₃ 4-Hydroybenzyl ist;
R₇ und R₈ Methyl sind;
X SO ist; und
n 3 ist;
(xxv) R₁ und R₉ OH sind;
R₂, R₄, R₅, R₆, und R₁₄ Wasserstoff sind;
R₃ Isopropyl ist;
R₇ und R₈ Methyl sind;
X S ist; und
n 3 ist;
(xxvi) R₁ und R₉ OH sind;
R₂, R₄, R₅, R₆, und R₁₄ Wasserstoff sind;
R₃ Isopropyl ist;
R₇ und R₈ Methyl sind;
X SO ist; und
n 3 ist;
(xxvii) R₁ und R₉ OH sind;
R₂, R₃, R₄, R₅, R₆, und R₁₄ Wasserstoff sind;
R₇ und R₈ Methyl sind;
X S ist; und
n 3 ist;
(xxviii) R₁ und R₉ OH sind;
R₂, R₃, R₄, R₅, R₆, und R₁₄ Wasserstoff sind;
R₇ und R₈ Methyl sind;
X SO ist; und
n 3 ist;
(xxix) R₁ NH₂ ist;
R₉ OH ist;
R₂, R₄, R₅, R₆, R₇, R₈, und R₁₄ Wasserstoff sind;
R₃ ausgewählt ist aus Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
X S ist; und
n 3 oder 4 ist;
(xxx) R₁ NH₂ ist;
R₉ OH ist;
R₂, R₄, R₅, R₆, R₇, R₈, und R₁₄ Wasserstoff sind;
R₃ ausgewähit ist aus Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
X SO ist; und
n 3 oder 4 ist;
(xxxi) R₁ und R₉ OH sind;
R₂, R₄, R₅, R₆, R₇, R₈, und R₁₄ Wasserstoff sind;
R₃ ausgewählt ist aus Wasserstoff, 4-Amino-1-butyl, 3-Amino-1-propyl, Hydroxymethyl, 2-Hydroxy-1-propyl, Methyl, Ethyl, 2-Propyl, Isobutyl, n-Propyl, n-Butyl, 2-Butyl, Methylthioethyl, Benzyl, 4-Hydroxybenzyl, 4-Methoxybenzyl, 3-Indolylmethyl, 4-Imidazolylmethyl, Phenyl, Carboxymethyl, Carboxyethyl, Carboxamidomethyl, und Carboxamidoethyl;
X (CH₂)ₖ ist, worin k 0,1 oder 2 ist; und
n 3 oder 4 ist;
(xxxii) R₁ und R₉ OH sind;
R₂, R₄, R₅, R₆, R₇, R₈, und R₁₄ Wasserstoff sind;
R₃ 4-Hydroxybenzyl ist;
X CH₂ ist; und
n 3 ist;
(xxxiii) R₁ und R₉ OH sind;
R₂, R₄, R₅, R₆, R₇, R₈, und R₁₄ Wasserstoff sind;
R₃ Isopropyl ist;
X CH₂ ist; und
n 3 ist;
(xxxiv) und R₉ OH sind;
R₂, R₄, R₅, R₆, R₇, R₈, und R₁₄ Wasserstoff sind;
R₃ 2-Butyl ist;
X CH₂ ist; und
n 3 ist;
(xxxv) R₁ und R₉ OH sind;
R₂, R₅, R₆, R₇, R₈, und R₁₄ Wasserstoff sind;
R₃ und R₄ miteinander verbunden sind, um einen Pyrrolidinring zu bilden;
X CH₂ ist; und
n 3 ist;

6. Verfahren zur Herstellung der Verbindung nach Anspruch 2, das folgende Schritte umfaßt:
(a) Zyklisieren eines Zwischenprodukts der Formel IV 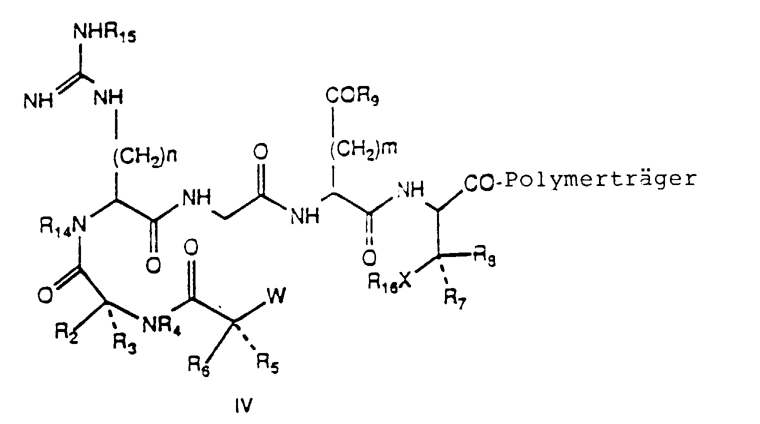 worin
R₁-R₉, n und m wie in Anspruch 2 definiert sind,
W Br, Cl, oder I ist,
R₁₄ ausgewählt ist aus Wasserstoff, C₁-C₆-Alkyl, und C₆-C₁₂-Aryl
R₁₅ 2,2,5,7,8-Pentamethylchroman-6-sulfonyl ist,
R₁₆ Wasserstoff ist, und
X S ist;
(b) Abspalten des gewünschten Produkts vom Harz;
(c) Abspalten aller Schutzgruppen; und
(d) Reinigen und Isolieren des biologisch aktiveren Isomers.

7. Verfahren zur Herstellung der Verbindung nach Anspruch 2, das folgende Schritte umfaßt:
(a) Zyklisieren eines Zwischenprodukts der Formel VI 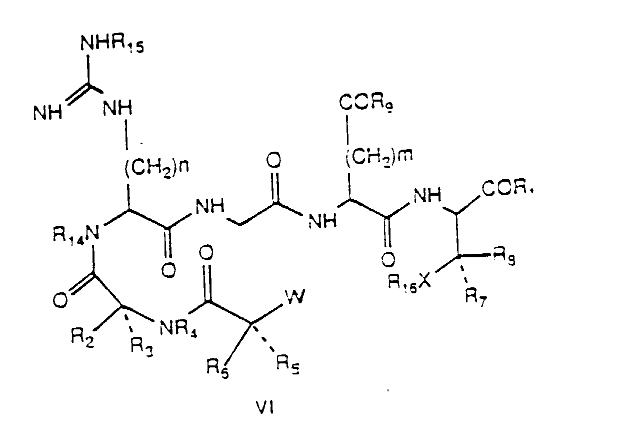 worin
R₁-R₉, n und m wie in Anspruch 2 definiert sind,
W Br, Cl, oder I ist,
R₁₄ ausgewählt ist aus Wasserstoff, C₁-C₆-Alkyl, und C₆-C₁₂-Aryl
R₁₅ 2,2,5,7,8-Pentamethylchroman-6-sulfonyl ist,
R₁₆ H ist, und
X S ist;
(b) Abspalten aller Schutzgruppen; und
(c) Reinigen und Isolieren des biologisch aktiveren Isomers.

8. Pharmazeutische Zusammensetzung, umfassend einen pharmazeutisch akzeptablen Exzipient und die Verbindung nach Anspruch 1.

9. Verwendung einer Verbindung nach Anspruch 1 bei der Herstellung eines Medikaments zur Hemmung der Blutplättchenaggregation.

10. Verwendung einer Verbindung nach Anspruch 1 bei der Herstellung eines Medikaments zur Verabreichung an ein Säugetier mit einer erhöhten Neigung zur Trombusbildung.

11. Zusammensetzung, umfassend eine Verbindung nach Anspruch 1 jeweils zur :
(a) Reduktion der Blutplättchenaggregation;
(b) Behandlung eines Säugetiers, das eine erhöhte Neigung zur Trombusbildung aufweist;
(c) Hemmung der Fibrinogenbindung an Blutplättchen in einem Säugetier.

12. Zusammensetzung, umfassend eine Verbindung nach Anspruch 1, worin R₁ C₁-C₆-Alkoxy ist, das verzweigte und ungesättigte Alkylgruppen einschließt.

13. Zusammensetzung, umfassend eine Verbindung nach Anspruch 1, worin R₉ C₁-C₆-Alkoxy ist, das verzweigte und ungesättigte Alkylgruppen einschließt.

14. Zusammensetzung, umfassend eine Verbindung nach Anspruch 1, worin R₁ und R₉ C₁-C₆-Alkoxy sind, die verzweigte und ungesättigte Alkylgruppen einschliessen.

15. Verwendung einer Zusammensetzung nach einem der Ansprüche 11 bis 13 bei der Herstellung eines Medikaments zur Behandlung eines Säugetiers mit erhöhter Neigung zur Thrombusbildung, worin die R₁- und R₉-Alkoxygruppen nach der Verabreichung an dieses Säugetier hydrolysierbar sind.

16. Verwendung einer Verbindung nach Anspruch 1 in Kombination mit einem thrombolytischen Mittel bei der Herstellung eines Medikaments zur Behandlung eines Säugetiers, das eine erhöhte Neigung zur Thrombusbildung aufweist.

17. Verwendung einer Verbindung nach Anspruch 1 in Kombination mit einem Antikoagulans bei der Herstellung eines Medikaments zur Behandlung eines Säugetiers, das eine erhöhte Neigung zur Thrombusbildung aufweist.

18. Verwendung nach Anspruch 10, worin das Medikament zur Verabreichung nach Angioplastie dient.

19. Zyklisches Peptid, das die Tripeptidsequenz Arg-Gly-Asp und eine Thioetherbindung im Zyklus enthält.

20. Zyklisches Peptid nach Anspruch 19, worin das zyklische Peptid 5 Aminosäuren im Zyklus enthält.

21. Zyklisches Peptid nach Anspruch 19, worin der Zyklus 17 oder 18 Atome im Ring enthält.

22. Zyklisches Peptid nach Anspruch 21, worin das zyklische Peptid zumindest eine D-α-Aminosäure enthält.

23. Zyklisches Peptid nach Anspruch 22, worin die D-α-Aminosäure sich in einer beliebigen Position im Zyklus befindet, mit Ausnahme der Arg-Gly-Asp-Sequenz.

24. Zyklisches Peptid nach Anspruch 20, worin der Schwefel der Thioetherbindung an zumindest einen Sauerstoff gebunden ist.

25. Zyklisches Pentapeptid, das die Tripeptidsequenz Arg-Gly-Asp enthält, worin der Zyklus eine Peptidbindung über eine Aminosäurenseitenkette umfaßt.

26. Zyklisches Pentapeptid nach Anspruch 25, worin die Peptidbindung über das 6-Carboxyl der 2-Amino-1,6-hexadecandisäure erfolgt.

27. Zyklisches Pentapeptid nach Anspruch 26, worin der Zyklus 17 oder 18 Atome im Ring enthält.

## Revendications

1. Composé de formule : 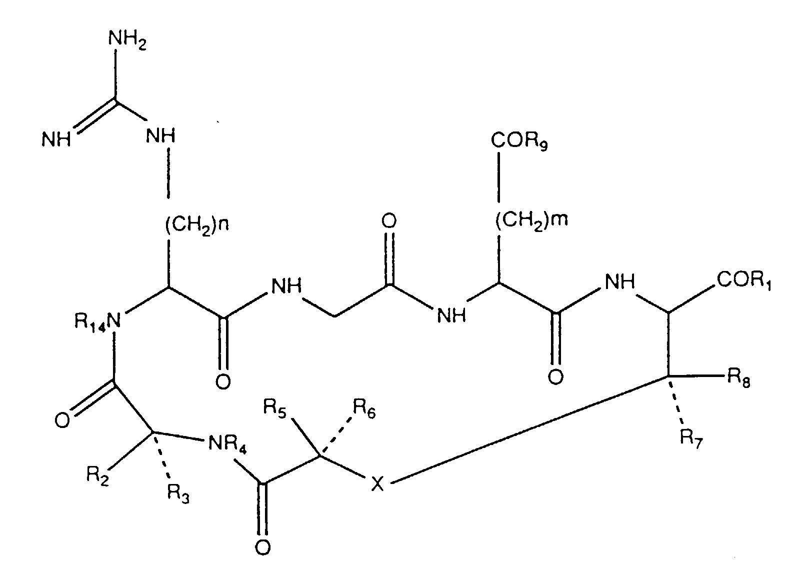 dans laquelle
R₁ et R₉ sont identiques ou différents et sont choisis entre des groupes
hydroxy,
alkoxy en C₁ à C₈,
alcénoxy en C₃ à C₁₂,
aryloxy en C₆ à C₁₂,
di(alkyle en C₁ à C₈)-amino-(alkoxy en C₁ à C₈),
acylamino-alkoxy en C₁ à C₈ choisi dans le groupe consistant en les groupes acétylaminoéthoxy, nicotinoylaminoéthoxy et succinamidoéthoxy,
pivaloyloxyéthoxy,
(aryle en C₆ à C₁₂)-(alkoxy en C₁ à C₈) dans lequel le groupe aryle est non substitué ou substitué avec un ou plusieurs des groupes nitro, halogéno (F, CI, Br, I), alkoxy en C₁ à C₄ et amino,
hydroxyalkoxy en C₂ à C₈,
dihydroxyalkoxy en C₃ à C₈, et
NR₁₀R₁₁ dans lequel R₁₀ et R₁₁ sont identiques ou différents et représentent l'hydrogène, un groupe alkyle en C₁ à C₈, alcényle en C₃ à C₈, aryle en C₆ à C₁₂, le groupe aryle étant non substitué ou substitué avec un ou plusieurs des groupes nitro, halogéno (F, Cl, Br, I), alkoxy en C₁ à C₄ et amino, (aryle en C₆ à C₁₂)-(alkyle en C₁ à C₈) dans lequel le groupe aryle est non substitué ou substitué avec un ou plusieurs des groupes nitro, halogéno (F, Cl, Br, I), alkoxy en C₁ à C₄ et amino ;
R₂, R₃, R₅, R₆, R₇ et R₈ sont identiques ou différents et sont choisis entre
l'hydrogène,
un groupe aryle en C₆ à C₁₂, le groupe aryle étant non substitué ou substitué avec un ou plusieurs des groupes nitro, hydroxy, halogéno (F, Cl, Br, I), alkyle en C₁ à C₈, halogénalkyle en C₁ à C₈, alkoxy en C₁ à C₈, amino, phényloxy, phényle, acétamido, benzamido, di(alkyle en C₁ à C₈)-amino, alkylamino en C₁ à C₈, aroyle en C₆ à C₁₂, alcanoyle en C₁ à C₈ et hydroxyalkyle en C₁ à C₈,
alkyle en C₁ à C₁₂ substitué ou non substitué, à chaîne ramifiée ou à chaîne droite, dans lequel les substituants sont choisis entre des substituants halogéno (F, Cl, Br, I),
alkoxy en C₁ à C₈,
aryloxy en C₆ à C₁₂ dans lequel le groupe aryle est non substitué ou substitué avec un ou plusieurs des groupes nitro, hydroxy, halogéno (F, Cl, Br, I), alkyle en C₁ à C₈, alkoxy en C₁ à C₈, amino, phényloxy, acétamido, benzamido, di(alkyle en C₁ à C₈)-amino, alkylamino en C₁ à C₈, aroyle en C₆ à C₁₂ et alcanoyle en C₁ à C₈,
isothio-uréido,
cycloalkyle en C₃ à C₇,
uréido,
amino,
alkylamino en C₁ à C₈,
di(alkyle en C₁ à C₈)amino
hydroxy,
aminoalkylthio en C₂ à C₈,
aminoalkoxy en C₂ à C₈,
acétamido,
benzamido dans lequel le noyau phényle est non substitué ou substitué avec un ou plusieurs des groupes nitro, hydroxy, halogéno (F, Cl, Br, I), alkyle en C₁ à C₈, alkoxy en C₁ à C₈, amino, phényloxy, acétamido, benzamido, di(alkyle en C₁ à C₈)amino, alkylamino en C₁ à C₈, aroyle en C₆ à C₁₂ et alcanoyle en C₁ à C₈,
arylamino en C₆ à C₁₂ dans lequel le groupe aryle est non substitué ou substitué avec un ou plusieurs des groupes nitro, hydroxy, halogéno, alkyle en C₁ à C₈, alkoxy en C₁ à C₈, amino, phényloxy, acétamido, benzamido, di(alkyle en C₁ à C₈)amino, alkylamino en C₁ à C₈, aroyle en C₆ à C₁₂ et alcanoyle en C₁ à C₈,
guanidino,
phtalimido,
mercapto,
alkylthio en C₁ à C₈,
arylthio en C₆ à C₁₂,
carboxy,
carboxamide,
carbo-(alkoxy en C₁ à C₈),
aryle en C₆ à C₁₂, le groupe aryle étant non substitué ou substitué avec un ou plusieurs des groupes nitro, hydroxy, halogéno, alkyle en C₁ à C₈, alkoxy en C₁ à C₈, amino, phényloxy, acétamido, benzamido, di(alkyle en C₁ à C₈)amino, alkylamino en C₁ à C₈, hydroxyalkyle en C₁ à C₈, aroyle en C₆ à C₁₂ et alcanoyle en C₁ à C₈, et
un hétérocycle aromatique, les groupes hétérocycliques ayant 5 à 10 atomes dans le noyau et contenant jusqu'à 2 hétéroatomes de O, N ou S ;
R₂ et R₃, R₅ et R₆ ou R₇ et R₈ peuvent facultativement et indépendamment être joints l'un à l'autre pour former un noyau carbocyclique ou hétérocyclique de 4 à 7 atomes dans lequel les hétéroatomes sont choisis entre O, S et NR₁₂ dans lequel R₁₂ est choisi entre
l'hydrogène, un groupe alkyle en C₁ à C₈, alcényle en C₃ à C₈, aryle en C₆ à C₁₂, (aryle en C₆ à C₁₂)-(alkyle en C₁ à C₈), alcanoyle en C₁ à C₈ et aroyle en C₆ à C₁₂, R₄ est choisi entre
l'hydrogène,
un groupe alkyle en C₁ à C₈,
un groupe cycloalkyle en C₃ à C₁₀,
un groupe aryle en C₆ à C₁₂, et
un groupe (aryle en C₆ à C₁₂) - (alkyle en C₁ à C₈) ; R₂ ou R₃ peut être facultativement joint à R₄ pour former un noyau pipéridine, pyrrolidine ou thiazolidine ; R₁₄ est choisi entre
l'hydrogène, des groupes alkyle en C₁ à C₈, alcényle en C₃ à C₈, aryle en C₆ à C₁₂ et (aryle en C₆ à C₁₂)-(alkyle en C₁ à C₈) ; X est choisi entre
un atome de O ou S,
un atome de S portant un ou deux atomes de O,
un groupe NR₁₃ dans lequel R₁₃ représente l'hydrogène, un groupe alkyle en C₁ à C₈, alcényle en C₃ à C₈, aryle en C₆ à C₁₂, (aryle en C₆ à C₁₂)-(alkyle en C₁ à C₈), alcanoyle en C₁ à C₈ ou aroyle en C₆ à C₁₂), et
un groupe aryle en C₆ à C₁₂,
un groupe alcanoyle en C₁ à C₈,
un groupe (CH₂)ₖ dans lequel k est un nombre entier de 0 à 5 ;
n est un nombre entier de 1 à 6 ;
m est un nombre entier de 0 à 4 ; et
ses sels pharmaceutiquement acceptables.

2. Composé de formule 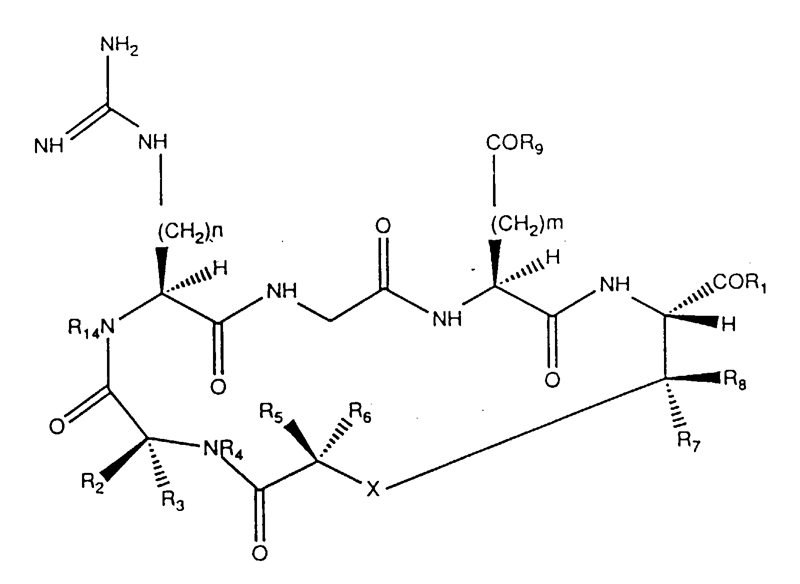 dans laquelle
R₁ et R₉ sont identiques ou différents et représentent des groupes hydroxy, NH₂, alkoxy en C₁ à C₄ ou benzyloxy ; R₂ représente l'hydrogène
R₃ est choisi entre
l'hydrogène,
un groupe alkyle en C₁ à C₆ ramifié ou non ramifié, non substitué ou substitué avec des substituants choisis entre les substituants amino, hydroxy, mercapto, méthylthio, carboxy, carboxamide, guanidino, phényle, 4-hydroxyphényle, 4-méthoxyphényle, 3-indolyle et 4-imidazolyle,
un groupe phényle non substitué ou substitué avec un à trois substituants qui peuvent consister indépendamment en substituants nitro, hydroxy, halogéno (F, CI, Br, I), alkyle en C₁ à C₄, alkoxy en C₁ à C₄, amino, phényloxy, phényle, acétamido, benzamido, di(alkyle en C₁ à C₄)amino, alkylamino en C₁ à C₄, halogénalkyle en C₁ à C₄, aroyle en C₆ à C₁₂ et alcanoyle en C₁ à C₄,
un groupe 1-naphtyle,
un groupe 2-naphtyle,
un groupe 2-thiényle,
un groupe 2-pyridyle,
un groupe 3-pyridyle, et
un groupe 4-pyridyle ;
R₅ et R₆ sont choisis indépendamment entre
l'hydrogène,
un groupe alkyle en C₁ à C₆ ramifié ou non ramifié, non substitué ou substitué avec des substituants choisis entre les substituants amino, hydroxy, mercapto, carboxy, carboxamide, guanidino, phényle ou 4-hydroxyphényle, 4-méthoxyphényle, 3-indolyle et 4-imidazolyle,
un groupe phényle non substitué ou substitué avec un à trois substituants qui peuvent être choisis indépendamment entre des substituants nitro, hydroxy, halogéno (F, CI, Br, I), alkyle en C₁ à C₄, alkoxy en C₁ à C₄, amino, phényloxy, phényle, acétamido, benzamido, di(alkyle en C₁ à C₄)amino, alkylamino en C₁ à C₄, halogénalkyle en C₁ à C₄, aroyle en C₆ à C₁₂ et alcanoyle en C₁ à C₄,
un groupe 1-naphtyle,
un groupe 2-naphtyle,
un groupe 2-thiényle,
un groupe 2-pyridyle,
un groupe 3-pyridyle, et
un groupe 4-pyridyle ;
R₇ et R₈ sont identiques ou différents et sont choisis entre
l'hydrogène,
un groupe alkyle en C₁ à C₄,
un groupe phényle non substitué ou substitué avec un à trois substituants choisis indépendamment entre des substituants hydroxy, halogéno (F, Cl, Br, I), alkyle en C₁ à C₄ et alkoxy en C₁ à C₄ ;
R₄ représente l'hydrogène ou peut être joint à R₃ pour former un noyau hétérocyclique choisi entre les noyaux pipéridine, pyrrolidine et thiazolidine ;
R₁₄ représente l'hydrogène ou un groupe méthyle ;
X est choisi entre
un atome de O ou S,
un atome de S portant un ou deux atomes de O,
un groupe NR₁₃ dans lequel R₁₃ est choisi entre l'hydrogène, des groupes alkyle en C₁ à C₄, benzyle, phényle, alcanoyle en C₁ à C₄, benzoyle, et
un groupe (CH₂)ₖ, dans lequel k a une valeur de 0 à 5 ;
n est égal à 3 ou 4 ;
m est égal à 1 ; et
ses sels pharmaceutiquement acceptables.

3. Composé de formule 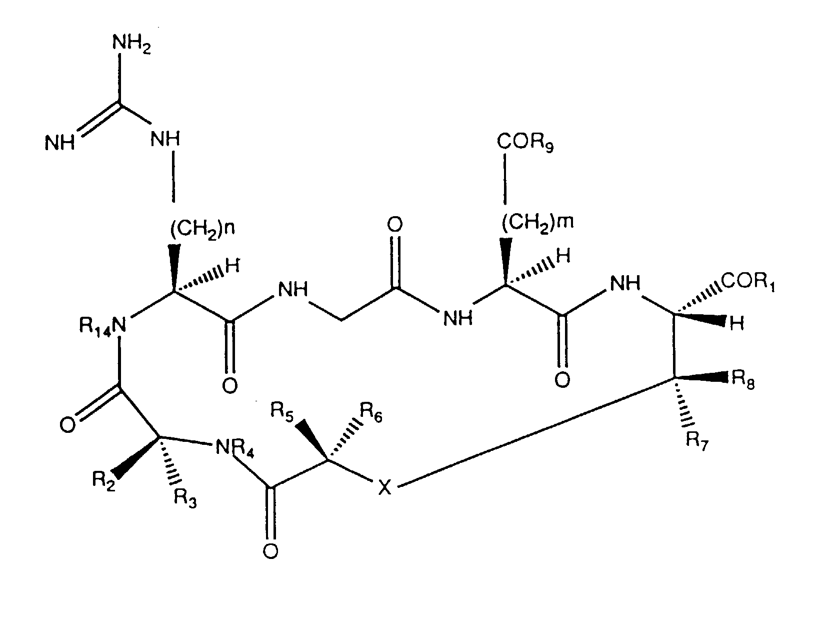 dans laquelle
R₁ et R₉ sont identiques ou différents et représentent des groupes hydroxy, NH₂, alkoxy en C₁ à C₄ ou benzyloxy ;
R₃ représente l'hydrogène
R₂ est choisi entre
l'hydrogène,
un groupe alkyle en C₁ à C₆ ramifié ou non ramifié, non substitué ou substitué avec des substituants choisis entre les substituants amino, hydroxy, mercapto, méthylthio, carboxy, carboxamide, guanidino, phényle, 4-hydroxyphényle, 4-méthoxyphényle, 3-indolyle et 4-imidazolyle,
un groupe phényle non substitué ou substitué avec un à trois substituants qui peuvent être choisis indépendamment entre des substituants nitro, hydroxy, halogéno (F, Cl, Br, I), alkyle en C₁ à C₄, alkoxy en C₁ à C₄, amino, phényloxy, phényle, acétamido, benzamido, di(alkyle en C₁ à C₄)amino, alkylamino en C₁ à C₄, halogénalkyle en C₁ à C₄, aroyle en C₆ à C₁₂ et alcanoyle en C₁ à C₄,
un groupe 1-naphtyle,
un groupe 2-napthyle,
un groupe 2-thiényle,
un groupe 2-pyridyle,
un groupe 3-pyridyle, et
un groupe 4-pyridyle ;
R₅ et R₆ sont choisis indépendamment entre
l'hydrogène,
un groupe alkyle en C₁ à C₆ ramifié ou non ramifié, non substitué ou substitué avec des substituants choisis entre les substituants amino, hydroxy, mercapto, carboxy, carboxamide, guanidino, phényle ou 4-hydroxyphényle, 4-méthoxyphényle, 3-indolyle et 4-imidazolyle,
un groupe phényle non substitué ou substitué avec un à trois substituants qui peuvent être choisis indépendamment entre des substituants nitro, hydroxy, halogéno (F, CI, Br, I), alkyle en C₁ à C₄, alkoxy en C₁ à C₄, amino, phényloxy, phényle, acétamido, benzamido, di(alkyle en C₁ à C₄)amino, alkylamino en C₁ à C₄, halogénalkyle en C₁ à C₄, aroyle en C₆ à C₁₂ et alcanoyle en C₁ à C₄,
un groupe 1-naphtyle,
un groupe 2-naphtyle,
un groupe 2-thiényle,
un groupe 2-pyridyle,
un groupe 3-pyridyle, et
un groupe 4-pyridyle ;
R₇ et R₈ sont identiques ou différents et sont choisis entre
l'hydrogène,
un groupe alkyle en C₁ à C₄,
un groupe phényle non substitué ou substitué avec un à trois substituants choisis indépendamment entre des substituants hydroxy, halogéno (F, Cl, Br, I), alkyle en C₁ à C₄ et alkoxy en C₁ à C₄ ;
R₄ représente l'hydrogène ou peut être joint à R₃ pour former un noyau hétérocyclique choisi entre les noyaux pipéridine, pyrrolidine et thiazolidine ;
R₁₄ représente l'hydrogène ou un groupe méthyle ;
X est choisi entre
un atome de O ou S,
un atome de S portant un ou deux atomes de O,
un groupe NR₁₃ dans lequel R₁₃ est choisi entre l'hydrogène, des groupes alkyle en C₁ à C₄, benzyle, phényle, alcanoyle en C₁ à C₄, benzoyle, et
un groupe (CH₂)ₖ, dans lequel k a une valeur de 0 à 5 ;
n est égal à 3 ou 4 ;
m est égal à 1 ; et
ses sels pharmaceutiquement acceptables.

4. Composé de formule : 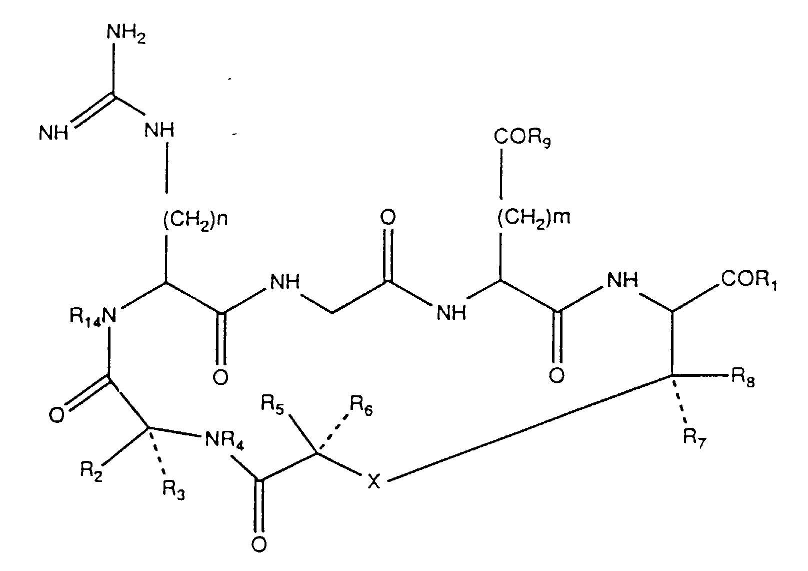 choisie entre
(i) la formule dans laquelle R₁ et R₉ représentent l'un et l'autre un groupe OH ; R₂, R₄, R₅, R₆, R₇ et R₈ représentent tous H ; R₃ représente un groupe 2-propyle ; et X représente un groupe CH₂ ;
(ii) la formule dans laquelle R₁ et R₉ représentent l'un et l'autre un groupe OH ; R₂, R₃, R₄, R₅, R₇ et R₈ représentent tous H ; R₆ représente un groupe 1-naphtyle ; et X représente S ;
(iii) la formule dans laquelle R₁ et R₉ représentent l'un et l'autre un groupe OH ; R₂, R₄, R₅, R₆, R₇ et R₈ représentent tous H ; R₃ représente un groupe 4-hydroxybenzyle ; et X représente un groupe SO ;
(iv) la formule dans laquelle R₁ et R₉ représentent l'un et l'autre un groupe OH ; R₂, R₄, R₅, R₆, R₇ et R₈ représentent tous H ; R₃ représente un groupe 4-hydroxybenzyle ; et X représente un groupe CH₂ ;
(v) la formule dans laquelle R₁ et R₉ représentent l'un et l'autre un groupe OH ; R₂, R₄, R₅ et R₆ représentent tous H ; R₃ représente un groupe 4-hydroxybenzyle ; R₇ et R₈ représentent l'un et l'autre un groupe CH₃ ; et X représente un groupe SO ;
(vi) la formule dans laquelle R₁ et R₉ représentent l'un et l'autre un groupe OH ; R₂, R₄, R₅, R₆, R₇ et R₈ représentent tous H ; R₃ représente un groupe 2-propyle et X représente un groupe SO ;
(vii) la formule dans laquelle R₁ et R₉ représentent l'un et l'autre un groupe OH ; R₂, R₄, R₅ et R₆ représentent tous H ; R₃ représente un groupe 4-hydroxybenzyle ; R₇ et R₈ représentent l'un et l'autre un groupe CH₃ ; et X représente S ;
(viii) la formule dans laquelle R₁ représente un groupe NH₂ ; R₂, R₄, R₅, R₆, R₇ et R₈ représentent tous H ; R₃ représente un groupe 4-hydroxybenzyle, R₉ représente un groupe OH ; et X représente un groupe SO ;
(ix) la formule dans laquelle R₁ et R₉ représentent l'un et l'autre un groupe OH ; R₂, R₄, R₅, R₇ et R₈ représentent tous H ; R₃ représente un groupe 1-hydroxy-1-éthyle, R₆ représente un groupe phényle ; et X représente S ;
(x) la formule dans laquelle R₁ et R₉ représentent l'un et l'autre un groupe OH ; R₂, R₄, R₅, R₆, R₇ et R₈ représentent tous H ; R₃ représente un groupe benzyle ; et X représente un groupe SO ;
(xi) la formule dans laquelle R₁ et R₉ représentent l'un et l'autre un groupe OH ; R₂, R₄, R₅, R₆, R₇ et R₈ représentent tous H ; R₃ représente un groupe 2-méthyl-1-propyle ; et X représente un groupe (CH₂)ₖ et K est égal à 0 ; (xii) la formule dans laquelle R₁ et R₉ représentent l'un et l'autre un groupe OH ; R₂, R₄, R₅, R₆, R₇ et R₈ représentent tous H ; R₃ représente un groupe 1-hydroxy-1-éthyle ; et X représente un groupe SO ;
(xiii) la formule dans laquelle R₁ et R₉ représentent l'un et l'autre un groupe OH ; R₂, R₄, R₅, R₆, R₇ et R₈ représentent tous H ; R₃ représente un groupe 2-propyle et X représente S ;
(xiv) la formule dans laquelle R₁ et R₉ représentent l'un et l'autre un groupe OH ; R₂, R₄, R₅, R₆, R₇ et R₈ représentent tous H ; R₃ représente un groupe 4-amino-1-butyle ; et X représente un groupe SO ;
(xv) la formule dans laquelle R₁ et R₉ représentent l'un et l'autre un groupe OH ; R₂, R₄, R₅, R₆, R₇ et R₈ représentent tous H ; R₃ représente un groupe 2-propyle et X représente un groupe (CH₂)ₖ et K est égal à 0 ;
(xvi) la formule dans laquelle R₁ représente un groupe NH₂ ; R₂, R₄, R₅, R₆, R₇ et R₈ représentent tous H ; R₃ représente un groupe 4-hydroxybenzyle ; R₉ représente un groupe OH ; et X représente S ;
(xvii) la formule dans laquelle R₁ et R₉ représentent l'un et l'autre un groupe OH ; R₂, R₃, R₄, R₅, R₇ et R₈ représentent tous H ; R₆ représente un groupe 2-naphtyle et X représente S ;
(xviii) la formule dans laquelle R₁ et R₉ représentent l'un et l'autre un groupe OH ; R₂, R₄, R₅, R₆, R₇ et R₈ représentent tous H ; R₃ représente un groupe 2-propyle ; et X représente un groupe NH ;
(xix) la formule dans laquelle R₁ et R₉ représentent l'un et l'autre un groupe OH ; R₂, R₄, R₅, R₆, R₇ et R₈ représentent tous H ; R₃ représente un groupe 1-hydroxy-1-éthyle ; et X représente S ;
(xx) la formule dans laquelle R₁ et R₉ représentent l'un et l'autre un groupe OH ; R₂, R₄, R₅, R₆, R₇ et R₈ représentent tous H ; R₃ représente un groupe 4-hydroxybenzyle ; et X représente S ;
(xxi) la formule dans laquelle R₁ et R₉ représentent l'un et l'autre un groupe OH ; R₂, R₃, R₄, R₅, R₇ et R₈ représentent tous H ; R₆ représente un groupe phényle ; et X représente S ;
(xxii) la formule dans laquelle R₁ et R₉ représentent l'un et l'autre un groupe OH ; R₂, R₄, R₅, R₆, R₇ et R₈ représentent tous H ; R₃ représente un groupe 4-hydroxybenzyle et X représente un groupe (CH₂)ₖ et K est égal à 0 ;
(xxiii) la formule dans laquelle R₁ et R₉ représentent l'un et l'autre un groupe OH ; R₂, R₄, R₅, R₆, R₇ et R₈ représentent tous H ; R₃ représente un groupe 2-méthyl-1-propyle ; et X représente S ;
(xxiv) la formule dans laquelle R₁ et R₉ représentent l'un et l'autre un groupe OH ; R₂, R₄, R₅, R₆, R₇ et R₈ représentent tous H ; R₃ représente un groupe benzyle et X représente un groupe (CH₂)ₖ et K est égal à 0 ;
(xxv) la formule dans laquelle R₁ et R₉ représentent l'un et l'autre un groupe OH ; R₂, R₄, R₅, R₇ et R₈ représentent tous H ; R₃ représente un groupe 4-hydroxybenzyle, R₆ représente un groupe phényle et X représente S ;
(xxvi) la formule dans laquelle R₁ et R₉ représentent l'un et l'autre un groupe OH ; R₂, R₄, R₅, R₆, R₇ et R₈ représentent tous H ; R₃ représente un groupe 3-indolylméthyle ; et X représente un groupe (CH₂)ₖ et K est égal à 0 ;
(xxvii) la formule dans laquelle R₁ et R₉ représentent l'un et l'autre un groupe OH ; R₂, R₄, R₅, R₆, R₇ et R₈ représentent tous H ; R₃ représente un groupe benzyle et X représente S ;
(xxviii) la formule dans laquelle R₁ et R₉ représentent l'un et l'autre un groupe OH ; R₂, R₄, R₅, R₆, R₇ et R₈ représentent tous H ; R₃ représente un groupe 4-hydroxybenzyle et X représente un groupe NH ;
(xxix) la formule dans laquelle R₁ et R₉ représentent l'un et l'autre un groupe OH ; R₂, R₄, R₅, R₆, R₇ et R₈ représentent tous H ; R₃ représente un groupe 1-hydroxyéthyle et X représente un groupe (CH₂)ₖ et K est égal à 0 ;
(xxx) la formule dans laquelle R₁ et R₉ représentent l'un et l'autre un groupe OH ; R₂, R₃, R₄, R₅ et R₆ représentent tous H ; R₇ et R₈ représentent un groupe CH₃ et X représente S
(xxxi) la formule dans laquelle R₁ et R₉ représentent l'un et l'autre un groupe OH ; R₂, R₃, R₄, R₅, R₆, R₇ et R₈ représentent tous H ; et X représente un groupe SO ;
(xxxii) la formule dans laquelle R₁ et R₉ représentent l'un et l'autre un groupe OH ; R₂, R₅, R₆, R₇ et R₈ représentent tous H ; R₃ et R₄ représentent un groupe CH₂CH₂CH₂ ; et X représente S ;
(xxxiii) la formule dans laquelle R₁ et R₉ représentent l'un et l'autre un groupe OH ; R₂, R₄, R₅, R₆, R₇ et R₈ représentent tous H ; R₃ représente un groupe 3-indolylméthyle et X représente S ;
(xxxiv) la formule dans laquelle R₁ et R₉ représentent l'un et l'autre un groupe OH ; R₂, R₄, R₅, R₆, R₇ et R₈ représentent tous H ; R₃ représente un groupe hydroxyméthyle et X représente S ;
(xxxv) la formule dans laquelle R₁ et R₉ représentent l'un et l'autre un groupe OH ; R₂, R₄, R₅, R₆, R₇ et R₈ représentent tous H ; R₃ représente un groupe 4-imidazolylméthyle et X représente S ;
(xxxvi) la formule dans laquelle R₁ et R₉ représentent l'un et l'autre un groupe OH ; R₂, R₄, R₅, R₆, R₇ et R₈ représentent tous H ; R₃ représente un groupe méthyle et X représente S.

5. Composé suivant la revendication 2, choisi parmi des composés dans lesquels
(i) R₁ et R₉ représentent des groupes OH ;
R₂, R₄, R₅, R₆, R₇, R₈, R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente S ; et
n est égal à 3 ou 4 ;
(ii) R₁ et R₉ représentent des groupes OH ;
R₂, R₄, R₅, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente un groupe SO ; et
n est égal à 3 ou 4 ;
(iii) R₁ et R₉ représentent des groupes OH ;
R₂, R₃, R₄, R₇, R₈ et R₁₄ représentent l'hydrogène ;
R₅ et R₆ sont choisis indépendamment entre
l'hydrogène, les groupes méthyle, éthyle, propyle, isopropyle, butyle, isobutyle, benzyle, phényle, 2-trifluorométhylphényle, 3-trifluorométhylphényle, 4-trifluorométhylphényle, 1-naphtyle, 2-naphtyle, 4-biphényle et pentafluorophényle ;
X représente S ; et
n est égal à 3 ou 4 ;
(iv) R₁ et R₉ représentent des groupes OH ;
R₂, R₃, R₄, R₇, R₈ et R₁₄ représentent l'hydrogène :
R₅ et R₆ sont choisis indépendamment entre
l'hydrogène, les groupes méthyle, éthyle, propyle, isopropyle, butyle, isobutyle, benzyle, phényle, 2-trifluorométhylphényle, 3-trifluorométhylphényle, 4-trifluorométhylphényle, 1-naphtyle, 2-naphtyle, 4-biphényle et pentafluorophényle ;
X représente un groupe SO ; et
n est égale à 3 ou 4 ;
(v) R₁ et R₉ représentent des groupes OH ;
R₂, R₃, R₄, R₅, R₆ et R₁₄ représentent l'hydrogène ;
R₇ et R₈ sont choisis indépendamment entre l'hydrogène, les groupes méthyle et phényle ;
X représente S ; et
n est égal à 3 ou 4 ;
(vi) R₁ et R₉ représentent des groupes OH ;
R₂, R₃, R₄, R₅, R₆ et R₁₄ représentent l'hydrogène ;
R₇ et R₈ sont choisis indépendamment entre l'hydrogène, les groupes méthyle et phényle ;
X représente un groupe SO ; et
n est égal à 3 ou 4 ;
(vii) R₁ et R₉ représentent des groupes OH ;
R₂, R₄, R₇, R₈ et R₁₄ représentent l'hydrogène ;
R₃ est choisi entre les groupes
4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
R₅ et R₆ sont choisis indépendamment entre
l'hydrogène, les groupes méthyle, éthyle, propyle, isopropyle, butyle, isobutyle, benzyle, phényle, 2-trifluorométhylphényle, 3-trifluorométhylphényle, 4-trifluorométhylphényle, 1-naphtyle, 2-naphtyle, 4-biphényle et pentafluorophényle ;
X représente S ; et
n est égal à 3 ou 4 ;
(viii) R₁ et R₉ représentent des groupes OH ;
R₂, R₄, R₇, R₈ et R₁₄ représentent l'hydrogène ;
R₃ est choisi entre les groupes
4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
R₅ et R₆ sont choisis indépendamment entre
l'hydrogène, les groupes méthyle, éthyle, propyle, isopropyle, butyle, isobutyle, benzyle, phényle, 2-trifluorométhylphényle, 3-trifluorométhylphényle, 4-trifluorométhylphényle, 1-naphtyle, 2-naphtyle, 4-biphényle et pentafluorophényle ;
X représente un groupe SO ; et
n est égal à 3 ou 4 ;
(ix) R₁ et R₉ représentent des groupes OH ;
R₂, R₄, R₅, R₆ et R₁₄ représentent l'hydrogène ;
R₃ est choisi entre les groupes
4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
R₇ et R₈ sont choisis indépendamment entre
l'hydrogène, les groupes méthyle et phényle ;
X représente S ; et
n est égal à 3 ou 4 ;
(x) R₁ et R₉ représentent des groupes OH ;
R₂, R₄, R₅, R₆ et R₁₄ représentent l'hydrogène ;
R₃ est choisi entre les groupes
4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
R₇ et R₈ sont choisis indépendamment entre
l'hydrogène, les groupes méthyle et phényle ;
X représente un groupe SO ; et
n est égal à 3 ou 4 ;
(xi) R₁ et R₉ représentent des groupes OH ;
R₂, R₃, R₄ et R₁₄ représentent l'hydrogène ;
R₅ et R₆ sont choisis indépendamment entre
l'hydrogène, les groupe méthyle, éthyle, propyle, isopropyle, butyle, isobutyle, benzyle, phényle, 2-trifluorométhylphényle, 3-trifluorométhylphényle, 4-trifluoro-méthylphényle, 1-naphtyle, 2-naphtyle, 4-biphényle et pentafluorophényle ;
R₇ et R₈ sont choisis indépendamment entre
l'hydrogène, les groupes méthyle et phényle ;
X représente S ; et
n est égal à 3 ou 4 ;
(xii) R₁ et R₉ représentent des groupes OH ;
R₂, R₃, R₄ et R₁₄ représentent l'hydrogène ;
R₅ et R₆ sont choisis indépendamment entre
l'hydrogène, les groupes méthyle, éthyle, propyle, isopropyle, butyle, isobutyle, benzyle, phényle, 2-trifluorométhylphényle, 3-trifluorométhylphényle, 4-trifluorométhylphényle, 1-naphtyle, 2-naphtyle, 4-biphényle et pentafluorophényle ;
R₇ et R₈ sont choisis indépendamment entre
l'hydrogène, les groupes méthyle et phényle ;
X représente un groupe SO ; et
n est égal à 3 ou 4 ;
(xiii) R₁ et R₉ représentent des groupes OH ;
R₂, R₄, R₅, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente O ; et
n est égal à 3 ou 4 ;
(xiv) R₁ et R₉ représentent des groupes OH ;
R₂, R₄, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente un groupe NR₁₃ dans lequel R₁₃ est choisi entre
l'hydrogène, des groupes alkyle en C₁ à C₄, alcanoyle en C₁ à C₄, benzoyle et benzyle ; et
n est égal à 3 ou 4 ;
(xv) R₁ et R₉ représentent des groupes OH ;
R₂, R₄, R₅, R₆, R₇, R₈ et R₁₄ représentent l'hydrogène ;
R₃ représente un groupe 4-hydroxybenzyle ;
X représente S ; et
n est égal à 3 ;
(xvi) R₁ et R₉ représentent des groupes OH ;
R₂, R₄, R₅, R₆, R₇, R₈ et R₁₄ représentent l'hydrogène ;
R₃ représente un groupe 4-hydroxybenzyle ;
X représente un groupe SO ; et
n est égal à 3 ;
(xvii) R₁ et R₉ représentent des groupes OH ;
R₂, R₄, R₅, R₆, R₇, R₈ et R₁₄ représentent l'hydrogène ;
R₃ représente un groupe isopropyle
X représente S ; et
n est égal à 3 ;
(xviii) R₁ et R₉ représentent des groupes OH ;
R₂, R₄, R₅, R₆, R₇, R₈ et R₁₄ représentent l'hydrogène ;
R₃ représente un groupe isopropyle ;
X représente un groupe SO ; et
n est égal à 3 ;
(xix) R₁ et R₉ représentent des groupes OH ;
R₂, R₄, R₅, R₆, R₇, R₈ et R₁₄ représentent l'hydrogène ;
R₃ représente un groupe 2-butyle ;
X représente S ; et
n est égal à 3 ;
(xx) R₁ et R₉ représentent des groupes OH ;
R₂, R₄, R₅, R₆, R₇, R₈ et R₁₄ représentent l'hydrogène ;
R₃ représente un groupe 2-butyle ;
X représente un groupe SO ; et
n est égal à 3 ;
(xxi) R₁ et R₉ représentent des groupes OH ;
R₂, R₅, R₆, R₇, R₈ et R₁₄ représentent l'hydrogène ;
R₃ et R₄ sont joints l'un à l'autre pour former un noyau pyrrolidine ;
X représente S ; et
n est égal à 3 ;
(xxii) R₁ et R₉ représentent des groupes OH ;
R₂, R₅, R₆, R₇, R₈ et R₁₄ représentent l'hydrogène ;
R₃ et R₄ sont joints l'un à l'autre pour former un noyau pyrrolidine ;
X représente un groupe SO ; et
n est égal à 3 ;
(xxiii) R₁ et R₉ représentent des groupes OH ;
R₂, R₄, R₅, R₆ et R₁₄ représentent l'hydrogène ;
R₃ représente un groupe 4-hydroxybenzyle ;
R₇ et R₈ représentent des groupes méthyle ;
X représente S ; et
n est égal à 3
(xxiv) R₁ et R₉ représentent des groupes OH ;
R₂, R₄, R₅, R₆ et R₁₄ représentent l'hydrogène ;
R₃ représente un groupe 4-hydroxybenzyle ;
R₇ et R₈ représentent des groupes méthyle
X représente un groupe SO ; et
n est égal à 3 ;
(xxv) R₁ et R₉ représentent des groupes OH ;
R₂, R₄, R₅, R₆ et R₁₄ représentent l'hydrogène ;
R₃ représente un groupe isopropyle
R₇ et R₈ représentent des groupes méthyle
X représente S ; et
n est égal à 3 ;
(xxvi) R₁ et R₉ représentent des groupes OH ;
R₂, R₄, R₅, R₆ et R₁₄ représentent l'hydrogène ;
R₃ représente un groupe isopropyle
R₇ et R₈ représentent des groupes méthyle
X représente un groupe SO ; et
n est égal à 3 ;
(xxvii) R₁ et R₉ représentent des groupes OH ;
R₂, R_{3,} R₄, R₅, R₆ et R₁₄ représentent l'hydrogène ;
R₇ et R₈ représentent des groupes méthyle ;
X représente S ; et
n est égal à 3 ;
(xxviii) R₁ et R₉ représentent des groupes OH ;
R₂, R_{3,} R₄, R₅, R₆ et R₁₄ représentent l'hydrogène
R₇ et R₈ représentent des groupes méthyle
X représente un groupe SO ; et
n est égal à 3 ;
(xxix) R₁ représente un groupe NH2 ;
R₉ représente un groupe OH ;
R₂, R₄, R₅, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente S ; et
n est égal à 3 ou 4 ;
(xxx) R₁ représente un groupe NH₂ ;
R₉ représente un groupe OH ;
R₂, R₄, R₅, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente un groupe SO ; et
n est égal à 3 ou 4 ;
(xxxi) R₁ et R₉ représentent des groupes OH ;
R₂, R₄, R₅, R₆, R₇, R₈ et R₁₄ représentent l'hydrogène ;
R₃ est choisi entre
l'hydrogène, les groupes 4-amino-1-butyle, 3-amino-1-propyle, hydroxyméthyle, 2-hydroxy-1-propyle, méthyle, éthyle, 2-propyle, isobutyle, n-propyle, n-butyle, 2-butyle, méthylthioéthyle, benzyle, 4-hydroxybenzyle, 4-méthoxybenzyle, 3-indolylméthyle, 4-imidazolylméthyle, phényle, carboxyméthyle, carboxyéthyle, carboxamidométhyle et carboxamidoéthyle ;
X représente un groupe (CH₂)ₖ dans lequel k est égal à 0, 1 ou 2 ; et
n est égal à 3 ou 4 ;
(xxxii) R₁ et R₉ représentent des groupes OH ;
R₂, R₄, R₅, R₆, R₇, R₈ et R₁₄ représentent l'hydrogène ;
R₃ représente un groupe 4-hydroxybenzyle ;
X représente un groupe CH₂ ; et
n est égal à 3 ;
(xxxiii) R₁ et R₉ représentent des groupes OH ;
R₂, R₄, R₅, R₆, R₇, R₈ et R₁₄ représentent l'hydrogène ;
R₃ représente un groupe isopropyle
X représente un groupe CH₂ ; et
n est égal à 3 ;
(xxxiv) R₁ et R₉ représentent des groupes OH ;
R₂, R₄, R₅, R₆, R₇, R₈ et R₁₄ représentent l'hydrogène ;
R₃ représente un groupe 2-butyle ;
X représente un groupe CH₂ ; et
n est égal à 3 ;
(xxxv) R₁ et R₉ représentent des groupes OH ;
R₂, R₅, R₆, R₇, R₈ et R₁₄ représentent l'hydrogène ;
R₃ et R₄ sont joints l'un à l'autre pour former un noyau pyrrolidine ;
X représente un groupe CH₂ ; et
n est égal à 3.

6. Procédé de préparation du composé suivant la revendication 2, qui comprend :
(a) la cyclisation d'un intermédiaire de formule IV 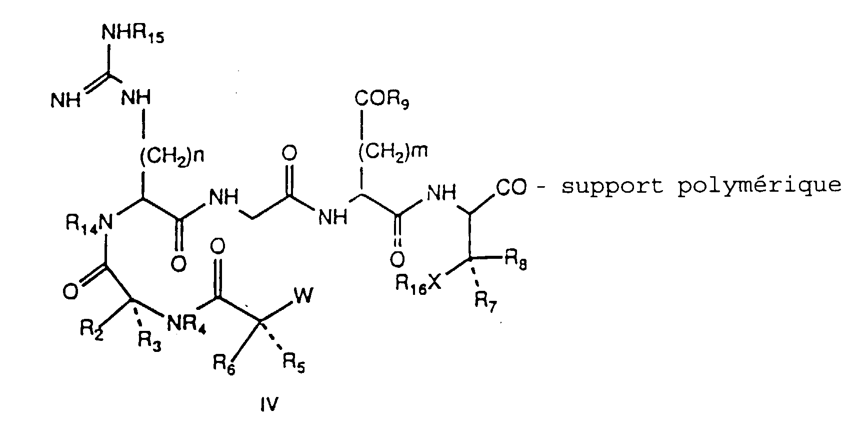 dans laquelle
R₁ à R₉, n et m répondent aux définitions suivant la revendication 2,
W représente Br, Cl ou I,
R₁₄ est choisi entre l'hydrogène, des groupes alkyle en C₁ à C₆ et aryle en C₆ à C₁₂,
R₁₅ représente un groupe 2,2,5,7,8-pentaméthylchromane-6-sulfonyle,
R₁₆ représente l'hydrogène, et
X représente S ;
(b) le clivage du produit désiré de la résine ;
(c) le clivage de tous les groupes protecteurs ;
(d) la purification et l'isolement de l'isomère le plus actif du point de vue biologique.

7. Procédé de préparation du composé suivant la revendication 2, qui comprend :
(a) la cyclisation d'un intermédiaire de formule VI 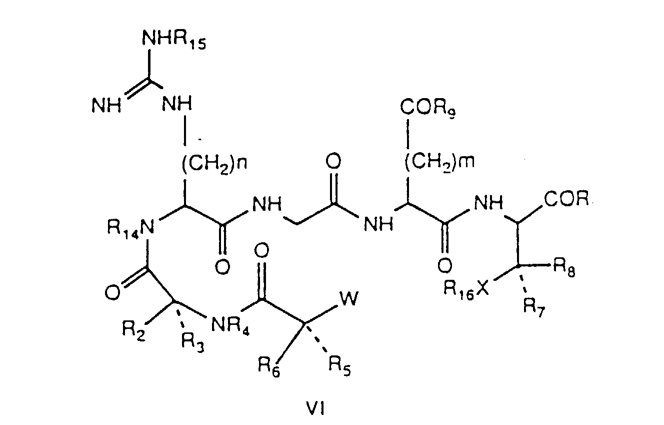 dans laquelle
R₁ à R₉, n et m répondent aux définitions suivant la revendication 2,
W représente Br, Cl ou I,
R₁₄ est choisi entre l'hydrogène, des groupes alkyle en C₁ à C₆ et aryle en C₆ à C₁₂ ;
R₁₅ représente un groupe 2,2,5,7,8-pentaméthylchromane-6-sulfonyle,
R₁₆ représente H, et
X représente S ;
(b) le clivage de tous les groupes protecteurs ; et
(c) la purification et l'isolement de l'isomère le plus actif du point de vue biologique.

8. Composition pharmaceutique comprenant un excipient pharmaceutiquement acceptable et le composé suivant la revendication 1.

9. Utilisation d'un composé suivant la revendication 1 dans la production d'un médicament destiné à inhiber l'agrégation plaquettaire.

10. Utilisation d'un composé suivant la revendication 1 dans la production d'un médicament destiné à être administré à un mammifère ayant une tendance accrue à la formation de thrombus.

11. Composition comprenant un composé suivant la revendication 1 pour l'une quelconque des actions suivantes :
(a) réduction de l'agrégation plaquettaire ;
(b) traitement d'un mammifère qui présente une tendance accrue à la formation de thrombus ;
(c) inhibition de la liaison du fibrinogène aux plaquettes chez un mammifère.

12. Composition comprenant un composé suivant la revendication 1, dans lequel R₁ représente un groupe alkoxy en C₁ à C₆ qui comprend des groupes alkyle ramifiés et insaturés.

13. Composition comprenant un composé suivant la revendication 1, dans lequel R₉ représente un groupe alkoxy en C₁ à C₆, qui comprend des groupes alkyle ramifiés et insaturés.

14. Composition comprenant un composé suivant la revendication 1, dans lequel R₁ et R₉ représentent l'un et l'autre un groupe alkoxy en C₁ à C₆ qui comprend des groupes alkyle ramifiés et insaturés.

15. Utilisation d'une composition suivant l'une quelconque des revendications 11 à 13 dans la production d'un médicament destiné au traitement d'un mammifère qui présente une tendance accrue à la formation de thrombus, dans laquelle les groupes alkoxy R₁ et R₉ sont hydrolysables après administration audit mammifère.

16. Utilisation d'un composé suivant la revendication 1 en association avec un agent thrombolytique dans la production d'un médicament destiné au traitement d'un mammifère qui présente une tendance accrue à la formation de thrombus.

17. Utilisation d'un composé suivant la revendication 1 en association avec un anticoagulant dans la production d'un médicament destiné au traitement d'un mammifère qui présente une tendance accrue à la formation de thrombus.

18. Utilisation suivant la revendication 10, dans laquelle le médicament est destiné à être administré après angioplastie.

19. Peptide cyclique contenant la séquence tripeptidique Arg-Gly-Asp et une liaison thioéther dans le cycle.

20. Peptide cyclique suivant la revendication 19, ledit peptide cyclique contenant 5 aminoacides dans le cycle.

21. Peptide cyclique suivant la revendication 19, dans lequel le cycle contient 17 ou 18 atomes dans un noyau.

22. Peptide cyclique suivant la revendication 21, ledit peptide cyclique contenant au moins un D-α-aminoacide.

23. Peptide cyclique suivant la revendication 22, dans lequel le D-aminoacide est présent en n'importe quelle position dans le cycle à l'exception de la séquence Arg-Gly-Asp.

24. Peptide cyclique suivant la revendication 20, dans lequel l'atome de soufre de la liaison thioéther est lié à au moins un atome d'oxygène.

25. Pentapeptide cyclique contenant la séquence tripeptidique Arg-Gly-Asp, dans lequel le cycle comprend une liaison peptidique à une chaîne latérale d'aminoacides.

26. Pentapeptide cyclique suivant la revendication 25, dans lequel la liaison peptidique est réalisée par l'intermédiaire du groupe carboxyle en position 6 de l'acide 2-amino-1,6-hexanedioique.

27. Pentapeptide cyclique suivant la revendication 26, dans lequel le cycle contient 17 ou 18 atomes dans un noyau.
